# EUROPEAN PATENT SPECIFICATION

(11) **EP 1 871 310 B1**
(45) Date of publication and mention of the grant of the patent: **27.07.2016**
(21) Application number: 06750814.3
(22) Date of filing: 20.04.2006
(51) Int. Cl.: A61F 2/95

(54) **MEDICAL APPARATUS FOR RAPID INSERTION**
MEDIZINISCHES GERÄT ZUR SCHNELLEN EINFÜHRUNG
APPAREIL MEDICAL POUR INSERTION RAPIDE

(30) Priority: 20.04.2005 US 673199 P
(43) Date of publication of application: 02.01.2008
(73) Proprietor: Cook Medical Technologies LLC, Bloomington, IN 47404 (US)
(72) Inventor: PAL, Dharmendra, Wilmington, Massachusetts 01887 (US); AGNEW, Charles, W., West Lafayette, Indiana 47906 (US); FLAGLE, Jacob, A., Indianapolis, Indiana 46239 (US); LENTZ, D., Christian, Bloomington, Indiana 47401 (US); MELSHEIMER, Jeffry, S., Springville, Indiana 47462 (US); OSBORNE, Thomas, A., Bloomington, Indiana 47401 (US); PARKER, Fred, T., Unionville, Indiana 47468 (US)
(74) Representative: Mathys & Squire LLP
(86) International application number: PCT/US2006/014864
(87) International publication number: WO 2006/113863

(56) References cited:
- EP-A- 1 095 634
- US-A1- 2004 186 506

## Description

### Technical Field

The present invention relates to medical devices generally used percutaneously or through a delivery apparatus (such as an endoscope) for the delivery of self-expanding devices, as well as methods of making and using such devices.

### BACKGROUND OF THE INVENTION

This invention relates generally to medical devices and, in particular, to a delivery system that employs a catheter device for use with self-expanding devices such as stents, prosthetic valve devices, and other implantable articles at a selected location inside a patient's body. More particularly, the device includes a flexible, kink-resistant middle section having an outer sheath and inner compression member and having a distal end comprising an inner guide channel member and an outer guide channel member, the inner and outer guide channel members being configured to facilitate rapid insertion of a wire guide and delivery of self-expanding devices.

### SELF-EXPANDING DEVICES

Medical delivery and placement systems and tools have grown out of the need for implanting medical devices in endovascular and other body lumens of a patient. The present invention relates to new and useful medical devices generally used percutaneously or through an apparatus such as an endoscope for delivering a particular type of implantable medical devices: self-expanding devices. Types of self-expanding devices include various medical devices, such as stents, prosthetic valve devices, and other implantable articles for placement inside a patient's body. Although use of the present invention is not limited to stents, a discussion of stent technology provides a general and beneficial introduction into the variety of self-expanding devices that the present invention delivers and places in endovascular and other body lumens.

Minimally invasive surgical stent technology has.become popular since the introduction of stents to the medical device market in the United States in the early 1990s. For more than a decade, stents have proven to provide an excellent means for maintaining vessel patency, and have become widely accepted in the medical field.

By way of background, stents are configured to be implanted into body vessels having a passageway in order to reinforce, support, repair, or otherwise enhance the performance of the passageway. The term "passageway" is understood to be any lumen, channel, flow passage, duct, chamber, opening, bore, orifice, or cavity for the conveyance, regulation, flow, or movement of bodily fluids and/or gases of an animal. As an example, stents have been used in the passageways of an aorta, artery, bile duct, blood vessel, bronchiole, capillary, esophagus, fallopian tube, heart, intestine, trachea, ureter, urethra, vein, and other locations in a body (collectively, "vessel") to name a few.

Depending on the vessel in which it is to be implanted, a stent may come in a variety of different configurations. In general, a stent may comprise a ring, or stack of rings, each ring being formed of struts and apices connecting the struts, whereby the stent defines an approximately tube-like configuration. Furthermore, the stent perimeter, when viewing the stent end-on, may define a structure roughly, but not exactly, resembling a round cylinder if the struts are straight, because the struts follow a straight line from the apex on one end of the strut to the apex on the other end of the strut.

In addition to their having a variety of configurations, stents also come in different types as defined by the way they expand. For instance, various types of expandable stents have been described that are self-expanding, balloon-expandable, or a combination thereof where the stent is partially self-expanding and partially balloon-expandable. Expandable stents are normally evaluated, more or less, with respect to four performance criteria: the radially outward expansile force that the stent exerts in the vessel passageway on an interior surface of the vessel; the small diameter to which the stent is capable of being compressed for the insertion procedure; the ability of the stent to traverse curved passageways; and the stability of the stent in being resistant to migrating from its originally implanted position.

A balloon-expandable stent is generally mounted on an expandable member, such as a balloon, provided on the distal end of a delivery system, such as a catheter or tubular delivery device, to be discussed more fully below. In operation, a physician or other healthcare professional (collectively, "physician") inserts the catheter into and navigates the catheter through a vessel passageway by advancing the catheter to a desired location adjacent the target site within the vessel passageway of a patient. In a subsequent step, the physician pulls back a sheath or other covering member to withdraw from and releasably expose the stent for deployment. In another step, the physician inflates the balloon to plastically deform the stent into a substantially permanent expanded condition. The physician then deflates the balloon and removes the catheter or delivery device from the patient's body.

For a self-expanding stent, the stent is resiliently compressed into a collapsed first, smaller diameter, carried by the delivery system, and due to its construction and material properties, the stent expands to its second, larger diameter upon deployment. In its expanded configuration, the stent exhibits sufficient stiffness so that it will remain substantially expanded and exert a radially outward force in the vessel passageway on an interior surface of the vessel.

One particularly useful self-expanding stent is the Z-stent, introduced by Cook Incorporated, due to its ease of manufacturing, high radial force, and self-expanding properties. Examples of the Z-stent are found in United States Patent Nos. 4,580,568; 5,035,706; 5,282,824; 5,507,771; and 5,720,776. The Zilver stent, introduced by Cook Incorporated, is another particularly useful self-expanding stent due to its nitinol platform and use of the Z-stent design properties. Examples of the Zilver stent are found in United States Patent Nos. 6,743,252 and 6,299,635. By way of example only, one or more of these designs have been utilized in stents for applications involving the bronchioles, trachea, thoracic aortic aneurysms (stent-graft), abdominal aortic aneurysms (stent-graft), intestines, biliary tract, and prosthetic venous valve devices. The Z-stent and Zilver stent are capable of being compressed, inserted into a catheter or delivery device, pushed out into the passageway of a vessel, and then self-expanded to help keep the vessel passageway in an open state. A few of the embodiments of devices using one of these stents are the Zilver® 518 biliary self-expanding stent and the Zenith® AAA Endovascular Graft for the endovascular treatment of an abdominal aortic aneurysm.

A stent-of the self-expanding type, like the balloon-expandable type, may assume a collapsed tubular configuration having a smaller diameter and an expanded tubular configuration having a larger diameter. In its collapsed smaller diameter configuration, the stent can be constrained by a delivery system in order to be positioned in the vessel passageway where desired. The stent is then deployed by the delivery system and expanded.

In both the self-expanding and balloon-expandable types of stents (and combinations thereof), a delivery system for percutaneous positioning of the stents in the vessel passageway may use a wire guide. The physician uses the wire guide to maneuver a portion of the delivery system through the vessel passageway and to the desired position therein.

### WIRE GUIDE

A wire guide can be used to place a delivery system, such as a catheter or tubular delivery device, into a vessel passageway percutaneously. A physician may use a cannula or a needle as a way of introducing the wire guide. For instance, the physician may create an incision in the patient and then position the cannula in the incision for the insertion of the wire guide. The typical wire guide has proximal and distal ends. A physician inserts the distal end into the proximal end of the cannula or needle and then out a distal end of the cannula or needle, and thus into the vessel passageway. Once inside the vessel, the wire guide may be advanced and manipulated until the distal end of the wire guide reaches its destination. Alternatively, the physician may place the cannula or needle into any one of a variety of vessels, such as an artery, bile duct, brachial vein, cephalic vein, or other vessel as described above, and then may introduce the wire guide through the needle into the vessel. In subsequent steps, the physician may withdraw the needle over the wire guide and introduce a guide catheter over the wire guide and into the patient.

Regardless of how the physician places the wire guide into a vessel passageway for use with a traditional delivery system, the physician must ensure that the catheter or other medical device has the right length relative to the length of the wire guide. Consequently, the catheter or other medical device will be inserted over the wire guide to a precise length inside the patient so that a distal end of the catheter or other medical device is positioned at or near the distal end of the wire guide in proximity to a target site for treatment, diagnosis, or medical intervention. However, a long, straightened portion of he wire guide will remain outside of the patient's body and may get caught on outside objects and disturb the placement of the catheter.

Additional procedures that involve wire guides often require exchanging of catheters or medical devices. For example, a catheter may be used for the delivery of a self-expanding device such as a stent. In that case, the stent may be deployed, the catheter withdrawn and reloaded with an additional stent or replaced with a separate catheter carrying another stent, or even exchanged for a different medical device. This procedure may prove cumbersome and time consuming, because as explained below, the overall length of a wire guide in an over-the-wire delivery system needs to be substantially longer than the length of the delivery catheter (e.g., the wire guide may need to be twice as long as the catheter). Furthermore, the proximal portion of the wire guide outside the patient's body must remain a sterile field, but as explained below, when the wire guide needs removing or the catheter exchanging for another catheter or other medical device, then an additional person in the operating room needs to hold onto the proximal end of the wire guide to keep the wire guide straight and to prevent unintended proximal or distal movement of the wire guide.

### CATHETER

Many delivery systems employ a catheter, sheath, cannula, introducer, or other medical delivery device or tube-like structure, which must "slide over" the wire guide yet fit "within" a guide catheter or a working channel of an endoscope or an external accessory channel device used with an endoscope (individually and collectively, "catheter"). The catheter comprises a lumen for the wire guide. Generally stated, these delivery systems may fall within two categories. The first category of delivery systems to have been used, and consequently the first to be discussed below, is commonly referred to as an "over-the-wire" catheter system. The other category of delivery systems is sometimes referred to as a "rapid exchange" catheter system. In either system, a wire guide is used to position the delivery system within a vessel passageway. The typical wire guide has proximal and distal ends. A physician inserts the distal end into the vessel passageway, advances, and maneuvers the wire guide until the distal end reaches its desired position within the vessel passageway.

In the "over-the-wire" catheter delivery system, a physician places the catheter over the wire guide, with the wire guide being received into a lumen that extends substantially through the entire length of the catheter. In this over-the-wire type of delivery system, the wire guide may be back-loaded or front-loaded into the catheter. In front-loading an over-the-wire catheter delivery system, the physician inserts the distal end of the wire guide into the catheter's lumen at or near the catheter's proximal end. In back-loading an over-the-wire catheter delivery system, the physician inserts a distal portion of the catheter over the proximal end of the wire guide. The back-loading technique is more common when the physician has already placed the wire guide into the patient, which is typically the case today. In either case of back-loading or front-loading an over-the-wire catheter delivery system, the proximal and distal portions of the catheter will generally envelop the length of the wire guide that lies between the catheter first and second ends. While the wire guide is held stationary, the physician may maneuver the catheter through the vessel passageway to a target site at which the physician is performing or intends to perform a treatment, diagnostic, or other medical procedure.

Because this delivery system fits over the wire guide, the replacement or exchange of catheters or medical devices requires that the portion of the wire guide protruding from the patient's body be longer than the replacement or exchanged catheter or medical device. For instance, if 60 centimeters (cm) of the catheter is to be inserted into the patient, then a wire guide will need to be slightly longer than approximately 120cm, because the physician will need to hold or secure the proximal portion of the wire guide (i.e., the portion extending out of the body) as the delivery catheter is being back-loaded onto the wire guide. Consequently, the overall length of the wire guide may need to be at least more than 120 cm. Likewise, replacement or exchange of a catheter or medical device requires that a physician secure the proximal end of this lengthy wire guide while the catheter is withdrawn from the patient until the distal end of the catheter is then slipped off of the proximal end of the wire guide. This procedure requires a considerable operational time when the catheter is initially placed over the wire guide for insertion into the patient and is withdrawn from the wire guide for exchange or replacement. Furthermore, the extra wire guide length makes control and manipulation of the wire guide difficult, cumbersome, and usually requires an extra person in the sterile field of the procedure to help control and protect the proximal end of the wire guide.

Therefore, there is a need for an apparatus that enables a user to quickly and easily advance a catheter over a wire guide. The present invention solves these and other problems.

Another problem with conventional over-the-wire delivery systems is tendency for the stent-carrying inner catheter to kink during stent deployment. It should be understood in conceptual terms how self-expanding devices (such as stents, prosthetic valve devices, and other implantable articles) are generally deployed from the delivery system. Using a self-expanding stent as an example, inner and outer catheters are utilized for delivering the implantable stent to a deployment site well within the vessel passageway of the patient. The stent is releasably positioned on a platform located at the distal end of a stent-carrying inner catheter. On the platform, the stent is seated axially intermediate optional distal and proximal radiopaque restraint markers and sandwiched transversely (i.e., compressed) between the outer catheter and the inner catheter.

Typically (using the self-expanding stent for illustration), the stent-constraining outer catheter is withdrawn proximally over the stent. As is conventional, "distal" means away from the physician when the device is inserted into a patient, while "proximal" means closest to or toward the physician when the device is inserted into a patient. The outer catheter and inner catheter must be flexible enough to navigate a vessel passageway. In particular, the inner catheter may be susceptible to kinking when the stent-constraining outer catheter is withdrawn to deploy the self-expanding device, because the self-expanding device exerts friction and drag on the interior surface of the stent-constraining outer catheter, owing to the self-expanding device's radially outward expansile force property as discussed above. Thus, the self-expanding device and the stent-carrying inner catheter will be prone to withdraw together with the stent-constraining outer catheter. As a result, the inner catheter may be prone to kink upon withdrawal of the outer catheter.

Therefore, there is a need for an apparatus that enables the user to withdraw the stent-constraining outer catheter proximally over the stent without causing the stent-carrying inner catheter to move proximally or to kink. The present invention solves these and other problems.

The present invention has enough flexibility to navigate through the vessel passageway with an outer catheter, and an inner compression member has sufficient stiffness to "push" the stent in order to counter the urge for the stent (or a stent-carrying catheter) to prolapse proximally with the withdrawing of the outer guide channel member. As will be understood, "pushing" on the inner compression member will keep a stent-carrying inner catheter (and therefore the stent) from translating as a result of an outer catheter being pulled over the stent; thereby "pushing" holds the stent in place at the desired deployment site within the patient's body.

Another aspect of the present invention is to design the inner compression member so that it optionally has a dual functionality. For instance, in addition to being a flexible and sufficiently stiff member to resist kinking during stent deployment, the inner compression member in one embodiment also carries and removes fluids, where the term "fluid" is understood as including but not limited to gases, air, water, contrast fluid, oil, saline solution, blood sample, flushing solution, medication, drug eluting therapeutic substance, or other liquid, gas, fluid, or medium that is biocompatible or capable of being made biocompatible.

Turning now to a delivery system that is sometimes referred to as a "rapid exchange delivery system," the traditional rapid exchange delivery system may be prone to the same susceptibility of kinking as discussed above and, additionally, may be prone to kink the wire guide as explained below. Like the over-the-wire delivery system, the conventional rapid-exchange system utilizes an outer catheter and an inner catheter, which are substantially co-axial. The conventional rapid-exchange delivery system further comprises a distat segment with a stent-carrying inner member. Unlike the over-the-wire system where the wire guide is received into the lumen of the inner catheter and extends the entire length of the inner catheter, in the rapid exchange delivery system the wire guide occupies a catheter lumen extending only through the distal segment of the outer catheter.

With respect to the distal segment of the outer catheter in a traditional rapid exchange delivery system, the wire guide enters a distal section and, in particular, a lumen of a stent-carrying inner catheter at a distal opening and exits the stent-carrying inner catheter at a side opening proximal to the distal opening. The wire guide in the conventional rapid exchange system bends substantially and diverges significantly away from the longitudinal axis of the stent-carrying inner catheter as it converges toward an exit side port located in an outer wall of a stent-constraining outer catheter. Thus, the wire guide enters the delivery system at the distal portion of the inner catheter, exits the side opening in the inner catheter and the side port of the outer catheter, and then runs proximally along the outside surface of the outer catheter wall and is substantially parallel with or "side-by-side" with the inner catheter.

The bend that the wire guide goes through, in the traditional rapid exchange system, as it passes from the lumen of a stent-carrying inner catheter to the exit side port in the outer wall of the stent-constraining outer catheter proximately causes and contributes to added resistance (compared to a wire guide experiencing substantially no or considerably reduced bending). For instance, the wire guide turns significantly away from the longitudinal axis of the lumen of the stent-carrying inner catheter when the physician withdraws the outer catheter proximally to remove, replace, or exchange the delivery system with a new catheter containing another self-expanding stent or a different medical device. As a result of its bending, the wire guide tends to kink. A kink in the wire guide may cause difficulty during a later step where, after the stent is deployed, the outer catheter during withdrawal catches on the wire guide kink and pulls the distal end of the wire guide away from the deployment site. Also, after the delivery system is withdrawn from the proximal end of the wire guide, the inner catheter may need to be reloaded onto the wire guide (e.g., with an additional stent, or replaced with a catheter carrying a different medical device) and will then encounter the wire guide kink as the inner catheter moves distally.

Buckling and bowing, as two forms of kinking, of the distal segment of the device may occur with a traditional design of a rapid exchange delivery system in the vicinity where the wire guide exits the inner catheter side opening and turns substantially transverse to the inner catheter in order to leave the side exit port of the relatively juxtaposed proximal opening in the stent-carrying member and the side port in the outer wall of the stent-constraining outer catheter. Owing to the transverse position of the side opening in the outer wall of the stent-carrying inner catheter and the transverse side exit port in the outer wall of the stent-constraining outer sheath, there is an open cutout profile in the traditional device that may have a tendency to buckle or bow at the cutout when traversing within the narrow confines of the vessel passageway. The tendency to buckle or bow may decrease with a closure of the cutout by elongating the exit side port in the outer wall of the stent-constraining outer catheter, but this may cause weakness in pull strength or bending properties of the outer catheter. Indeed, the outer catheter and the stent-carrying inner catheter are basically of thin-wall construction: while increasing the thickness of the walls at the location of the inner catheter side opening and the outer catheter exit side port minimally improves the level of resistance to buckling or bowing, this level is still often considered unacceptable. In addition, increasing the thickness of the walls is generally considered undesirable because it necessitates the use of a larger delivery device or entry hole in the patient than would otherwise be required (owing to the increased profile of the catheter). Furthermore, the larger catheter profile may also cause unnecessary irritation of the patient's vessel, as well as limiting the size of the vessel in which the delivery system may operate.

Certain embodiments of the present invention solve these and other problems by providing a breech position opening

Another challenge with the traditional design of a rapid exchange delivery system is proper alignment of the stent-carrying inner catheter side opening and the exit side port of the stent-constraining outer catheter. Misalignment may occur during several steps. For instance, the stent-carrying inner catheter and the stent-constraining outer catheter may rotate or their relative axial positions may shift during navigation and positioning of the delivery system through the vessel passageway. Another example where misalignment may occur is during the withdrawal of the stent-constraining outer catheter. As the outer catheter retracts proximally from the relative position of the stent-carrying inner catheter to expose and to deploy the stent from the stent-carrying platform, misalignment may result. Increasing the size of the exit side port of the stent-constraining outer catheter alleviates some of the alignment challenges but further increases the problems of kinking, buckling, bending, and bowing as discussed above.

Thus, during positioning, operation, repeated use, or patient movement, the stent-constraining outer catheter may be bent or shifted axially or rotationally, thereby resulting in a kink in the sheath or misalignment of the side opening of the stent-carrying inner catheter and the side exit port of the outer catheter's outer wall. A kinked or misaligned catheter may become unusable and, even if useable may be difficult to straighten while positioned in the body of a patient. Consequently, the sheath must be manipulated within the patient's vessel passageway, giving rise to an increased duration in the medical procedure. Alternatively, the delivery system may need to be removed, leaving an enlarged, bleeding opening which typically cannot be reused. Vascular access must then be reattempted at an alternative site, and the procedure restarted. This can cause an unnecessary delay of time and, during emergency procedures, may be life threatening. In addition, in some cases, an acceptable alternative site is not available for introducing another delivery device.

The present invention solves these and other problems by providing a transition region for an inner guide channel member and an outer guide channel member.

Therefore, it would be desirable to have a medical device delivery system for self-expanding devices such as stents, prosthetic valve devices, and other implantable articles inside a patient's body, whereby a flexible, kink-resistant middle section delivery device has an outer sheath and an inner compression member, and a distal end of the device comprises an outer guide channel member and inner guide channel member that comprise a cooperatively kink-resistant wire guide lumen configured to facilitate rapid insertion of a wire guide and delivery of a self expanding device. Consequently, it is desirable to have medical systems and rapid insertion delivery devices for the delivery of self-expanding devices as taught herein, and methods of making and using such devices.

An exemplary rapid exchange stent delivery catheter system is shown in EP-A-1095634.

### Summary of the Invention

The present invention provides medical device delivery systems for the rapid insertion of medical devices.

In one embodiment, an inner guide channel member has a first end portion having an entry port and a second end portion having an exit port and a guide channel therebetween and having an outer surface for a stent platform intermediate the first and second end portions. A proximal stent restraint is disposed about and operatively coupled to the inner guide channel member intermediate the inner guide channel member first and second end portions, and the stent platform distal the proximal stent restraint and proximal the inner guide channel member entry port. An outer guide channel member has first and second end portions defining a channel therebetween for receiving the inner guide channel member second portion, wherein the guide channels are substantially aligned.

In another embodiment, an inner guide channel member has a first end portion with an entry port and a second end portion with an exit port and defining a channel therebetween and having a stent platform intermediate the ports. An outer guide channel member has first and second portions defining a channel sized to slidably receive the inner guide channel member second end portion. The outer guide channel member has a stent constraining surface disposed about the inner guide channel member stent platform, and has a stepped profile with a first outer diameter intermediate the outer guide channel member first and second end portions, and a second smaller outer diameter near the outer guide channel member exit port. The exit ports are in fluid communication.

In still another embodiment, the delivery apparatus includes a system proximal portion having a handle and stylet. A middle section delivery device has an elongate outer sheath operatively coupled to the handle, and an elongate inner compression member having a proximal end and a distal mating end portion, the inner compression member proximal end being operatively coupled to the stylet. A system distal portion has an inner guide channel member with a first and second end portion defining a channel, an outer guide channel member axially slidably receiving the inner guide channel member second end portion. The outer guide channel member has an entry port and an exit port defining a guide channel therebetween, and a stepped profile having a first outer diameter intermediate the outer guide channel member first and second end portions and a second smaller outer diameter located at or near the outer guide channel member second end portion. The system distal portion and middle section delivery device are operatively coupled.
In still another embodiment, methods of using a delivery apparatus for the rapid insertion of medical devices for intracorporeal procedures are provided. A delivery system is provided having a system proximal portion having a handle and a stylet and intended to remain outside a patient, a middle section delivery device having an outer sheath and an inner compression, and a system distal portion having an inner guide channel member and an outer guide channel member having a stepped profile, the system distal portion further comprising a self-expanding deployment device mounting region, a transition region, and a proximal breech opening. A self-expanding device is loaded into a radially compressed state at the stent mounting region. The system distal portion is slide over a previously positioned guiding device. The outer guide channel member is retracted proximally relative to the inner member so as to deploy the self-expanding device.

### Brief Description of the Drawing

Figure 1 is a schematic view, broken away, of a medical device system.
Figure 2 is an exploded side view, broken away, of a proximal portion of a medical device.
Figure 2A shows longitudinally sectioned exploded side views of a handle first connector and a handle second connector.
Figure 2B shows a longitudinally sectioned side view of operatively coupled first and second connectors according to Figures 2A.
Figure 2C shows a longitudinally sectioned side view of a handle first connector and a handle second connector according to Figure 2B operatively coupling a strain relief member and/or an outer sheath.
Figure 3 is a longitudinally sectioned view along a partial length of an embodiment of a catheter used for an outer sheath of a middle section delivery device and/or for an outer guide channel member of a distal portion of a medical device.
Figures 4A and 4B are longitudinal sectional perspective side views, broken away, of embodiments of an outer sheath for a middle section delivery device comprising multifilar material.
Figure 4C is an alternative embodiment of an outer sheath, broken away, for a middle section delivery device comprising a wrapped round coil.
Figure 4D is an alternative embodiment of an outer sheath, broken away, for a middle section delivery device comprising a cable tubing or spiral cut cannula.
Figure 4E is an alternative embodiment of an outer sheath, broken away, for a middle section delivery device comprising a cable tubing connected to spiral cut cannula.
Figure 4F is an alternative embodiment of an outer sheath, broken away, for a middle section delivery device comprising a round braid.
Figure 4G is an alternative embodiment of an outer sheath, broken away, for a middle section delivery device comprising a flat braid.
Figure 5 is a schematic sectional side view, broken away, of an integral outer sheath of a middle section delivery device and an outer guide channel member of a distal portion of a medical device.
Figure 5A provides a longitudinally sectioned schematic side view, broken away, of a brace device for use in a middle section delivery device and/or for bridging the middle section delivery device and a distal portion of a medical device.
Figure 5B shows a cross sectional view of Figure 5A taken along the lines 5B-5B.
Figures 5C through 5G show alternative perspective views of a bridge associating a partial schematic middle section delivery device and a partial schematic distal portion of a medical device according to the invention, the bridge comprising a span.
Figures 5H through 5K show perspective schematic views of an alternative embodiment of a bridge comprising a plurality of spans.
Figure 5L shows an insert having a flared end.
Figure 5M shows an alternative embodiment of an insert.
Figure 5N shows an embodiment of an insert having perforations.
Figure 5O shows an embodiment of an insert having slots.
Figure 5P shows a schematic sectional side views, broken away, of an outer sheath and an outer guide channel member bridged by interfacing distal and middle inserts.

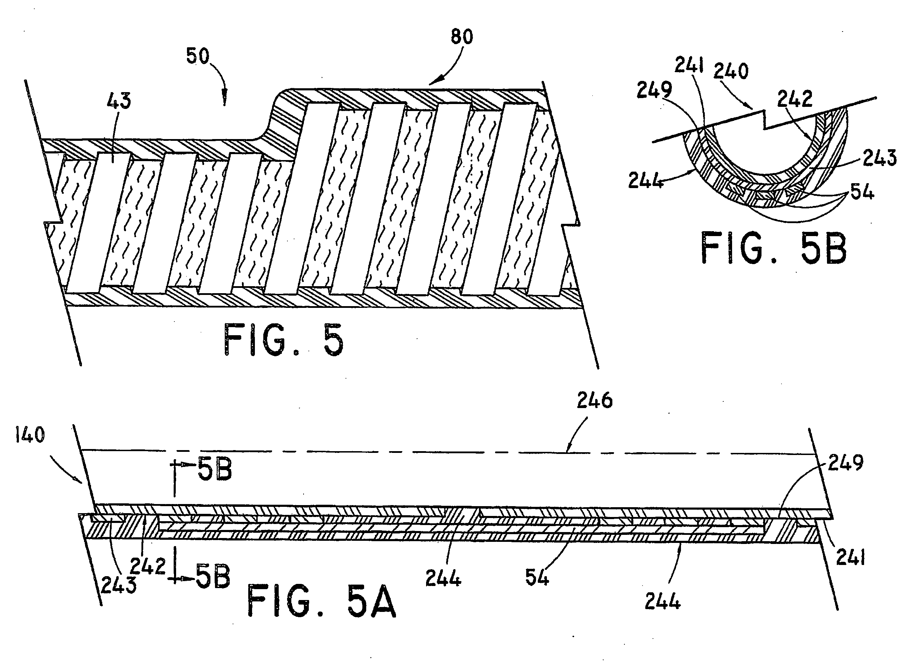

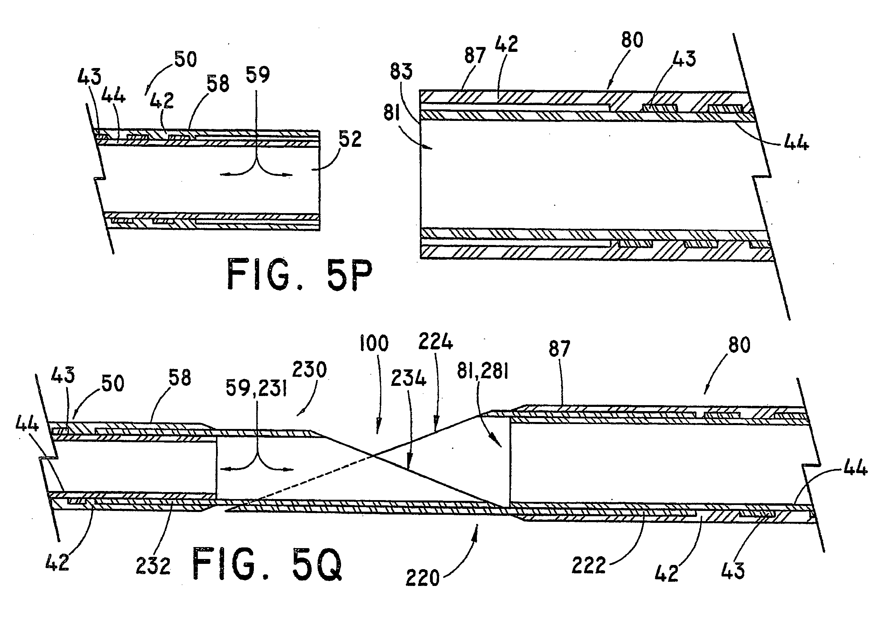

Figure 5P shows a schematic sectional side views, broken away, of an outer sheath and an outer guide channel member.
Figure 5Q shows a schematic sectional side views, broken away, of an outer sheath and an outer guide channel member bridged by interfacing distal and middle inserts.

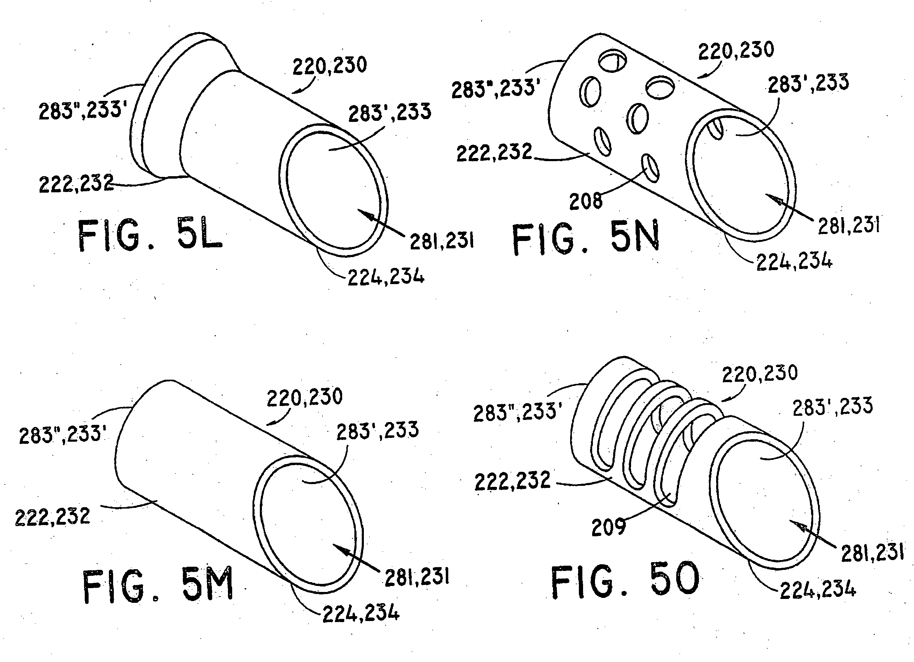

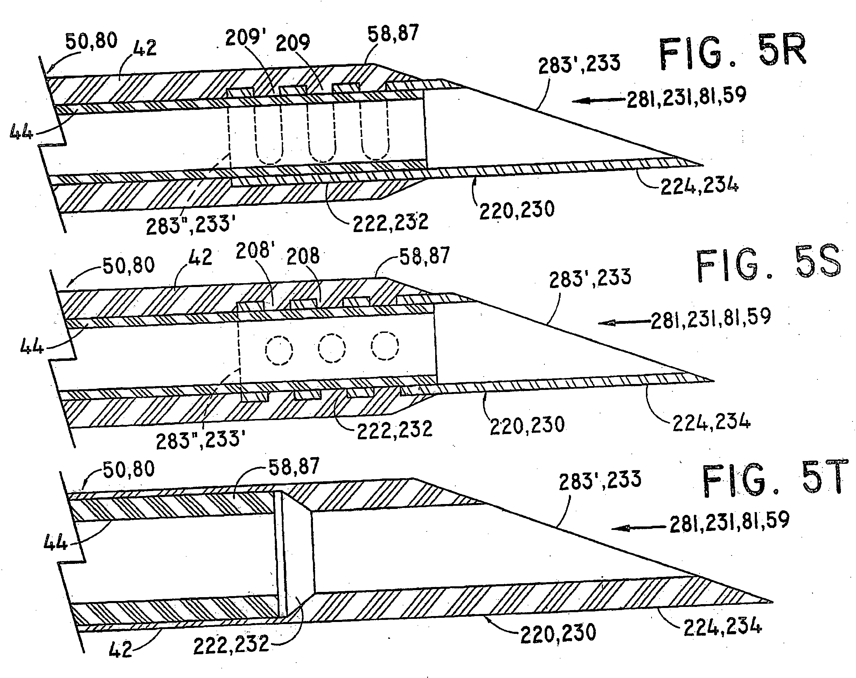

Figure 5R illustrates a sectional side view, broken away, of an embodiment of an insert according to Figure 5O secured to one of the outer sheath of the middle section delivery device or second end of the outer guide channel member.
Figure 5S illustrates a sectional side view, broken away, of an embodiment of an insert according to Figure 5N secured to one of the outer sheath of the middle section delivery device or second end of the outer guide channel member.
Figure 5T illustrates a sectional side view, broken away, of an embodiment of a bridging insert according to Figure 5L secured to one of the outer sheath of the middle section delivery device or second end of the outer guide channel-member.

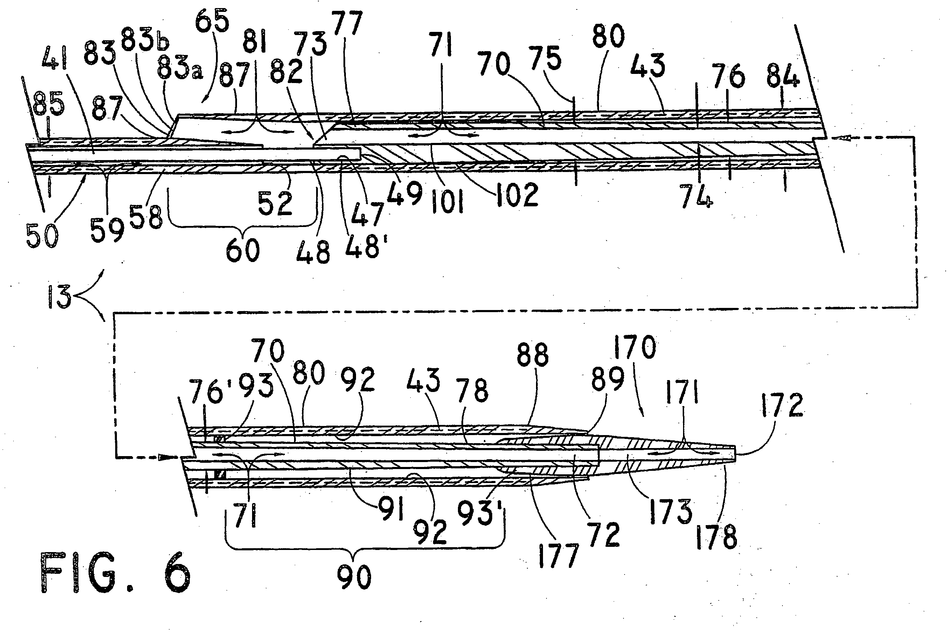

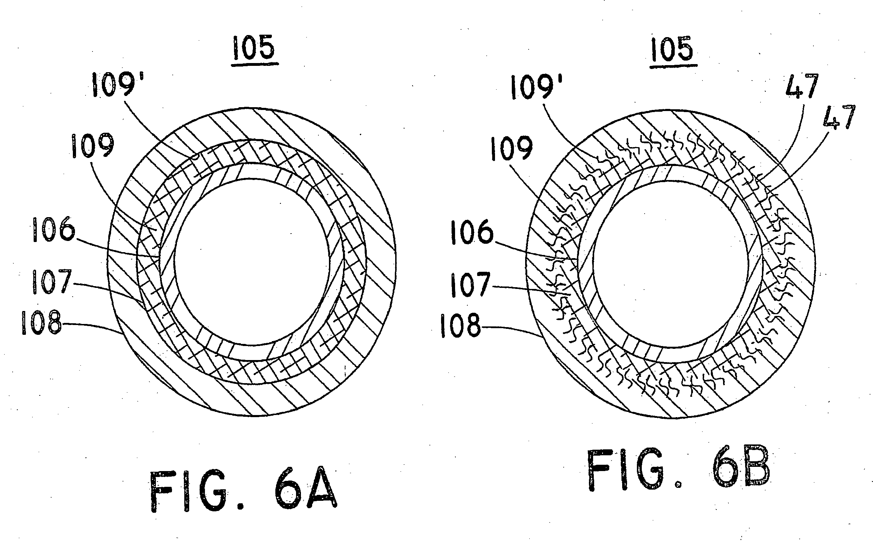

Figure 6 is a longitudinally sectioned view, broken away, of a distal portion of medical device delivery system.
Figures 6A and 6B schematically represent cross sectional views of melt bonding, where Figure 6A shows components before melt bonding while Figure 6B shows the components after melt bonding.

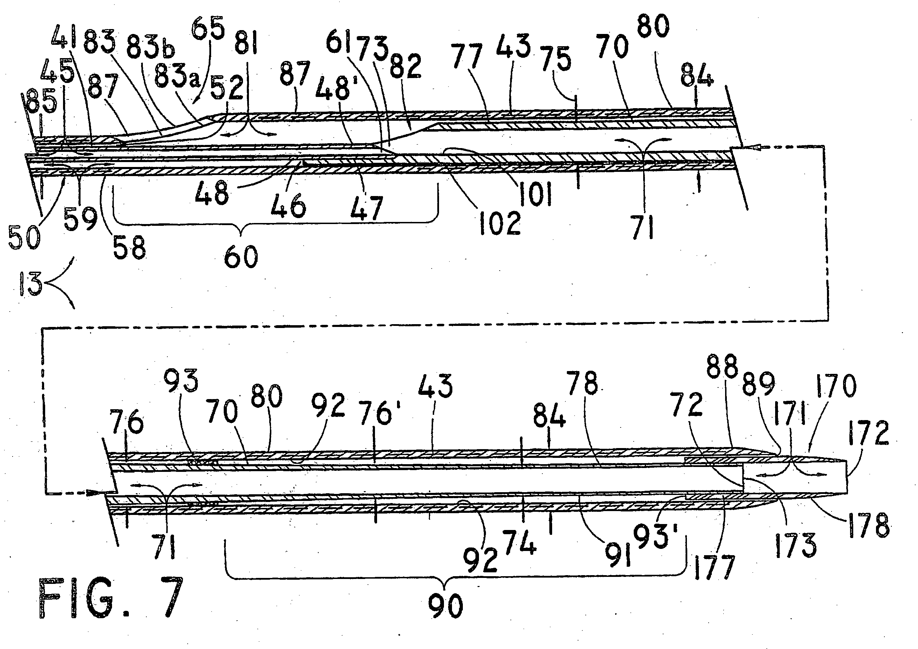

Figure 7 is a longitudinally sectioned view, broken away, of an alternative embodiment of a distal portion of a medical device delivery system.

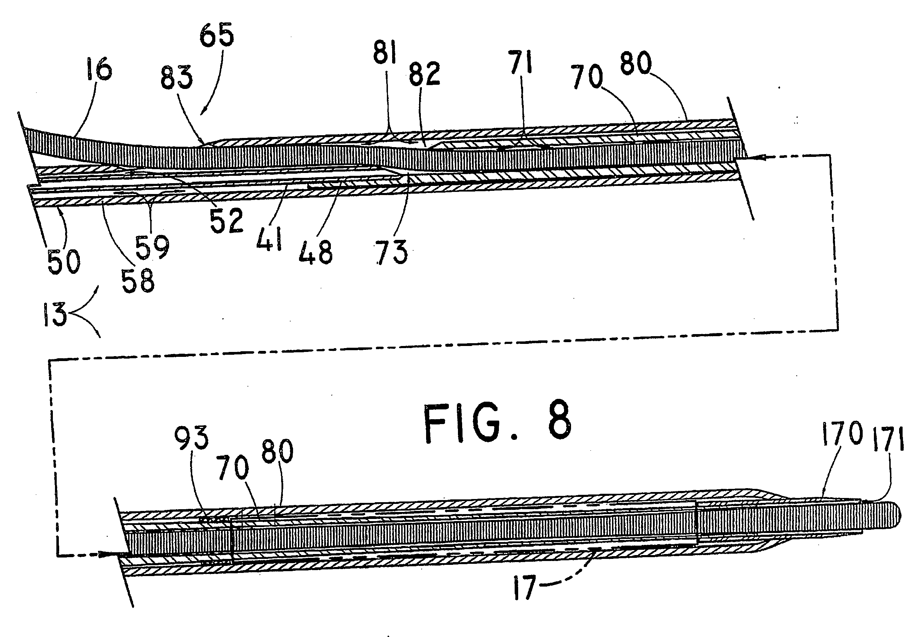

Figure 8 is a longitudinally sectioned view of an embodiment of a distal portion, shown having a portion of a wire guide.

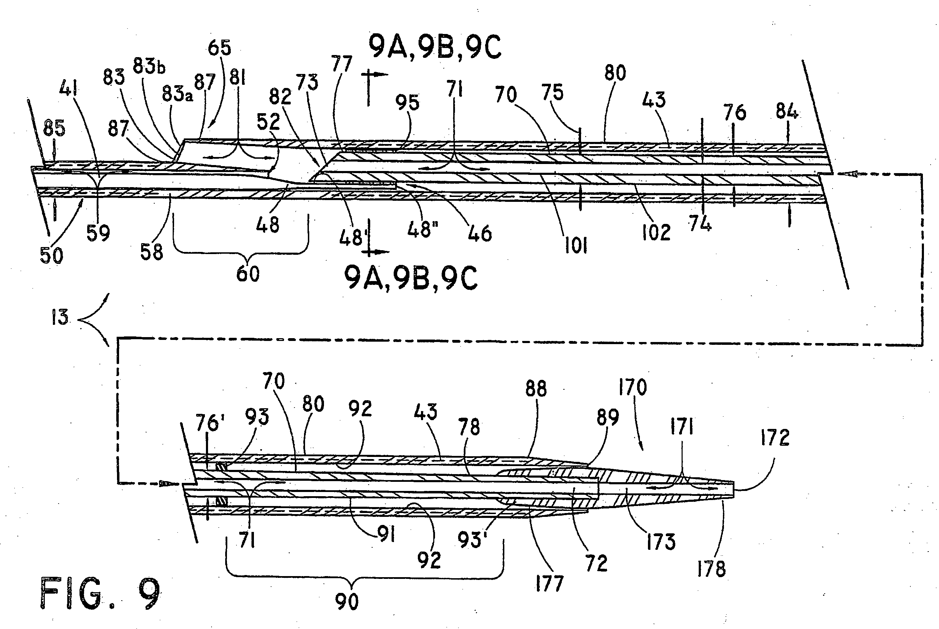

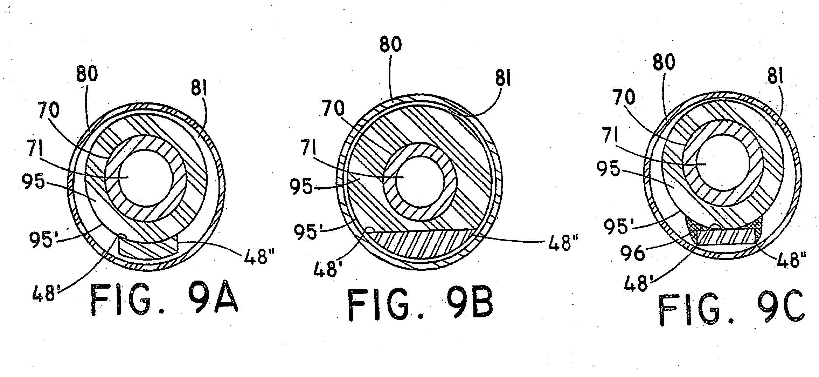

Figure 9 is a longitudinally sectioned view, broken away, of another embodiment of a distal portion of a medical device delivery system.
Figures 9A, 9B, and 9C show cross sectional views of Figure 9 taken along the lines 9A-9A, 9B-9B, and 9C-9C, respectively.

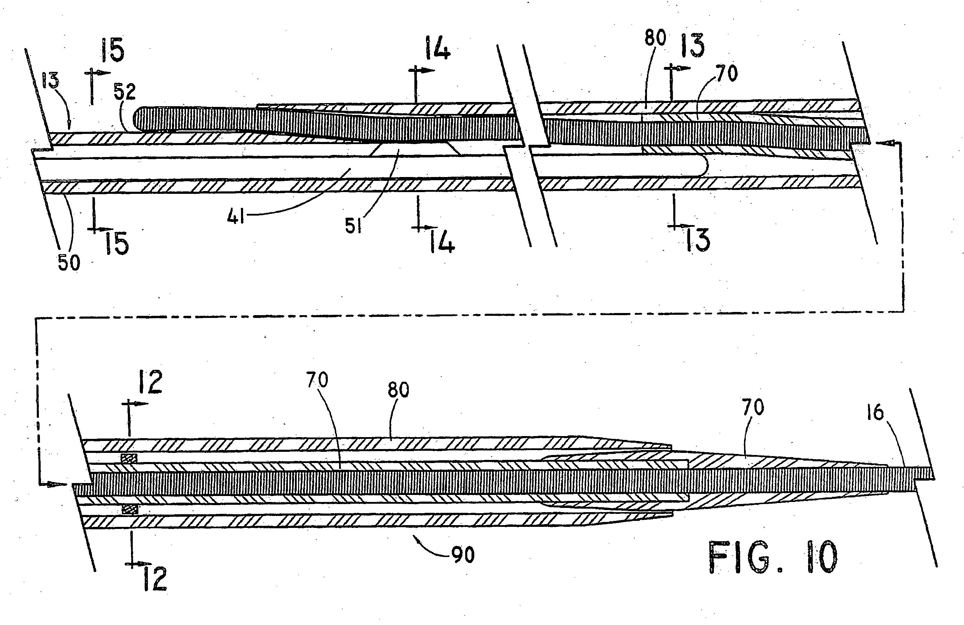

Figure 10 is a longitudinal sectional side view of an alternative embodiment of a distal portion of a device for the rapid insertion of self-expanding stents shown in an un-deployed delivery state.

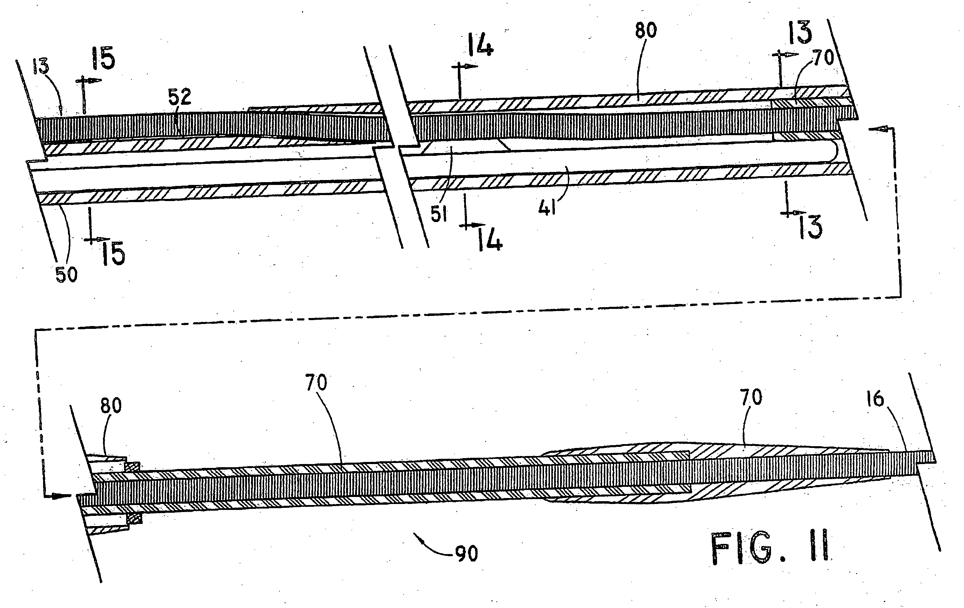

Figure 11 is a view of Figure 10 shown in one illustrative deployed delivery state.

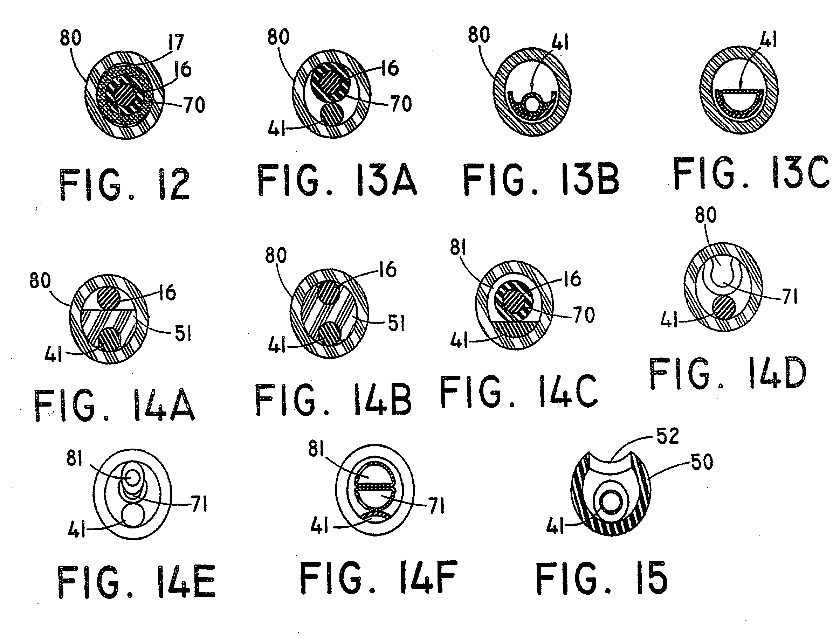

Figure 12 is a cross sectional view of Figure 10 taken along the line 12-12.
Figures 13A, 13B, and 13C are alternative embodiments of cross sectional views of Figures 10 and 11 taken along the line 13-13.
Figures 14A, 14B, 14C, 14D, 14E, and 14F are alternative embodiments of cross sectional views of Figures 10 and 11 taken along the line 14-14.
Figure 15 is one embodiment of a cross sectional view of Figures 10 and 11 taken along the line 15-15.

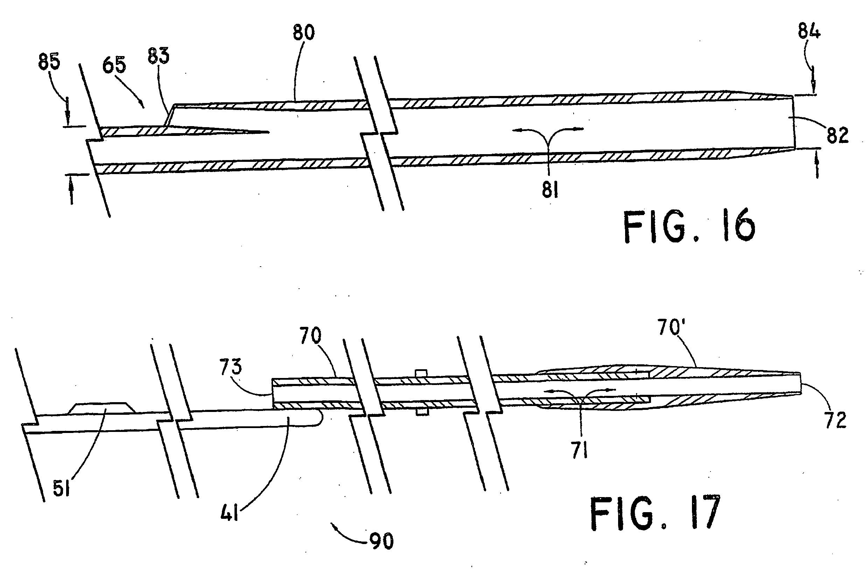

Figure 16 is a longitudinal schematic sectional side view, broken away, of a portion of an embodiment of an outer guide channel member according to Figure 10.
Figure 17 is a longitudinal schematic sectional side view, broken away, of a portion of an embodiment of an inner compression member and an inner guide channel member according to Figure 10.

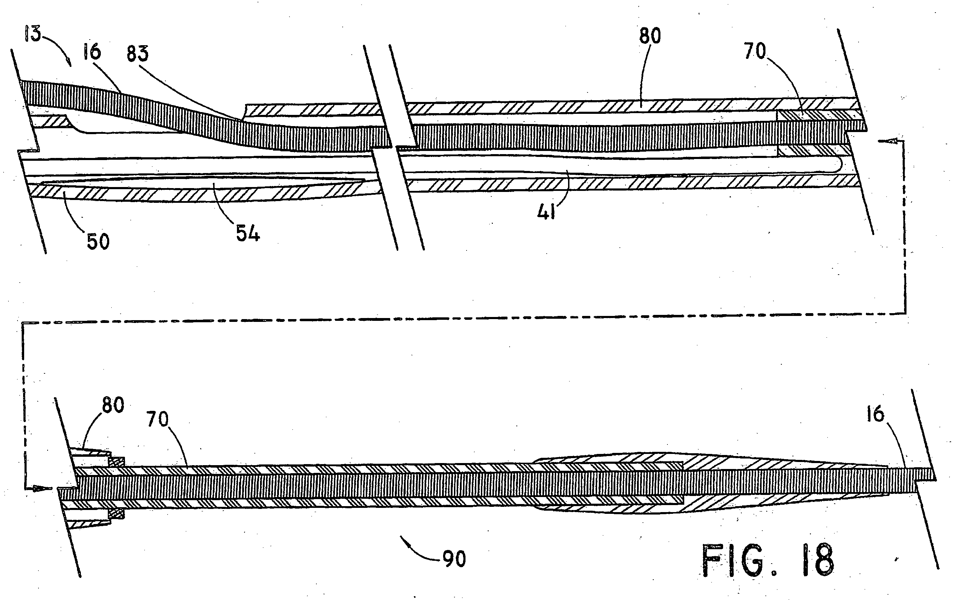

Figure 18 is longitudinal sectional side view of an alternative embodiment of a distal portion of a device for the rapid insertion of self-expanding stents shown in an illustrative deployed delivery state, and shown with a wire guide.

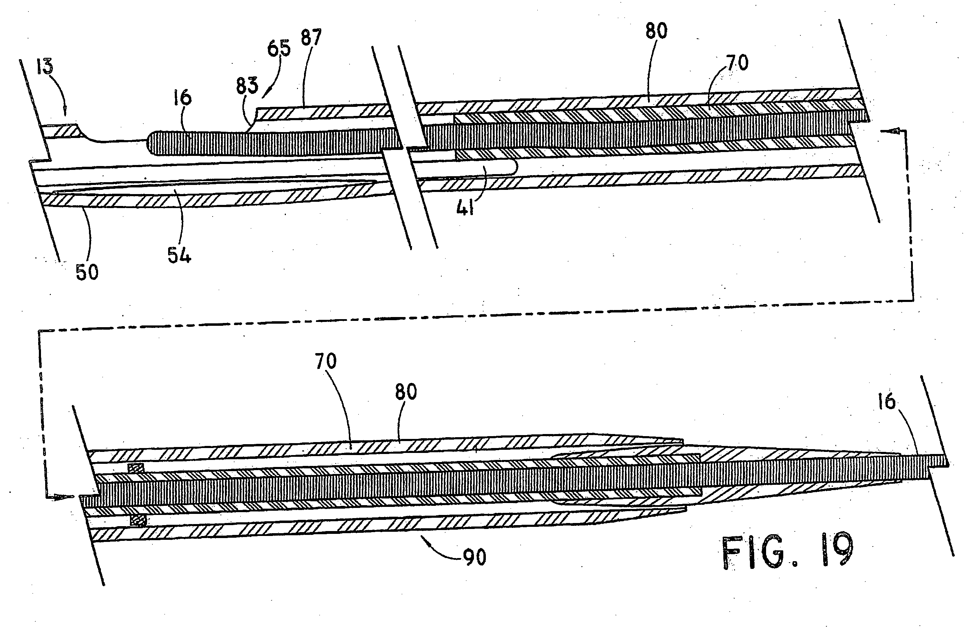

Figure 19 is an alternative view of Figure 18 shown in an un-deployed delivery state, and shown with a wire guide.

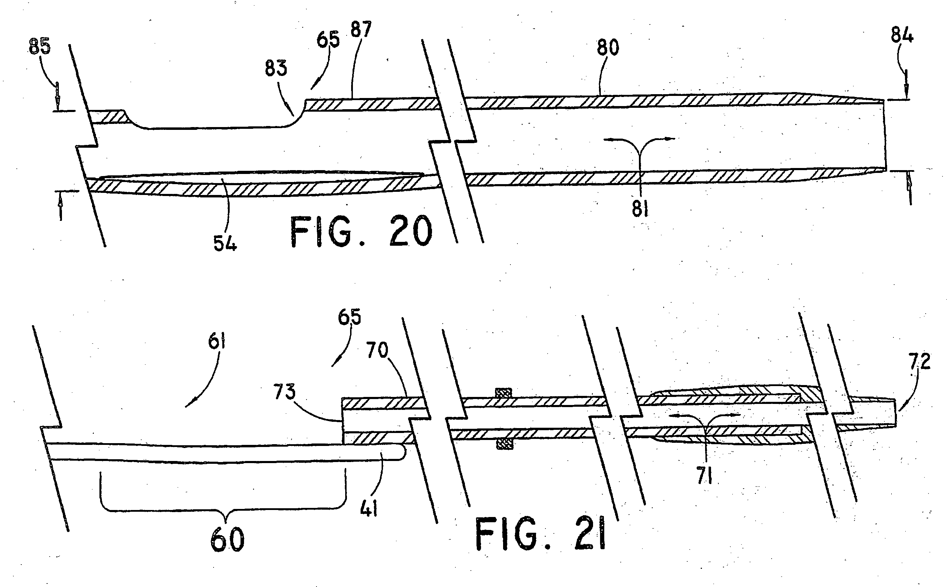

Figure 20 is a view of a portion of an embodiment of an outer guide channel member depicted in Figure 18.
Figure 21 is a longitudinal schematic sectional side view, broken away, of a portion of an embodiment of an inner compression member and an inner guide channel member according to Figure 18.

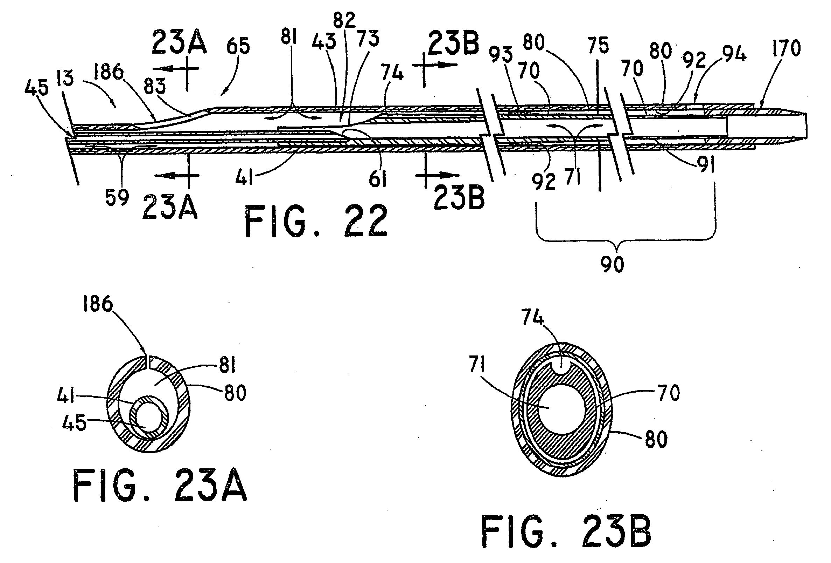

Figure 22 is a longitudinal sectional side view of an alternative embodiment of a distal portion of a medical device.
Figure 23A is a cross sectional view of one embodiment of Figure 22 taken along the line A-A.
Figure 23B is a cross sectional view of one embodiment of Figure 22 taken along the line B-B.

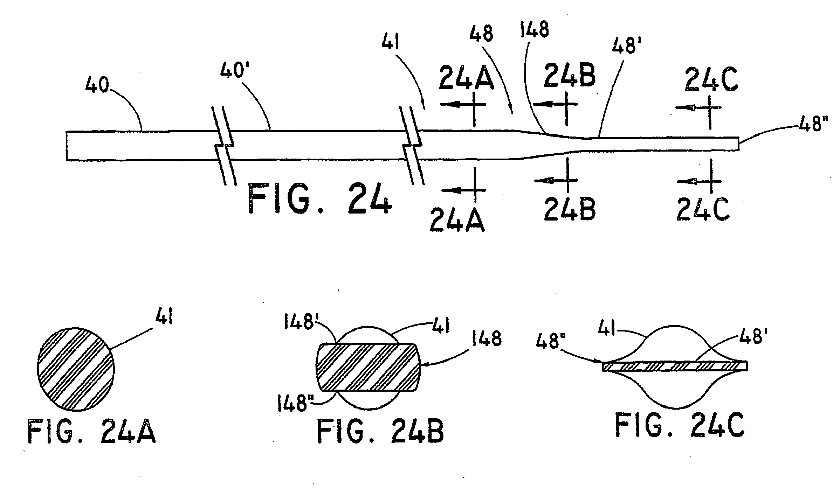

Figure 24 shows a schematic side view of an inner compression member.
Figures 24A, 24B, and 24C show cross sectional views of Figure 24 taken along the lines A-A, B-B, and C-C, respectively.

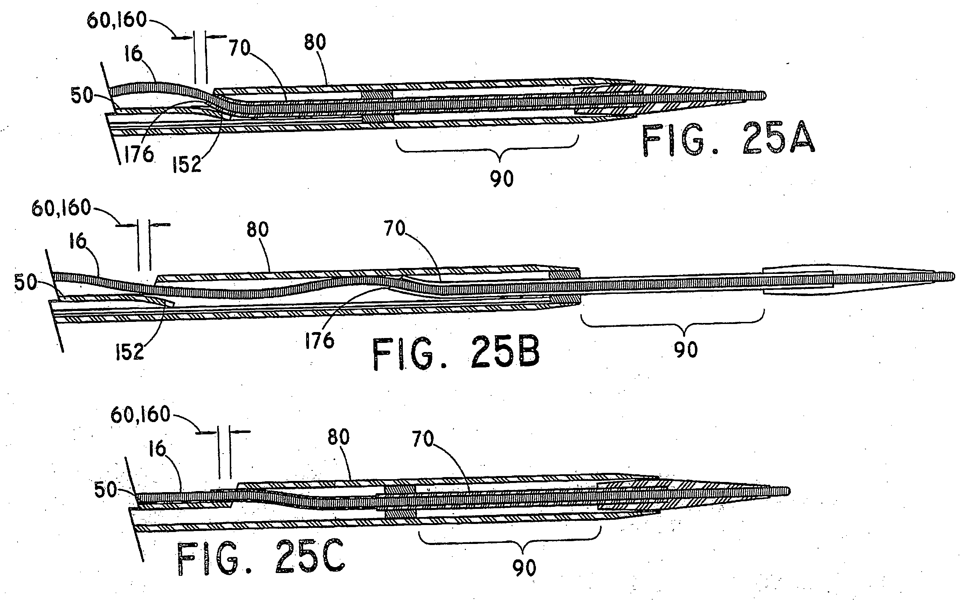

Figures 25A through 25C are longitudinal schematic sectional views, broken away, of alternative embodiments of a distal portion of a medical device.

### Detailed Description

The present invention relates to medical devices, and in particular to a delivery system configured for rapid insertion delivery of self-expanding devices such as stents, prosthetic valve devices, and other implantable articles inside a patient's body. For conciseness and ease of description of the embodiments of the invention, the term "stent" and its variations shall refer individually and collectively (without limiting the invention) to all self-expanding devices used with the invention, such as stents, prosthetic valve devices, and other implantable articles inside a patient's body.

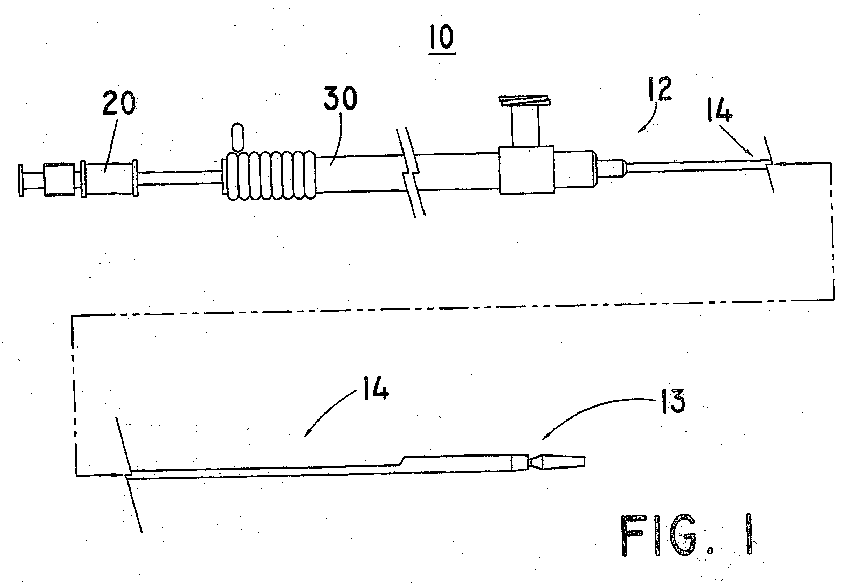

In Figure 1, an illustrative embodiment of a delivery system 10 having a host of uses, including for the rapid insertion of self-expanding stents, is provided. The delivery system 10 comprises a system proximal portion 12, a middle section delivery device 1,4, and a system distal portion 13-shown in a partially deploying position.

In the embodiment shown in Figure 1, the proximal portion 12 remains outside of the patient's body. The proximal portion 12 comprises a handle 30 and an optional pusher stylet 20.

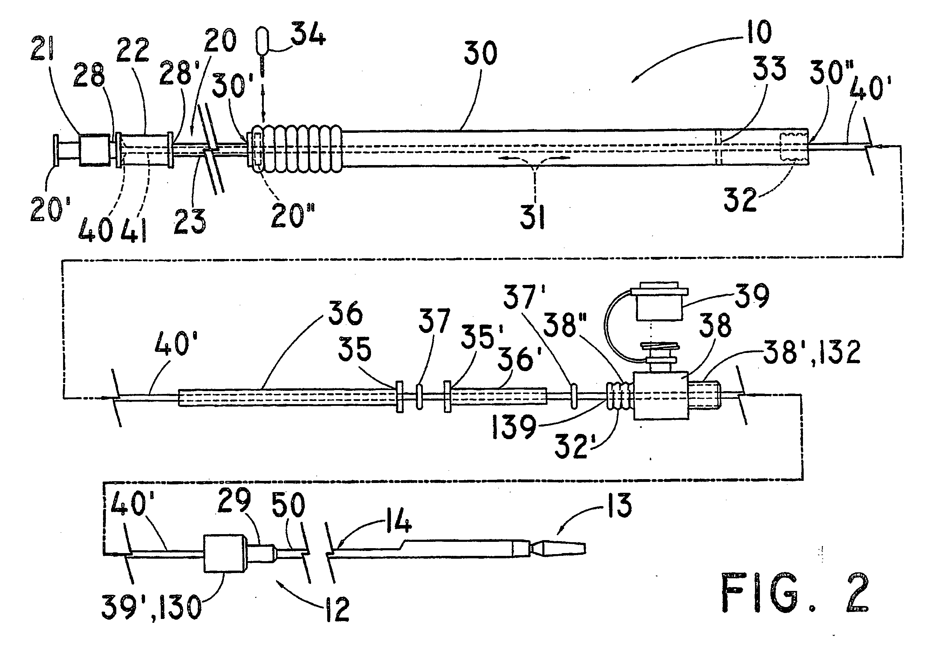

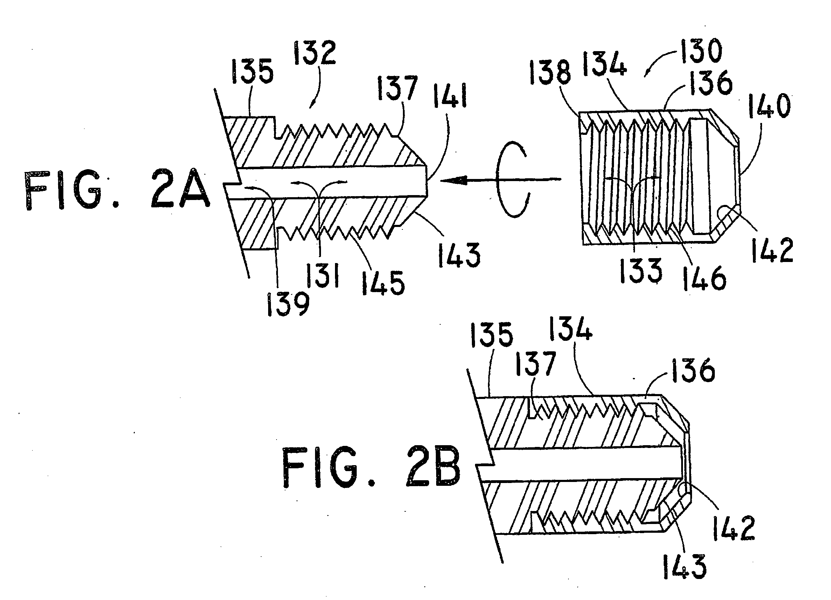

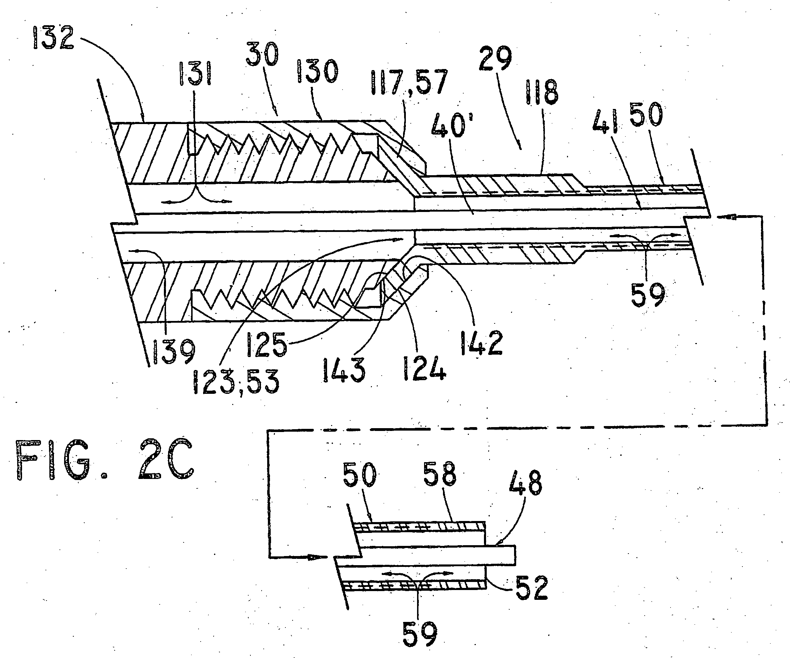

Figure 1 depicts a schematic representation of the handle 30 and the optional pusher stylet 20 shown more particularly in Figure 2. In general, a handle 30 retracts an outer guide channel member (discussed below) of the distal portion 13 of the delivery system 10 to deploy an implantable prosthesis such as self-expanding, balloon expandable, or non-expanding stents, prosthetic valve devices, and other implantable articles (individually and collectively, "stent," "stents," "implantable prosthesis," and "implantable prostheses") at a selected location inside a patient's body. The handle 30 may comprise any mechanical, electromechanical, pneumatic, or hydraulic handle configured in communication with-directly or indirectly through intervening parts-the distal portion's outer guide channel member. Communication would include, by way of illustration and not by way of limitation, a handle 30 that uses or is otherwise associated with, directly or indirectly, an elongated mechanical wire, rod, shaft, cable, sheath, pneumatic tube, or hydraulic pistons, cylinders and/or flow paths configured for moving the outer guide channel member proximally in order to deploy a stent.

Figure 2 provides a schematic view, broken away, of a delivery system 10 for rapid insertion of self-expanding stents, but could be used with other implantable prostheses described above. The delivery system 10 shown in Figure 2 is one embodiment of the proximal portion 12, middle section delivery device 14, and distal portion 13 shown in a partially deploying position. The middle section delivery device 14 extends distally from the proximal portion 12, and a distal portion 13 extends to a position that is distal the middle section delivery device 14. More particularly, Figure 2 shows an exploded view of the proximal portion 12 of the delivery system 10 according to one embodiment of the invention, with an emphasis on the handle 30 and the optional stylet 20. Features of one embodiment of a handle 30 and pusher stylet 20 are discussed below.

The handle 30 comprises any tubular structure having a distal aperture 30" and a proximal aperture 30', the apertures defining a chamber 31 therebetween. In general, the handle 30 is a component, instrument, mechanism, tool, device, apparatus, or machine configured for directly or indirectly retracting an outer guide channel member (discussed below) of the distal portion 13 of the device to expose and, ultimately, to deploy a stent at a selected location inside a patient's body.

The handle 30 is axially slideable relative to an elongate (long) inner compression member 41 that comprises a proximal end 40 and a middle section 40'. As discussed more fully below, the inner compression member 41 helps to keep the stent from moving proximally with proximal movement of the handle 30, which handle movement causes the outer guide channel member to withdraw proximally over the stent in order to expose and thereby to deploy the stent. Thus, the inner compression member helps to "push" the stent or stent carrying inner guide channel member in order to counter the urge for the stent or stent carrying member to prolapse proximally with the withdrawing of the outer guide channel member. As will be understood, "pushing" on the inner compression member will keep the stent carrying inner guide channel member (and therefore the stent) from translating as a result of an outer sheath or outer guide channel member being pulled over the stent; thereby "pushing" holds the stent in place at the desired deployment site within the patient's body. In one embodiment, the handle 30 is a unidirectional handle that is axially slideable relative to the inner compression member 41 and/or the optional pusher stylet 20 in order to deploy a stent. In one embodiment, the inner compression member 41 is secured to a pusher stylet 20.

As shown in Figure 2, one embodiment of a pusher stylet 20 comprises a proximal end 20', a distal end 20", and a cannula 23 intermediate the proximal and distal ends 20', 20", respectively, and a receptacle 22. The cannula 23, as should be understood, comprises any suitable hollow plastic or metal tube. As a hollow tube, the cannula 23 optionally allows the inner compression member 41 to pass proximally through the cannula 23 and to the proximal end 20' so that the inner compression member proximal end 40 (such as a flared proximal end 40) may secure to a plug 21 that fits within the receptacle 22, wherein Figure 2 shows the proximal end 20', plug 21, and an optional securing material 28 are shown in an exploded view relative to the receptacle 22 into which they may be secured. Furthermore, the cannula 23 assists with keeping that portion of the inner compression member substantially straight.

The stylet 20 is optional, because in an alternative embodiment the physician may hold the inner compression member proximal end 40' directly in order to "push" (e.g., hold substantially stationary) the stent carrying inner guide channel member (and therefore the stent). This controls the stent carrying inner guide channel member and stent from translating as a result of an outer sheath or outer guide channel member being pulled over the stent, so that the stent remains at the desired deployment site within the patient's body. Alternatively, the stylet 20 is any stationary handle secured to the inner compression member 41 for achieving the "pushing" (e.g., hold substantially stationary) of the stent or stent carrying inner guide channel member while the outer sheath or outer guide channel member are moved proximally.

The stylet distal end 20" is housed within the handle chamber 31 and is flared or otherwise flanged sufficiently to be larger than the handle proximal aperture 30' so as not to pull out of the chamber 31. In one embodiment, the stylet distal end 20" is secured to the distal portion of the stylet cannula 23, while in another embodiment the stylet distal end 20" is formed integral with the distal portion of the stylet cannula 23. Consequently, the stylet distal end 20" functions as a proximal stop that prevents the stylet cannula 23 from backing all the way out the handle while being axially slideable within the handle chamber 31. Thus, the stylet 20 will not slide off the handle 30, if so desired. The stylet distal end 20" may also, in one embodiment, function as a distal stop against a restraint 33 formed in the handle chamber 31 intermediate the handle proximal and distal apertures 30', 30", respectively, where intermediate should be understood to be any position between, and not necessarily equidistant to, the handle apertures 30', 30". As a result of the stylet distal end 20", the handle 30 may slide axially the distance separating the handle restraint 33 and the stylet distal end 20", which has a maximum distance of when the stylet distal end 20" is abutting the handle proximal aperture 30'.

A threaded tapered plug 21 and threaded tapered receptacle 22 optionally secure the inner compression member proximal end 40. In one embodiment, the inner compression member proximal end 40 is flared. Securing material 28, such as glue, adhesives, resins, welding, soldering, brazing, chemical bonding materials or combinations thereof and the like (collectively and individually, "glue") may be used to keep the threaded tapered plug 21 from backing out of the threaded tapered receptacle 22. A portion of the cannula 23 and stylet distal end 20" are received within the handle chamber 31 distal to the handle proximal aperture 30' as previously explained.

By optionally placing the inner compression member proximal end 40 in mechanical communication with the plug 21 and receptacle 22, the gripping and "pushing" (e.g., hold substantially stationary) on the stylet 20 (e.g., the receptacle 22) thereby helps to keep the inner compression member 41 from moving away from the distal portion 13 and, accordingly, counters the tendency for a stent or stent carrying member to move proximally during withdrawal of the outer guide channel member as will be explained below. Of course, the inner compression member may be secured elsewhere by the stylet 20, such as at or near the stylet distal end 20" or intermediate the stylet proximal and distal ends 20', 20", respectively, and the stylet distal end 20" may extend to a position at or near the distal end aperture 30" of the handle 30.

Figure 2 shows a middle section 40' that extends distally from the proximal end 40 of the inner compression member 41. In one embodiment, the middle section 40' passes through the handle 30 (and may pass through the cannula 23 and/or bushings housed within the handle chamber 31 or other portions of the proximal portion 12). In one embodiment, the middle section 40' is elongate (at least 50.0 cm or longer as described below) and extends to a distance distally of the handle 30 and to a position at or near the medical system delivery device distal portion 13. It should be understood that, by describing the middle section 40' as passing through the handle 30, the middle section 40' does not necessarily need to pass proximally through the entire length of the handle 30, such as in an embodiment (by way of example and not by way of limitation) where the proximal end 40 of the inner compression member 41 is secured to a distal portion of the cannula 23 and/or the stylet distal end 20" extending within the handle chamber 31 to a position at or near the handle restraint 33.

In addition to holding a threaded tapered plug 21 and optionally the proximal end 40 of the inner compression member 41, the threaded tapered receptacle 22 may secure the proximal portion of the optional cannula 23. Glue 28' may be used at or near an interface of the cannula 23 and distal aperture of the threaded tapered receptacle 22. The glue 28' serves many functions, such as to keep dust from settling within the threaded tapered receptacle 22, to make the cannula 23 easier to clean, and to give aesthetics and a smooth feel to the device.

The handle 30 slidably receives the distal portion of the cannula 23 within the handle aperture 30' and handle chamber 31. As a result, the handle 30 is slideable relative fo the stylet 20-(e.g., slideable relative to the threaded tapered plug 21, threaded tapered receptacle 22, and the cannula 23). In use, the physician grips the handle 30 in one hand and grips the stylet 20 (e.g., the receptacle 22) in the other hand. The physician holds the stylet 20 relatively stationary, which prevents the inner compression member and inner guide channel member and its stent carrying portion from moving proximally, and then withdraws the handle 30 proximally relative to the stationary stylet 20 and inner compression member 41. As a result, the physician is thereby retracting an outer guide channel member (discussed below) of the distal portion 13 of the delivery system 10 to expose and, ultimately, to deploy a stent locatable at the distal portion 13 of the delivery system 10. The handle 30 is in communication with-directly or indirectly through intervening parts-the outer guide channel member at the distal portion 13.

As shown in Figure 2, some of those optional parts may include the following: a first bushing 36 having an optional first bushing flange 35; a second bushing 36' having an optional second bushing flange 35'; an intermediate seal 37 intermediate the first and second bushing flanges 35, 35', respectively; a second seal 37' intermediate the second bushing flange 35' and a check flow body 38; and a detachable cap 39, such a Luer cap by way of example but not by way of limitation. In one embodiment, one or both of the intermediate seal 37 and the second seal 37' is from a class such as an O-ring. In another embodiment, one or both of the intermediate seal 37 and the second seal 37' is a cylinder or disk with a center aperture, and may be made from material that comprises an O-ring. The bushings 36, 36' are hollow plastic or metal tubes that take up space within the handle 30 so that the inner compression member has less room to buckle. Fully assembled in one embodiment, the first bushing 36 is inserted within the cannula 23 and the first bushing flange 35 is distal to and abutting the handle restraint 33, which is sized to interfere with the bushing flange 35 to prevent the bushing flange 35 from moving proximal to the handle restraint 33. The second bushing flange 35' is distal to and optionally abutting the bushing flange 35 so to prevent it from moving proximal the first bushing flange 35, and the second bushing 36' is inserted within an opening 139 of the check flow body 38. The intermediate seal 37 and the second seal 37' help to prevent fluids that could be used with the device (discussed below) from entering the handle chamber 31, which directs fluids distally, which fluids may be conveyed through an outer sheath 50 of the middle section delivery device 14 and system distal portion 13. In one embodiment, the handle restraint 33 is from a class such as a counter bore wherein the restraint 33 comprises, by way of example only and not by way of limitation, a flat-bottomed cylindrical enlargement of the handle chamber 31 sized for receiving a first bushing flange 35, an intermediate seal 37, a second bushing flange 35', and/or a check flow body proximal mating end 38" intermediate the restraint 33 and the handle distal aperture 30".

The handle 30 and check flow body 38 operatively couple with the handle distal aperture 30" receiving a check flow body proximal mating end 38" and being secured together by any suitable means, including but not limited to a crimp, friction fit, press fit, wedge, threading engagement, glue, adhesives, resins, welding (laser, spot, etc.), soldering, brazing, adhesives, chemical bonding materials, or combinations thereof. In one embodiment, the handle 30 comprises a coupling member 32 and the check flow body proximal mating end 38" comprises a coupling member 32', the coupling members 32, 32' being complementary to hold the handle 30 and check flow body proximal mating end 38" together. In one embodiment, the coupling members 32, 32' may form complementary threads. If it is desired to achieve quicker assembly for manufacturing purposes, then the coupling members 32, 32' may be an array of circumferential ridges that form an interference fit when pressed together. If a one-time snap fit is desired, then the coupling members 32, 32' may be circumferential ridges in the form of barbs. In another embodiment, the handle 30 and check flow body proximal mating end 38" may be put together and taken apart for servicing, in which case the coupling members 32, 32' may be circumferential ridges in the form of knuckle threads (e.g., circumferential ridges forming complementary undulating waves). The operatively coupled handle 30 and check flow body proximal mating end 38" according to these embodiments may be fixed such that they do not rotate relative to each other, or may rotate while preventing undesired axial separation.

During use, the detachable cap 39 may be detached or opened and the device flushed with saline to remove air in order to help keep air out of the patient. The intermediate seal 37 and the second seal 37' ensure that any flushed fluid moves distally in the device and does not back up into the handle 30, such as between the handle restraint 33 and the first bushing 36, into the handle chamber 31, or out the handle proximal aperture 30'. The detachable cap 39 (such as a Luer cap) keeps saline from backing out of the check flow body 38, air from flowing into the check flow body 38, and blood from rushing out during periods of high blood pressure inside the patient.

The medical device delivery systems 10 may be used to deploy an implantable prosthesis that is a balloon expandable or self-expanding stent, prosthetic valve device, or other implantable articles provided on the distal portion of a delivery system. In operation, a physician inserts the distal portion and at least a portion of the middle section delivery device into a vessel passageway, and advances them through the vessel passageway to the desired location adjacent the target site within the vessel passageway of a patient. In a subsequent step, the physician moves the handle proximally, which withdraws the outer sheath and/or the outer guide channel member and releasably exposes the stent for deployment. In another step, the physician inflates the expandable member, such as a balloon, positioned under the stent inner surface to plastically deform the stent into a substantially permanent expanded condition. The physician may inflate the expandable member by injecting fluid such as saline from a syringe into the inner compression member 41, via pusher stylet 20, through a Luer fitting at the proximal end 20'. Therefore, the fluid is directed distally to the expandable member, filling the expandable member chamber and expanding the stent. The physician then deflates the balloon and removes the catheter or delivery device from the patient's body.

In one embodiment as shown in Figure 2, the handle 30 further comprises a check flow body distal mating end 38' and a connector cap 39'

(optionally detachable) secured to the check flow body distal mating end 38', and a strain relief 29. In one embodiment, the connector cap 39' is from a class of fasteners such as nuts, and in one embodiment is a flare nut. The connector cap 39' functions to hold (or assist in holding in combination with the check flow body distal mating end 38') a flared proximal portion of an outer sheath 50 and/or a flared strain relief 29 disposed about (and optionally extending proximally from) that held portion of the outer sheath 50. The strain relief member 29 provides a kink resistant point where the outer sheath 50 connects to the connector cap 39' and/or the check flow body distal mating end 38'.

The check flow body distal mating end 38' and connector cap 39' may be operatively coupled mechanically, chemically, and/or chemical-mechanically. In one embodiment, the connector cap 39' is crimped, friction fitted, press fitted, and/or wedged into engagement onto the check flow body distal mating end 38'. In another embodiment for example, the check flow body distal mating end 38' and connector cap 39' are operatively coupled by glue, adhesives, resins, welding (laser, spot, etc.), soldering, brazing, adhesives, chemical bonding materials, or combinations thereof.

According to Figure 2A, yet another embodiment of the connector cap 39' comprises a handle first connector 130 and the check flow body distal mating end 38' comprises a handle second connector 132. According to Figure 2A, the handle first and second connectors 130, 132, respectively, function to operatively couple a strain relief member 29 operatively coupled to the proximal portion of the outer sheath 50 (discussed below). In one embodiment, the handle first connector 130 is from a class of fasteners such as nuts, and in one embodiment is a flare nut. Optionally, the distal portion of the second bushing 36' is sized (but for the second bushing flange 35') to be received within a check flow body proximal opening 139 in communication with the second connector 132.

Figure 2A shows an exploded longitudinally sectioned side view of one embodiment of a portion of the handle comprising a first connector 130 and a second connector 132. The handle first connector 130 further comprises a proximal portion 134 and a distal portion 136. An opening 138 at the proximal portion 134 and an opening 140 at the distal portion 136 and define a lumen 133 therebetween. There is an engaging surface 142 at or near the distal portion 136. A threaded first piece 146 is disposed within the lumen 133 and intermediate the handle first connector distal end opening 140 and proximal end opening 138. The handle second connector 132 further comprises a proximal portion 135 and a distal portion 137. An opening 141 at the distal portion 137 and check flow body proximal opening 139 (e.g., Fig. , 2) at the proximal portion 135 define a lumen 131 therebetween. There is an engaging surface 143 at or near the distal portion 137. A threaded second piece 145 is disposed on the outside surface and intermediate the handle second connector distal end opening 141 and the check flow body proximal opening 139.

According to one embodiment shown in Figures 2A and 2B, the second connector distal portion 137 is received within the first connector proximal end opening 138. The first connector 130 and second connector 132 are operatively coupled by a threading engagement between the first connector threaded first piece 146 and the second connector threaded second piece 145. Alternatively, the first connector 130 and second connector 132 are operatively coupled mechanically, chemically, and/or -chemical mechanically. In one embodiment for example, the first connector 130 and second connector 132 are crimped, friction fit, press fit, and/or wedged into engagement. In another embodiment for example, the first connector 130 and second connector 132 are operatively coupled by glue, adhesives, resins, welding (laser, spot, etc.), soldering, brazing, adhesives, chemical bonding materials, or combinations thereof.

Figure 2B shows the second connector threaded second piece 145 operatively coupled to the first connector threaded first piece 146 such that the second connector proximal portion 135 is proximal to the first connector proximal portion 134 and the second connector distal portion 137 is located at or near the first connector distal portion 136. As shown in Figure 2B, the second connector engaging surface 143 is spaced proximal to the first connector engaging surface 142 for receiving and compressing a strain relief member second end portion therebetween.

Figure 2C shows one embodiment of an optional strain relief member 29 comprising a tubular first end portion 118 and a flared second end portion 117. According to Figure 2C, the medical device delivery system includes an elongate outer sheath 50 (e.g., Figs. 2, 2C, 3, 4A-4G, 5M, 5R-5T, 6, 7, 8, 9, 10-11, 18-19, 25A-25D). Like elements from the previous drawings, embodiments, and description from above are labeled the same. The term elongate is used, not lexicographically but instead, to describe embodiments according to the embodiment that measures at least about 50.0 cm or measures within one of the ranges of lengths exceeding 50.0 cm and as more fully discussed above.

More particularly, Figure 2C shows that the outer sheath 50 comprises a proximal end portion 57 and a distal end portion 58. The distal end portion 58 comprises an opening 52 and the proximal end portion 57 comprises an opening 53; the openings define a passageway 59 therebetween. In one exemplary embodiment according to Figure 2C, the strain relief member tubular first and second end portions 118,117, respectively, are disposed about and operatively coupled to the outer sheath proximal end portion 157. In another embodiment, the tubular first end portion 118 disposes about the outer sheath proximal end portion 157 while the flared second end portion 117 extends proximally from outer sheath proximal end portion 157. In addition, the strain relief member second end portion 117 and/or outer sheath proximal end portion 57 comprise an opening 123 in fluid communication with the outer sheath passageway 59.

By way of example only and not by way of limitation, the terms "operatively coupling," "operatively coupled," "coupling," "coupled," and variants thereof are not used lexicographically but instead are used to describe embodiments of the invention having a point, position, region, section, area, volume, or configuration at which two or more things are mechanically, chemically, and/or chemical-mechanically bonded, joined, adjoined, connected, associated, united, mated, interlocked, conjoined, fastened, held together, clamped, crimped, friction fit, pinched, press fit tight, nested, wedged, and/or otherwise associated by a joint, a junction, a juncture, a seam, a union, a socket, a melt bond, glue, adhesives, resins, welding (laser, spot, etc.), soldering, brazing, adhesives, chemical bonding materials, implanted arrangement, or combinations thereof.

Figure 2C shows the strain relief member second end portion 117 and/or outer sheath proximal end portion 57 being operatively coupled between the first connector 130 and the second connector 132, and the second connector lumen 131 being in fluid communication with the outer sheath passageway 59. In one embodiment, the strain relief member second end portion 117 and/or outer sheath proximal end portion 57 comprises a first opposing surface 124 and a second opposing surface 125. The first connector engaging surface 142 is disposed against the first opposing surface 124 and the second connector engaging surface 143 is disposed against the second opposing surface 125, whereby the strain relief member second end portion 117 and/or outer sheath proximal end portion 57 becomes operatively coupled between the first and second connector engaging surfaces 142, 143, respectively. In one embodiment, the operatively coupled strain relief member second end portion 117 and/or outer sheath proximal end portion 57 is compressed (e.g., sandwiched) between the first and second connector engaging surfaces 142, 143.

Thus, the check flow body 38 provides an optional three way connector. The check flow body proximal mating end 38" and handle coupling member 32 are operatively coupled. The side port is controlled by the detachable connector cap 39. The body distal mating end 38' is operatively coupled to a second connector cap 39', or optionally the handle second connector 132 is received within and operatively coupled to a handle second connector cap 130.

The foregoing description of a proximal portion 12 of a medical device delivery system 10 according to one embodiment of the invention may be one assembly during shipping, or may include a two-part assembly or more. Otherwise stated, the stylet 20 and handle 30 may be sold already combined or may be combined after purchase by inserting the stylet cannula 23 into the handle at the hospital via the threaded tapered plug 21 and threaded tapered receptacle 22. An optional safety lock 34 helps to ensure against unintentional actuation by preventing distal movement of the stylet distal end 20" by extending inwardly within the handle chamber 30 through a slot in the handle outer wall distal to the handle proximal aperture 30'. Consequently, the optional safety lock 34 thereby maintains the handle 30 in an undeployed position until the physician is ready to deploy an implantable prosthesis (e.g., a self-expanding, balloon expandable, or non-expanding stent; prosthetic valve devices, and other implantable articles) at a selected location inside a patient's body.

### MIDDLE SECTION DELIVERY DEVICE

A delivery system 10 as shown in Figures 1 and 2 comprises a middle section delivery device 14. According to the invention, the middle section delivery device 14 is intermediate the proximal portion 12 (Figs. 1, 2) and the distal portion 13 (Figs. 1, 2) of the delivery system 10. The term "intermediate" is intended to describe embodiments of the invention whereby the middle section delivery device 14 is intermediary, intervening, lying or occurring between two extremes, or spatially in a middle position, state, or nature-though not necessarily equidistant-between the distal tip of the distal portion 13 and the proximal tip of the proximal portion 12. Furthermore, the middle section delivery device 14 may overlap or be partially inserted into a portion of the distal portion 13 and/or the proximal portion 12. In another embodiment, a portion of the middle section delivery device 14 (such as the sheath 50 explained below) and the distal portion outer guide channel member 80 (e.g., Figs. 5M, 6, 7, 8, 9, 10-11, 16, 18-20, 22, 25A-25D) may be an elongate tubular catheter or Flexor® sheath of integral construction.

According to the invention, a middle section delivery device 14 is a flexible, elongate (long, at least about 50.0 centimeters ("cm")) tubular assembly. In one embodiment, the middle section delivery device 14 is from approximately 100.0 centimeters ("cm") to approximately 125.0 cm for use when placing a distal portion 13 of the invention within a patient's body, although it may be sized longer or shorter as needed depending on the depth of the target site within the patient's body for delivering the stent. The term "tubular" in describing this embodiment includes any tube-like, cylindrical, elongated, shaft-like, rounded, oblong, or other elongated longitudinal shaft extending between the proximal portion 12 and the distal portion 13 and defining a longitudinal axis. As used herein and throughout to describe embodiments of the invention, the term "longitudinal axis" should be considered to be an approximate lengthwise axis, which may be straight or may at times even be curved because the middle section delivery device 14, for instance, is flexible and the distal portion 13 also may be substantially or partially flexible.

A middle section delivery device 14 comprises an outer sheath 50 (e.g., Figs. 2, 2C, 3, 4A-4G, 5M, 5R-5T, 6, 7, 8, 9, 10-11, 18-19, 25A-25D). Figure 2C shows that the outer sheath 50 is generally tubular and comprises a proximal end portion 57 and a distal end portion 58 and defining a passageway 59 therebetween (e.g., Fig. 2C). In one embodiment, the distal end portion 58 comprises an opening 52 and the proximal end portion 57 comprises an opening 53, which openings define the passageway 59. The middle section delivery device 14 further comprises an elongate inner compression member 41 (e.g., Figs. 2, 2C, 6, 7, 8, 9, 9A-9C, 10, 11, 17-19, 20, 22, 24-24B). The outer sheath passageway 59 is configured for slidably receiving the inner compression member 41, a catheter, or other medical device.

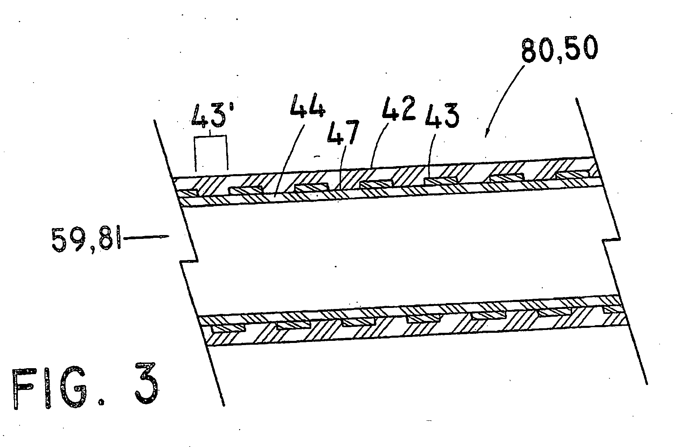

Figure 3 depicts an enlarged, longitudinally sectioned view along a partial length of one embodiment of an outer sheath 50 for use as the middle section delivery device 14, with the delivery system's proximal and distal portions 12,13, respectively, of the device being removed for clarity. In one embodiment, the outer sheath 50 comprises three layers: an inner layer 44 comprising Teflon material; a middle layer comprising a stainless steel circumferential spiral coil 43; and an outer layer 42 comprising a nylon, a polyether block amide ("PEBA"), and/or other melt bonding material discussed below. The outer layer 42 and inner layer 44 optionally may comprise a lubricious material, one example of which includes a fluorocarbon such as polytetrafluoroethylene (PTFE), to present a slideable surface to allow easier inserting and retracting the middle section delivery device 14 for deploying a self-expanding stent, as will be explained later.

The wall of the inner layer 44 of the outer sheath 50 has sufficient radial rigidity to decrease any tendency of bulging, kinking, and the like under an internal radial expansile force. In other words, the inner layer 44 resists an inner object from protruding or becoming embedded into the inner layer 44, which is beneficial to the slideability of an outer sheath 50. The coil 43 may be compression fitted or wound around the inner layer 44. The coil 43 includes a plurality of turns, and preferably includes uniform spacings 43' between the turns of the coil 43. The coil 43 may be formed of any suitable material that will provide appropriate structural reinforcement, such as stainless-steel flat wire or biologically compatible metals, polymers, plastics, alloys (including super-elastic alloys), or composite materials that are either biocompatible or capable of being made biocompatible.

Although the embodiment in Figure 3 shows a flat ribbon shaped wire coil 43, coils of other cross-sectional dimensions, such as round wire, may also be used. When flat wire stainless steel is used, the coil 43 is optionally formed from wire that is about 0.008 cm (0.003 inches) thick by about 0.03 cm (0.012 inches) wide. In one embodiment, the turns of coil 43 are uniformly spaced 43' apart by approximately 0.03 cm (0.0118 inches). While Figure 3 shows an embodiment that uses coils 43 having uniformly spaced turns and a constant pitch, this is not required and coils 43 may be spaced 43' by non-uniform distances or at varying distances. In one embodiment, the ends of coil 43 are positioned approximately 0.500 cm (0.197 inches) proximal to the distal portion 13 and approximately 1.501 cm (0.591 inches) distal to the proximal portion 12.

The outer sheath 50 for use with the middle section delivery device 14, and the outer guide channel member 80 (e.g., Figs. 5M, 6,7, 8, 9, 10-11, 16, 18-20, 22, 25A-25D) and/or the inner guide channel member 70 (e.g., Figs. 6, 7, 8, 9, 10-11, 17-19, 21, 22, 25A-25D) for use with the distal portion 13, are available for purchase from Cook Incorporated, of Bloomington, Indiana under the trade name of "Flexor®." Examples of the Flexor® sheath devices, materials, and methods of manufacturing them are found in United States Patent Nos. 5,700,253 and 5,380,304. The Flexor® sheath is particularly suited for the outer sheath 50 of the middle section delivery device 14 and/or the outer guide channel member 80 of the distal second end portion 13 due to its thin PTFE liner on the inside wall of the inner layer 44, thin flat wire coil 43, and Nylon and/or PEBA overcoat 42 that captures the coil 43 and PTFE liner 44 and binds the structure together. The PTFE inner layer 44 of the Flexor® sheath resists an expansile inner object from protruding or becoming embedded into the inner layer 44 and, thereby, provides a slick, smooth surface that slides (e.g., across the surface of a stent if the Flexor® sheath is used with the distal portion 13 or across the surface of an inner compression member 41 if the Flexor® sheath is used with the middle section delivery device 14) relatively easily when retracted to expose, release, and deploy the stent or allow the outer sheath 50 to move relative to the inner compression member 41, and the outer guide channel member 80 to move relative to the inner guide channel member 70, during deployment of the stent.

As an alternative to purchasing the outer sheath 50 for use with the middle section delivery device 14 and the outer guide channel member 80 for use with the distal portion 13 from Cook Incorporated, one may manufacture the outer sheath and outer guide channel member from various component parts. For instance, one may purchase a tubular inner layer 44 comprising a lubricious material comprising a fluorocarbon such as polytetrafluoroethylene (PTFE or Teflon) from Zeus, Inc. in Orangeburg, South Carolina, and dispose that inner layer 44 over a mandrel. Alternatively, a sheet of material comprising Teflon may be positioned on a mandrel and formed into a tubular body for the inner layer 44 by any suitable means known to one skilled in the art.

The tubular inner layer 44 (whether formed from a sheet on a mandrel or purchased as a tube and slid onto a mandrel) may be slightly longer than the desired length described above for the outer sheath 50 and/or outer guide channel member 80, and slightly longer than the mandrel. In one embodiment, the tubular inner layer 44 may extend about 5.0 cm from each mandrel end. As explained below, the "loose" ends of the tubular inner layer 44 help during manufacturing of the device.

The mandrel-tubular inner layer 44 assembly is prepared for a middle layer comprising a stainless steel circumferential spiral coil 43 as described above and available for purchase from Cook Incorporated or Sabin Corporation in Bloomington, Indiana. As purchased, the coil 43 comes in a long, pre-coiled configuration and will be cut by hand or machine to the desired length either before or after winding the coil about the inner layer 44 to the desired length. As an alternative, one may manufacture the coil from raw material available from Fort Wayne Medical in Fort Wayne, Indiana, and process it into a spiral coil 43 shape.

The operator may apply the spiral coil 43 about the mandrel-tubular inner layer 44 assembly by hand or machine. If by hand, then an end of the spiral coil 43 may be started onto the tubular inner layer 44 by any suitable means, for example, such as hooking and winding (e.g., wrapping) the coil 43 around the tubular inner layer 44 in a pigtailed manner at an initial position a desired distance (e.g., 5.0 cm or more) from a first end of the tubular inner layer 44 and to a terminating position that is a desired distance (e.g., 5.0 cm or more) from a second end of the tubular inner layer 44, and then cutting the coil 43 at the terminating position before or after hooking the coil 43 onto the inner layer 44. If by machine, then chucks, for instance, may hold the opposing ends of the mandrel-tubular inner layer 44 assembly while the spiral coil 43 is threaded through an arm on a machine and started onto the tubular inner layer 44 at the initial position as described above. As the chucks rotate, the inner layer 44 rotates, and the arm moves axially down the length of the inner layer 44, thereby applying the coil 43 in a spiral configuration about the inner layer 44. The machine arm moves to a terminating position where the machine or operator cuts the coil before or after hooking the coil 43 onto the inner layer 44.

An operator then applies an outer layer 42 about the coil-inner layer-mandrel assembly. The outer layer 42 may comprise a polyether block amide, nylon, and/or a nylon natural tubing (individually and collectively, "PEBA" and/or "nylon"). The outer layer 42 preferably has a tubular configuration that disposes about (e.g., envelaping, surrounding, wrapping around, covering, overlaying, superposed over, encasing, ensheathing, and the like) a length of the coil-inner layer-mandrel assembly.

Heat shrink tubing, available from many suppliers, including Zeus, Inc. in Orangeburg, South Carolina for instance and also Cobalt Polymers in Cloverdale, California, may be disposed about the outer layer-coil-inner layer-mandrel assembly. Heating the assembly causes the outer layer 42 to melt. The inner surface of the outer layer 42 thereby seeps through spaces 43' in or between middle layer coils 43 and bonds to both the outer surface of the inner layer 44 and the coils 43. In one embodiment, the inner surface of the outer layer 42 forms a melt bond 47 (explained below) to the outer surface of the inner layer 44. Upon cooling, a solid-state bond results such that the assembly comprises the three layers discussed above. The operator removes the shrink wrap (e.g., by cutting) and withdraws the mandrel. The operator may cut the Flexor® sheath to a desired length for an outer sheath 50 and/or outer guide channel member 80.

The temperature, total rise time, and dwell time for the heat shrink-outer layer-coil-inner layer-mandrel assembly will vary depending on many factors including, for instance, the actual melt bonding material that the outer layer 42 comprises, and also the diameter of the desired Flexor® sheath. For example, the baking parameters for a 2.5 French Flexor® sheath may be approximately 193°C (380 degrees Fahrenheit) for about five minutes, while the baking parameters for a 4 French Flexor® sheath may be approximately 193°C (380 degrees Fahrenheit) for about six minutes.

In addition to the outer sheath 50, the middle section delivery device 14 further comprises an inner compression member 41. The delivery device 14 (and, thus, the outer sheath 50 and inner compression member 41) may be constructed to have any diameter and length required to fulfill its intended purposes.

The outer sheath 50, for instance, may be available in a variety of lengths, outer diameters, and inner diameters. In one embodiment, the outer sheath 50 may have a substantially uniform outer diameter in the range from approximately 2 French to approximately 7 French, and in one embodiment the diameter is from approximately 4 French to approximately 5 French in diameter. Otherwise stated, the outer sheath 50 may range from about 0.025 cm (0.010 inches) to about 0.229 cm (0.090 inches) in diameter, and in one embodiment the diameter is approximately 0.127 cm (0.050 inches). Likewise, the passageway 59 may be available in a variety of diameters. In one embodiment, the inner diameter ranges from about 0.081 cm (0.032 inches) to about 0.102 cm (0.040 inches) and in a preferred embodiment the passageway 59 is approximately 0.081 cm (0.032 inches). The diameter may be more or less than these examples, however, depending on the intended vessel passageway for the device. For instance, a larger vessel passageway (e.g., greater expandable inner diameter) may tolerate a bigger device with an outer sheath 50 having a correspondingly greater diameter. Conversely, a narrower vessel passageway may require a thinner outer sheath 50. Likewise, the overall length may vary. In one embodiment, the outer sheath 50 will have a length from about 50.0 cm (or about 19.685 inches) to about 125.0 cm (or about 49.213 inches), and more particularly between about 70.0 cm (or about 27.559 inches) and about 105.0 cm (or about 41.339 inches), and in yet another embodiment the length is approximately 100.0 cm (or about 39.370 inches).

The inner compression member 41 comprises an elongated pusher bar, stiffening member, or stiff polymer that helps to "push" the stent by pushing the stent carrying inner guide channel member at or near the distal portion 13 in order to counter the urge for the stent or stent carrying member to move as a result of an outer sheath or outer guide channel member being pulled over the stent; thereby "pushing" holds the stent in place at the desired deployment site within the patient's body. The inner compression member 41 "pushes" the stent by helping to prevent or minimize the inner guide channel member from prolapsing, recoiling, kinking, buckling, or moving; thereby keeping the inner guide channel member's stent platform on which the stent is disposed (discussed later) substantial stationary, for the most part, relative to the proximal retraction of the distal outer guide channel member (discussed below) that exposes and, thus, deploys the stent. The phrase "at or near" as used herein to describe an embodiment of the invention includes a location that is at, within, or a short distance such as about 0.1 cm to about 15.0 cm, although other ranges may apply, for instance from about 0.5 cm to about 10.0 cam.

The overall length of the inner compression member 41 may vary, as desired. In one embodiment the inner compression member 41 has a length from about 50.0 cm to about 175.0 cm, and more particularly between about 75.0 cm and 150.0 cm, and in one embodiment the length is approximately 125.0 cm to about 140.0 cm. A portion of the inner compression member 41 (e.g., the proximal end 40 and/or middle section 40') may be contained within the handle 30 and the stylet 20, as explained above (Fig. 2).

Likewise, the diameter or width of the inner compression member 41 may vary. In one embodiment, the inner compression member 41 has a diameter or width ranging from about 0.025 cm (0.010 inches) to about 0.076 cm (0.030 inches), by way of example only and not by way of limitation. In one embodiment, the inner compression member 41 has a diameter or width that is approximately 0.041 cm (0.016 inches). The diameter or width may be more or less than these illustrative ranges. For example, a deeper target site within a patient may require a thicker inner compression member 41 for greater pushability, but may tolerate lesser flexibility. In addition, the material that the inner compression member 41 comprises determines whether a smaller and more flexible inner compression member 41 will give suitable flexibility, and also determines whether a wider inner compression member 41 may have the flexibility of a thinner inner compression member 41 made of different material. Furthermore, the inner compression member 41 may have a curved transverse cross-section, such as, for example, a circular cross-section, or it may have a polygonal cross-section, such as, for example, a rectangular cross-section. Alternatively, the transverse cross-section of the inner compression member may include both curved and straight portions. According to one embodiment, the inner compression member 41 may have a nonuniform diameter or width along its length. These various diameters, widths, and cross-sections may occur at the inner compression member proximal end 40, the inner compression member middle section 40', and/or the inner compression member distal mating end portion 48. It should be understood that the diameter, width, and/or cross-section of the inner compression member 41 may taper.

Also, an inner compression member 41 may have an outer surface comprising a lubricious PTFE material and/or an inner surface 44 of the outer sheath 50 may comprise a lubricious PTFE material against the inner compression member 41, in order to allow easy retraction of the outer sheath 50, which is in communication with a distal outer guide channel member to deploy a self-expanding stent, as will be explained later.

Generally, the inner compression member 41 and outer sheath 50 may optionally be approximately the same in length, and the axial length of coil 43 will be less than the length of the inner compression member and outer sheath. In one embodiment, however, the inner compression member 41 comprises a proximal end 40 that extends proximal relative to the outer sheath. In yet another embodiment, the inner compression member extends to a position that is distal the outer sheath. In still another embodiment, the inner compression member 41 stops short of extending all the way to the distal tip of the delivery system 10, and may stop generally from 10 to 40 cm short of the distal tip of the delivery system 10, and in one embodiment it stops approximately 20 to 25 cm -short of the distal tip of the delivery system 10, where the distal end portion of the inner compression member 41 is operatively coupled to a proximal portion of an inner guide channel member.

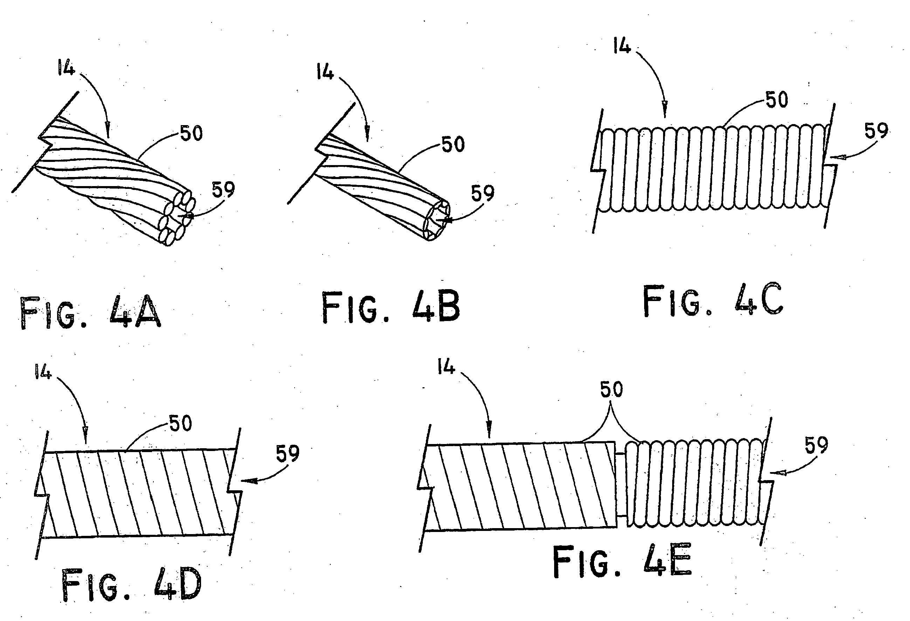

As an alternative to a Flexor® sheath discussed above, the outer sheath 50 may have a construction comprising multifilar material, round wire, cable tubing, spiral cut cannula, cable tubing connected to spiral cut cannula, round wire braid, flat braid, and any equivalents thereto and/or combinations thereof. Figures 4A through 4G are schematic views of alternative embodiments of an outer sheath 50 for use as the middle section delivery device 14, with the delivery system's proximal and distal portions 12, 13, respectively, of the device being removed for clarity.

In particular, Figures 4A and 4B illustrate alternative embodiments of an outer sheath 50 comprising a construction of multifilar material. The multifilar material is illustrated as tubular but could also be other shapes, such as plastic or metal cables or wires that are flat, rectangular, and the like. Such multifilar material or tubing may be obtained, for example, from Asahi-Intec USA, Inc. (Newport Beach, California). Materials and methods of manufacturing a suitable multifilar tubing are described in Published United States Patent Application 2004/0116833 (Koto et al.) having an Application Serial No. 10/611,664 and entitled, "Wire-Stranded Hollow Coil Body, A Medical Equipment Made Therefrom and a Method of Making the Same". Use of multifilar tubing in a vascular catheter device, for instance, is described in United States Patent No. 6,589,227 (Sonderskov Klint, et al.; Assigned to Cook Incorporated of Bloomington, Indiana and William Cook Europe of Bjaeverskov, Denmark).

The middle section delivery device 14 of Figures 3 and 4A-4G may taper distally to enhance flexibility in several ways. For example, flexibility increases by decreasing the outer diameter of the outer sheath 50. The portion of the outer sheath 50 having a smaller diameter is more flexible than the portion having a larger diameter. Such tapering also decreases the thickness of the outer sheath wall. A grinding process or other suitable process is used to reduce the outer diameter, as shown in Figure 4B. Alternatively, tapering may be used within the internal diameter of the outer sheath 50, enhancing flexibility by decreasing wall thickness without altering the outer diameter. A milling process or other suitable process is used to reduce the wall thickness without altering the outer diameter. The steepness and location of the tapering is determined by the desired application of the delivery system 10. For example, use of the device in coronary arteries will typically benefit from a smaller outer diameter, compared to use of the device in a bile duct, both for gross size and flexibility.

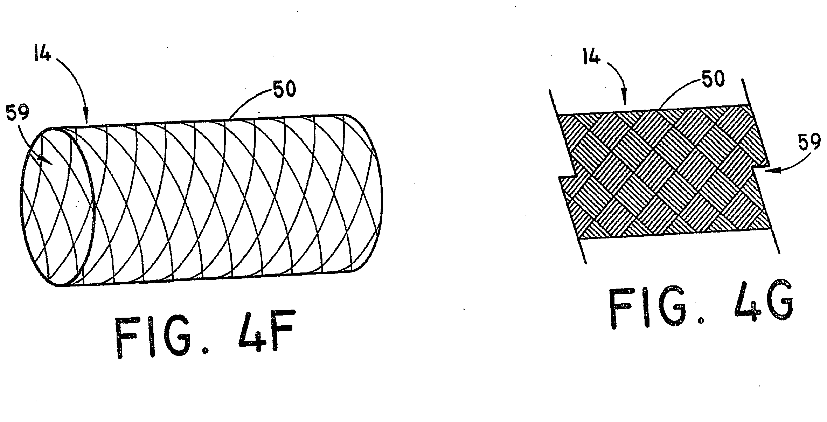

Figures 4C through 4E show alternative constructions of the outer sheath 50. While the multifilar materials of Figures 4A-4B are side-by-side and extend approximately longitudinally, Figure 4C shows an outer sheath 50 that is a wrapped round coil of an approximate perpendicular pitch relative to the longitudinal axis outer sheath 50. Figure 4D shows an outer sheath 50 that is a wrapped cable tubing or spiral cut cannula wherein the coil, compared to Figure 4C, has a more acute pitch relative to the longitudinal axis of the outer sheath 50. Figure 4E shows that various constructions can be combined for an outer sheath 50, such as one section constructed of the wrapped round coil of Figure 4C and one section constructed of the cable tubing/spiral cut cannula of Figure 4D. Other embodiments of an outer sheath 50 comprise a plurality of round wire braids (e.g., strands) of suitable material weaved as in Figure 4F or a flat braided construction as in as in Figure 4G. The term "plurality," as used herein and throughout to describe embodiments of the invention, means "two or more."

According to the embodiments of the outer sheath 50 shown in figures 3, 4A, 4B, 4C, 4D, 4E, 4F, and/or 4G may comprise a drug eluting coating. This coating may comprise a trerapeutic agent. Types of agents may be active, such as a medicine utilized in the medical procedure to, for example, assist with the healing process, assist with reducing bacterial count, and otherwise assist with delivering medication. Other types of agents may comprise inactive coatings, such as AQ® hydrophilic. Still other agents may comprise biodegradable polymers that are active, inactive, or polymeric.

### OPERATIVELY COUPLING THE MIDDLE SECTION DELIVERY DEVICE 14 AND THE DISTAL PORTION 13

Against the foregoing description of embodiments of the proximal portion 12 and middle section delivery device 14 and before discussing embodiments of the distal portion 13 of the delivery system, the invention further comprises associating the distal portion 13 and the middle section delivery device 14. These ways of operatively coupling the middle section delivery device 14 and distal portion 13 allow the outer guide channel member 80 of the distal portion 13 to move relative to the inner guide channel member 70 of the distal portion 13. In particular, the outer sheath 50 of the middle section delivery device 14 may be operatively coupled with the outer guide channel member 80 of the distal portion 13. Also, the inner compression member 41 of the middle section delivery device 14 may be operatively coupled with the inner guide channel member 70 of the distal portion 13. Therefore, the ways of constructing or joining the middle section delivery device 14 and distal portion 13 as described below are merely illustrations of non-limiting embodiments only.

In one embodiment, outer guide channel member 80 of the distal portion 13 and the outer sheath 50 of the middle section delivery device 14 may be constructed as comprising an integral unit. In other words, the same outer body sheath 50 construction shown and described in connection with Figures 4A-4G may comprise the outer sheath 50 of the middle section delivery device 14 as labeled, but it may also comprise an integral construction with the outer guide channel member of the distal portion.

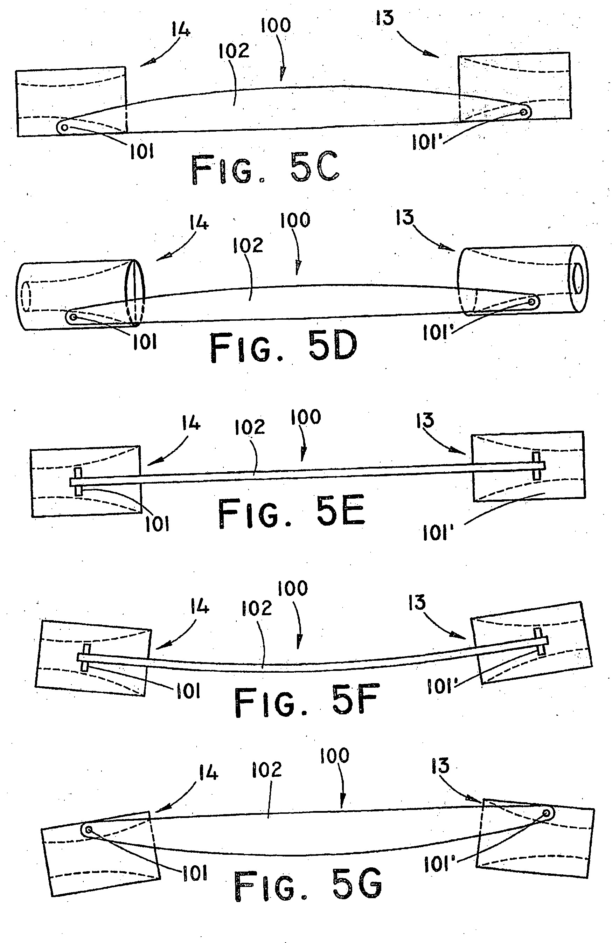

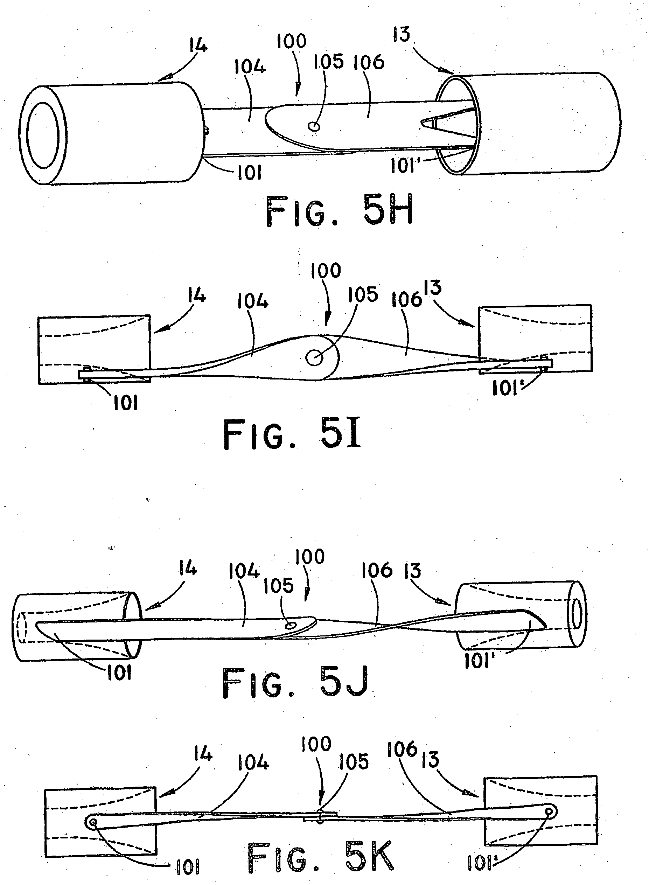

Figure 5 shows an alternative embodiment of an integral construction of the outer sheath 50 of the middle section delivery device and the outer guide channel member 80. In one embodiment, the outer sheath 50 and the outer guide channel member 80 comprise a Flexor® sheath or equivalent device shown in Figure 3 and described above. While Figure 5 shows an outer sheath and outer guide channel member 50, 80, respectively, having a coil 43, however, these outer bodies could use any other construction shown in Figures 4A-4G or combination thereof.

Moreover, where coil, multifilar material, weave, or braid (as described in the context of Figures 4A through 4G) is used in combination or individually, each may be continuous along the length of the outer sheath 50 of the middle section delivery device 14 and outer guide channel 80 of the distal portion 13 in one embodiment. In an alternative embodiment, a plurality of coils, multifilar materials, weaves, or braids may be joined end-to-end by a lap joint, brace, or other suitable means along the length of the outer sheath 50 of the middle section delivery device 14 and outer guide channel 80 of the distal portion 13. In a further alternative embodiment, a plurality of coil, multifilar material, weave, or braid may comprise a plurality of segments as where each segment is joined end-to-end by a lap joint, brace, or other suitable means along the length of the outer sheath 50 of the middle section delivery device 14 and outer guide channel 80 of the distal portion 13.

Another embodiment may be used for constructing or joining the middle section delivery device 14 and distal portion 13 in a way that permits the outer guide channel member 80 of the distal portion 13 to move relative to the inner guide channel member 70 of the distal portion 13. In this embodiment, the outer sheath 50 of the middle section delivery device 14 and the outer guide channel member 80 of the distal portion 13 comprise a plurality of units. Each unit may comprise the construction shown and described above in connection with the discussion relating to Figures 4A through 4G and/or Figure 5. Moreover, the plurality of units, such as the distal portion 13 and the middle section delivery device 14, may be assembled or joined together in various ways described next.

In one assembly, the distal portion 13 and the middle section delivery device 14 may be put together by any suitable connector. By way of example, the middle section delivery device 14 and distal portion 13 may be put together with a lap joint or a brace.

Figure 5A provides a longitudinally sectioned schematic side view, broken away, of a brace section 240 for use in associating the middle section delivery device and a distal portion of a medical device according to the invention. Figure 5B shows a cross sectional view of Figure 5A taken along the lines 5B-5B.

According to Figures 5A and 5B, the brace section 240 comprises a puller 54 that can be used to connect or fasten two structures. For instance, the puller 54 may be used to operatively couple the middle section delivery device 14 and the distal portion 13. In general, the puller 54 represents any bridged reinforcement piece of suitable material, such as stainless steel wire or other alloy, with a proximal flattened end and a distal flattened end, wherein the proximal flattened is placed between a Teflon inner layer 242 and/or coil 243 and a nylon outer layer 244 of an outer sheath 50 and wherein the distal flattened end is placed between a Teflon inner layer 242 and/or coil 243 and a nylon outer layer 244 of an outer guide channel member. The puller 54 may be heat bonded between the Teflon inner layer 242 and the nylon outer layer 244, or it may be adhesively bonded to the Teflon inner layer 242 and the nylon outer layer 244, or it may be welded, soldered, glued, or lap jointed to the coil 243.

Also, the puller 54 may join a plurality of coils, multifilar materials, weaves, or braids end-to-end. Furthermore, the puller 54 may join a plurality of segments comprising a coil, multifilar material, weave, or braid. Moreover, the puller 54, when used with a "Flexor®" sheath as an outer sheath 50 of a middle section delivery device 14 as previously described, provides additional strength anywhere support is needed to provide structural integrity, such as when there is a gap between the bridged components, or a reduced cross sectional area of a component compromises that component's structural integrity.

Figures 5A and 5B show the brace section 240 comprising three layers: an inner layer 242, a middle layer 243, and an outer layer 244. The inner layer 242 may comprise Teflon; the outer layer 244 may comprise nylon and/or PEBA; and the middle layer 243 may comprise stainless steel circumferential coil. Heating the brace section 240 to an appropriate temperature reduces the outer layer inner surface 249 to a liquid state. Treating, mechanical or chemical etching, sandblasting, or otherwise preparing the inner layer outer surface 241 improves the bonding properties of the inner layer outer surface 249. Consequently, the melted outer layer inner surface 249 seeps through spaces in or between middle layer coils 243 and bonds to both the inner layer outer surface 241 and to the coils 243.

Figure 9B shows that the brace section 240 may further comprise one or more pullers 54. The pullers 54 may include, for example, stainless steel flat wire. Optionally, a puller 54 may overlap, weave, or braid with the middle layer coils 243. The surface of the puller 54 may be treated, mechanical or chemical etched, sandblasted, or otherwise prepared and the outer layer inner surface 249 melted to bond the three layers 242, 243, 244 and the puller 54 anywhere two structures need connecting or fastening, or simply anywhere additional support is needed.

In one embodiment, the puller 54 is generally rectangular, polygonal, or irregular in shape or cross section and is positioned approximately parallel to a longitudinal axis 246 of the brace section 240. Alternatively, the distal portion 13 and the middle section delivery device 14 may be attached, adjoined, joined, or combined by a brace section 240, wherein the puller 54 is a mechanical connector selected from the group consisting of a wire, inward protrusion, nuts and bolts, pins, clamp, pincher, hook, fastener, joint, ring, sheath, ball and socket, full or partial bearing, shaft, thread, cable, rod, bar, ball or hemispherical ball, fulcrum, support, linkage, or other crimping, clutching, gripping, joining, or holding mechanical holding materials, to name a few illustrative examples of mechanical connectors for operatively coupling the middle section delivery device 14 and the distal portion 13 in accordance with an embodiment of the invention. Other examples of a puller 54 include glue, adhesives, resins, welding, soldering, brazing, adhesives, or chemical bonding materials or combinations thereof. By way of further illustration, the distal portion 13 and middle section delivery device 14 may be attached by a puller 54 that is a stainless steel coil.

Figures 5C through 5K show the distal portion 13 and middle section delivery device 14 are operatively coupled by a bridging device 100 as in Figures 5C through 5K. For clarity, the distal portion 13 and middle section delivery device 14 having a delivery device 40 with an outer sheath 50 and inner compression member 41 are not shown in full in Figures 5C through 5K, and instead they are only represented schematically as a truncated tubular distal portion 13 and a truncated tubular middle section delivery device 14.

A bridging device 100 has a low profile and may vary in length, thickness, and width. For example, the length may be from 1 cm to about 10 cm or more, and in one embodiment from about 2 cm to about 6 cm, or it may correspond to the approximate length of the self-expanding device in a compressed state or the approximate length that the outer guide channel member may need to slide relative to the inner guide channel member to deploy the self-expanding device. Moreover, the low profile of the bridging device 100-relative to the distal portion 13 or middle section delivery device 14-facilitates the exiting of a wire guide from a breech position opening 65 located at or near the rear, back, or proximal part of the distal portion 13. The low profile and length allow the physician to grab the exiting wire guide as needed. The physician may desire to feed the exiting wire guide into another instrument, into a passageway of the middle section, or through one or more eyelets attached to maintain the wire guide the middle section to substantially parallel with or side-by-side with the middle section delivery device 14.

The bridging device 100 further comprises a link member 101' joining the bridge to the distal portion 13 and a link member 101 joining the bridge to the middle section delivery device 14. The link members 101, 101' may be any puller 54 described above for assembling the middle section delivery device 14 and distal portion 13, where the puller 54 is a connector selected from the group consisting of mechanical structures, chemical bonding materials (resins, glue, adhesives, and the like), and welding (soldering, brazing, and the like) materials or combinations. Certain additional non-limiting examples of link members 101, 101' comprise a nut, bolt, screw, thread, suture, cotter and pin, spring, full or partial bearing, bearing surface, rivet, pin, shaft, thread, joint, or other linkage or securing mechanism. Furthermore, a link member 101, 101' may join the bridging device 100 to an outer surface or an inside surface of the middle section delivery device 14 or distal portion 13.

The bridging device 100 may comprise a single span construction or a plurality of spans. Figures 5C-5G show various views of an embodiment of a bridging device 100 comprising a span 102 and link members 101,101'. In alternative embodiments as shown in Figures 5H-5K, the bridging device 100 comprises a first span 104 joined to the middle section delivery device 14 by a link member 101, and a second span 106 joined to the distal portion 13 by a link member 101'. The first span 104 and second span 106 are joined together by an articulation member 105.

The bridging device 100 may be made of any suitable material used for the middle section delivery device 14 as described above or used for the distal portion 13 as described below. The material is resilient with sufficient column strength to be kink resistant. Optionally, the bridging device 100 may be pliable, elastic, twistable, and flexible. For illustration, only Figures 5F, 5I, and 5J are shown flexing, but the bridging device 100 of Figures 5C-5K may all be made flexible (if desired). Figure 5F shows the embodiment flexing. Figure 5I shows the embodiment with both first and second spans 104, 106, respectively, flexing torsionally. Figure 5J shows the embodiment with the second span 106 flexing torsionally. The flexibility of the bridging device 100 may be varied by the materials used to construct the bridging device 100, depending on the degree of flexibility desired.

Optionally, the span 102, first span 104, and second span 106 may articulate about link members 101, 101' as shown in Figures 5C-5G, and the articulation member 105 shown in Figures 5H-5K. When articulation is desired, the link members 101, 101' and articulation member 105 preferably comprise mechanical structures, such as a nut, bolt, screw, thread, suture, cotter and pin, spring, full or partial bearing, bearing surface, rivet, pin, shaft, thread, joint, or other linkage or securing mechanism that allows articulation. Furthermore, articulation may be controlled by limiting the range of motion from a range within about 1 degree to approximately 15 degrees.

Articulation, as used here, means that one or more of the following pieces is moveable relative to one or more of the other pieces: middle section delivery device 14, spans (102, 104, 106), and distal portion 13. Articulation may include one or more degrees of translational displacements and/or rotations. For instance, the articulation may be axial, longitudinal, forward, backward, orthogonal, lateral, transverse, rotational, pivotable, sloping incline or decline, swinging, torsional, revolving, and other forms of translation and/or rotation in an x, y, and/or z coordinate system.

According to Figures 5L through 5O, a distal insert 220 and proximal insert 230 may be used as an alternative embodiment of a bridging device 100. The embodiments for Figures 5L through 5O may be used for either or both the distal insert 220 and proximal insert 230, so alternative reference numerals are used, but are explained separately below.

Figures 5L through 5O show examples of a distal insert 220 having an opening 283" at a mating portion 222, an exit port 283' at an interfacing portion 224, and the openings 283', 283" defining a guide channel 281. Similarly, Figures 5L through 5O may represent examples of a proximal insert 230 having an opening 233' at a mating portion 232, an exit port 233 at an interfacing portion 234, and the openings 233', 233 defining a guide channel 231. The inserts 220, 230 are generally tubular, and one embodiment is a cannula.

Figure 5L shows an example of an insert 220, 230 that has an opening 283", 233' at a flared mating portion 222, 232 for securing to the outside of the outer sheath distal end and outer guide channel member second end 58, 87, respectively. It should be understood that the mating portions 222, 232 of the other Figures 5M through 5O also may secure, however, either to the outer sheath distal end or outer guide channel member second end 58, 87, respectively. The inserts 220, 230 may comprise any of the materials as previously described as making up the middle section delivery device 14 or later described as making up the distal portion 13. In one embodiment, the inserts 220, 230 are stainless steel cannulas.

The lengths of the inserts 220, 230 may vary from about 10 to 40 mm. In an alternative embodiment, the lengths are approximately 15 to 25 mm. In still another embodiment, the lengths are approximately 20 mm. The diameters of the guide channel 281, 231 also may vary. In most cases, the diameter will range from approximately 2 to 7 French, with a preferred embodiment from approximately 4 to 5 French in diameter. The openings 283', 233 may be at an angle between a range of zero to about 90 degrees relative to an axis of the insert 220, 230, respectively.

Figures 5N and 5O, in addition to having an opening 283", 233' at a mating portion 222, 232, and a port 283', 233 at an interfacing portion 224, 234 defining a channel 281, 231, further having perforations 208 and/or slots 209 intermediate the mating portion 222, 232 and the interfacing portion 224, 234. The perforations and slots 208, 209, respectively, which may be laser-cut, machined, milled, or drilled. The slots 209 of Figure 5O increase the flexibility of the inserts 220, 230. In addition, both the perforations and the slots 208, 209 optimize the bonding retention properties for securing the inserts 220, 230 to the outer member and outer sheath, respectively, especially where the mating portions 222, 232 are dispose between an inner layer 44 and outer layer 42 of the outer guide channel member or outer sheath 80, 50, respectively. In other words, the materials that form the inner and outer layers 44, 42 join during the welding process, thereby securing the mating portions 222, 232 between the inner and outer layers, as shown in Figures 5R and 5S.

Figures 5P represents sectional side schematic views, broken away, of an outer guide channel member 80 (discussed below) and an outer sheath 50. The outer guide channel member 80 comprises a second end portion 87 with an exit port 83 and a guide channel 81. The outer sheath 50 comprises a distal end 58 with a distal opening 52 and having a passageway 59.

A distal insert 220 has an outer member mating portion 222 secured to an outer guide channel member 80, while a proximal insert 230 has an outer sheath mating portion 232 bonded to the outer sheath distal end 58. In one embodiment, the outer member mating portion 222 of the distal insert may be secured to an inner layer 44 within the outer member guide channel 81, while the outer sheath mating portion 232 of the proximal insert 230 may be secured to an inner layer 44 within the outer sheath passageway 59. In an alternative embodiment, the mating portions 222, 232 of the distal insert 220 and proximal insert 230 may be received by and secure to the outer layer 42 of the outer guide channel member 80 and the outer sheath 50, respectively.

As shown in Figure 5Q, another embodiment comprises the mating portions 22, 232 of the distal insert 220 and proximal insert 230 being disposed between an inner layer 44 and an outer layer 42 of the outer guide channel member 80 and the outer sheath 50, respectively, wherein the mating portion 232 is optionally located distal to the outer sheath coil 43 and wherein the mating portions 222 is optionally located proximal to the coil 43 of the inner guide channel member 80. Optionally, an inner layer 44 comprises Teflon material and an outer layer 42 optionally comprises a nylon and/or PEBA material. Also, the mating portions 222, 232 optionally replace a middle layer, such as a coil 43, of the outer sheath distal end 58 and outer guide channel member second end portion 87, respectively. In one embodiment, the mating portions 222, 232 are secured to the outer sheath distal end 58 (between the inner and outer layers 44, 42, respectively) and outer member second end portion 87 (between the inner and outer layers 44, 42, respectively), respectively, by melt bonding during initial construction of the outer sheath distal end 58 and outer member second end portion 87. Alternatively, post construction of the distal end 58 and the outer guide channel member second end portion 87, the mating portions 222, 232 may be secured between the inner and outer layers 44, 42, respectively, of the outer sheath distal end 58 and outer guide channel member second end portion 87, respectively, by heat-bonding, for example.

Figure 5Q further shows an interfacing portion 224 of the distal insert 220 and an interfacing portion 234 of the proximal insert 230. The insert interfacing ends 224, 234 are brought together and operatively coupled by any suitable connection selected from the group consisting of glue, adhesive, resin, welding, fusing, soldering, brazing, wire, crimping, link member, interlocking member, or other mechanical or chemical bonding materials or combinations thereof. One example of welding includes laser welding. In addition, the insert interfacing ends 224, 234 may be operatively coupled by a solid-state bond resulting from heat-bonding, such as the melting and solidifying (fusing) of material of the interfacing ends 224, 234, which allows the joining of similar and dissimilar metals according to materials science.

Figure 5R shows a sectional side view, broken away, of a device having mating portions 222, 232 of an insert 220, 230, respectively, according to Figure 5O secured to the second end portion 87 of the outer guide channel member 80 or the distal end 58 of the outer sheath 50, respectively. The mating portions 222, 232 are disposed between an inner layer 44 and an outer layer 42 of the outer guide channel member 80 and outer sheath 50, respectively. A connector 209' is formed through the slot 209 via a solid-state bond resulting from heat-bonding, such as the melting and solidifying (fusing) of the material of the outer and inner layers 42, 44.

Figure 5S shows a sectional side view, broken away, of a device having openings 283", 233' at mating portions 222, 232 of a distal insert and proximal insert 220, 230, respectively, according to Figure 5N secured to the second end portion 87 of the outer guide channel member 80 or the distal end 58 of the outer sheath 50, respectively. The mating portions 222, 232 are disposed between an inner layer 44 and an outer layer 42 of the outer guide channel member 80 and outer sheath 50, respectively. A connector 208' is formed through the perforation 208 via a solid-state bond resulting from heat-bonding, such as the melting and solidifying (fusing) of the material of the outer and inner layers 42, 44.

Figure 5T shows a section side view, broken away, of an example of an insert 220, 230 according to an embodiment of Figure 5L. The mating portion 222 is flared for securing around the second end portion 87 of an outer guide channel member 80. Alternatively, the flared mating portion 232 is configured to secure around distal end 58 of the outer sheath 50. In both of these and like embodiments, the mating portion 222, 232 may also secure around the outer layer 42 or the inner layer 44 of the outer guide channel member 80 or outer sheath 50.

Optionally, the inner compression member distal mating end 48 may be secured to a proximal insert 230 and an inner guide channel member second end portion 77 may be secured to a distal insert 220. The insert interfacing ends 224, 234 are brought together and operatively coupled by any suitable connection discussed above. Alternatively, the proximal insert 230 may be omitted, and the distal insert interfacing end 224 may be operatively coupled directly to the inner compression member distal mating end 48 by glue, adhesive, resin, welding, fusing, soldering, brazing, melt bonding, crimping, or other mechanical or chemical bonding materials or combinations thereof.

### SYSTEM DISTAL PORTION 13

Now turning to embodiments of a distal portion 13 of medical device delivery systems according to the invention, Figures 6, 7, 8, and 9 show the distal portion 13 to be a relatively tubular body. Given the configuration of vessels, vessel passageways, a working channel of an endoscope, or an external accessory channel device used with an endoscope to be navigated, a mostly tubular distal end with a distal tapered, rounded, chamfered, or arrowhead shape may be better tolerated by the patient. Further, in certain embodiments, the distal portion of the distal portion 13 may be soft, rounded, and flexible so as to provide further protection for and care to the patient.

Figure 6 illustrates an embodiment of the distal end 13 of a delivery system for the rapid insertion of self-expanding stents (for example) comprising an inner guide channel member 70, an outer guide channel member 80 axially slideable relative to the inner guide channel member 70, a self-expanding deployment device mounting region 90 (e.g., a stent mounting region), and a transition region 60. As used in connection with describing embodiments of the inner and outer guide channel members 70, 80, respectively, the term "guide channel" is understood to be any aperture, bore, cavity, chamber, channel, duct, flow passage, lumen, opening, orifice, or passageway that facilitates the conveyance, evacuation, flow, movement, passage, regulation, or ventilation of fluids, gases, or a diagnostic, monitoring, scope, catheter, other instrument, or more particularly a wire guide (Fig. 8) or another component of the distal end (e.g., an inner guide channel member 70 relative to the outer member channel 81).

The distal end 13, according to the delivery system 10 and shown in Figures 6, 7, 8, and 9, may be made of any suitable material (natural, synthetic, plastic, rubber, metal, or combination thereof) that is rigid, strong, and resilient, although it should be understood that the material may also be pliable, elastic, and flexible. By way of illustration only and not by way of limitation, the distal end may comprise one or a combination of the following materials: metals and alloys such as nickel-titanium alloy ("nitinol") or medical grade stainless steel, and/or plastic and polymers such as polyether ether-ketone ("PEEK"), polytetrafluoroethylene (PTFE), nylon and/or a polyether block amide ("PEBA"), polyimide, polyurethane, cellulose acetate, cellulose nitrate, silicone, polyethylene terephthalate ("PET"), polyamide, polyester, polyorthoester, polyanhydride, polyether sulfone, polycarbonate, polypropylene, high molecular weight polyethylene, polytetrafluoroethylene, or mixtures or copolymers thereof, polylactic acid, polyglycolic acid or copolymers thereof, polycaprolactone, polyhydroxyalkanoate, polyhydroxybutyrate valerate, polyhydroxy-butyrate valerate, or another polymer or suitable material. Where it will not contact the patient (e.g., it is contained within a sheath, working channel of an endoscope, or an external accessory channel device used with an endoscope), the middle section delivery device 14 and distal end 13 do not need to be biocompatible. In contrast, where there is the possibility of patient contact, the material may need to be biocompatible or capable of being made biocompatible, such as by coating, chemical treatment, or the like.

The inner and-outer guide channel members 70, 80, respectively, may be made of any suitable material described above for use with the distal end 13. In one embodiment, the inner guide channel member 70 and the outer guide channel member 80 comprise PEEK material, which has the advantage of softening under heat before burning or degrading. PEEK tubing may be purchased from many suppliers, such as Zeus, Inc. in Orangeburg, South Carolina for instance.

Beginning with the inner guide channel member 70, a description will follow relating to features common to embodiments of a distal end 13 of a delivery system 10 for the rapid insertion of "stents" according to the invention. The inner guide channel member 70 is generally tubular and comprises a first end portion 78 and a second end portion 77 defining a wire guide channel 71 therebetween. Optionally, the inner guide channel member 70 is configured to be slidably nested, fitted, secured, or otherwise positioned within the outer guide channel member 80 such that at least one of the inner guide channel member first or second end portions 78, 77, respectively, is axially intermediate an outer guide channel member first end portion 88 and an outer guide channel member second end portion 87.

The first end portion 78 of the inner guide channel member 70 further comprises a wire guide entry port 72, and the second end portion 77 has a wire guide exit port 73. The entry and exit ports 72, 73, respectively, define and are in communication via the wire guide channel 71. A port, in describing an embodiment of an inner guide channel member 70 and an outer guide channel member 80 according to the invention, includes any structure that functions as an entry or exit aperture, cutout, gap, hole, opening, orifice, passage, passageway, port, or portal. The inner guide channel member entry port 72 is sized to receive a wire guide into the inner member guide channel 71, and the inner guide channel member 70 is configured so that the wire guide may exit proximally out the inner guide channel member exit port 73. Optionally, the exit port 73 is located at or near the transition region 60. In one embodiment of the present invent, the inner guide channel member 70 is a cannula (or catheter) having an entry port 72 and an exit port 73 as previously described and defining a guide channel 71 therebetween.

The inner guide channel member 70 further comprises an outer self-expanding deployment device mounting region 90 (e.g., an outer stent mounting region) positioned intermediate the inner guide channel member entry and exit ports 72, 73, respectively. The length of the inner guide channel member 70 of any of the embodiments of the present invention may vary generally from about 10.0 to about 40.0 cm. In one alternative embodiment, the length of the inner guide channel member 70 is approximately 15.0 to approximately 25.0 cm. In another embodiment, the length of the inner guide channel member 70 is approximately 20.0 cm. Also, the length of the inner guide channel member 70 may depend on the intended stent, and in another embodiment the length of the inner guide channel member 70 is approximately 15.0 cm for an 8.0 cm stent.

The inner guide channel member 70 further comprises inner and outer diameters. In one embodiment, both diameters are substantially uniform over the entire length of the inner guide channel member 70. By way of example, an internal diameter 74 might measure approximately 0.052 cm (0.0205 inches) at or near the inner guide channel member proximal second end portion 77, at or near the inner guide channel member distal first end portion 78, and intermediate the first and second end portions 78, 77, respectively. Likewise, an inner guide channel member 70 might have an outer diameter 75 that measures approximately 0.109 cm (0.0430 inches). Thus, the outer diameter 75 might measure approximately 0.109 cm (0.0430 inches) at or near the inner guide channel member proximal second end portion 77, at or near the inner guide channel member distal first end portion 78, and intermediate the first and second end portions 78, 77.

In an alternative embodiment to an inner guide channel member 70 having a substantially uniform outer diameter 75 along its length from about the second end portion 77 to about the first end portion 78, the inner guide channel member may also comprise a tapered outer diameter 76. In one embodiment, the inner guide channel member tapers distally to a second outer diameter 76' at or near the inner guide channel member first end portion 78 or intermediate the inner guide channel member first and second end portions 78, 77, respectively. The taper 76 has a decreased cross section, diameter, width, height, area, volume, thickness, and/or other configuration, shape, form, profile, structure, external outline, and/or contour relative to the outer diameter 75. In other words, the inner guide channel member second outer diameter 76' is smaller in cross section, diameter, width, height, area, volume, thickness, and/or other configuration, shape, form, profile, structure, external outline, and/or contour than the outer diameter 75.

Figure 6 further shows an optional atraumatic tip 170 coupled to the inner guide channel member first end portion 78. Extending distally from the inner guide channel member first end portion 78, the atraumatic tip 170 is tapered, rounded, chamfered, or arrowhead shape to be better tolerated by the patient. The atraumatic tip 170 comprises a distal first end portion 178 with a wire guide entry port 172 and a proximal second end portion 177 with a wire guide exit port 173, whereby the entry and exit ports define an atraumatic tip guide channel 171. The ports 172, 173 and channel 171 are sized to slidably receive a wire guide.

The atraumatic tip second end portion 177, as shown in Figure 4, may abut the outer guide channel member distal end portion 88 and, thereby, extend entirely distally beyond a distal opening 89 of the outer guide channel member first end portion 88. Optionally, the outer guide channel member distal opening 89 is spaced from the atraumatic tip 170 sufficient to allow delivery system to be flushed with saline that exits the distal end to remove air in order to help keep air out of the patient, as explained above. In the alternative and as shown in Figure 5, the atraumatic tip 170 may be configured to have a second end portion 177 that is beveled such that the atraumatic tip second end 172 is partially positioned within the outer member guide channel 81 and partially proximal to the outer guide channel member distal opening 89. The beveled design of the atraumatic tip second end portion 177 forms a proximal stop against the outer guide channel member distal opening 89 while permitting the atraumatic tip second end portion 177 to be partially slidably nested, fitted, secured, or otherwise positioned within the outer guide channel member first end portion 88 so that the outer guide channel member first end portion 88 overlaps the atraumatic tip 170 to form a suitable seal that substantially occludes passage of a wire guide between the atraumatic tip 170 and the distal opening 89 of the outer member first end portion 88 (Fig. 7). Furthermore, the atraumatic tip second end portion 177 comprises a stent distal restraint 93' as explained below.

In Figure 6, the outer guide channel member 80 also is generally tubular and comprises a first end portion 88 and a second end portion 87. The outer guide channel member 80 further comprises a wire guide entry port 82 proximal to the first end portion 88 and a proximal wire guide exit port 83 located at or near the second end portion 87. The entry and exit ports, 82, 83, respectively, define a guide channel 81 of the outer guide channel member 80, wherein the ports 82, 83 and channel 81 are sized to slidably receive a wire guide. The entry port 82 is configured to receive a wire guide into the outer member guide channel 81, and in one embodiment, the entry port 82 is defined by the inner guide channel member exit port 73. In that embodiment, the wire guide moves proximally through the inner member guide channel 71 and egresses from the inner guide channel member exit port 73, wherein the proximal passage of the inner guide channel member exit port 73 is designated as the outer guide channel member wire guide entry port 82. The outer guide channel member proximal wire guide exit port 83 is configured so that a wire guide may egress proximally out the outer member exit port 83. In one embodiment, the outer guide channel member distal opening 89 and exit port 83 define the guide channel 81 therebetween.

In one embodiment, the Flexor® sheath, manufactured and sold by Cook Incorporated of Bloomington, Indiana, may be adapted for use with the distal end 13 and/or the middle section delivery device 14. Otherwise stated, the Flexor® sheath, as shown in Figure 3 and described above, may be provided for the distal end 13 and/or the middle section delivery device 14. For instance, the distal end 13 may be constructed as comprising an integral Flexor® sheath tube with the middle section delivery device 14. Alternatively, a Flexor® tubing may be used for either the middle section delivery device 14 or the distal end 13, or both. Then, the separable middle section delivery device 14 and distal end 13 may be attached, adjoined, joined, or combined as taught herein above.

The Flexor® sheath has a PTFE inner lining 44 that provides a slick, smooth surface for sliding the outer sheath 50 and/or the outer guide channel member 80 proximally. With regard to the distal end 13, the outer guide channel member 80 slides relative to the inner guide channel member 70, and a stent constraining inner surface 92 of the outer guide channel member 80 would be the inner layer 44 described above, thereby resulting in minimal friction to a stent 17 on the stent platform 91. The slideable inner surface 92 of the Flexor® sheath exhibits a second benefit of minimizing damage or misalignment to the stent. Indeed, because self-expanding stents continuously exert an expanding force against the inside surface 92 of the outer guide channel member 80, any substantial friction or drag between the stent and the inner surface 92 of the outer guide channel member 80 as the outer guide channel member 80 withdraws may damage the stent or cause the stent to be deployed slightly off of the target site.

The thin flat wire reinforcing coil 43 of the Flexor® sheath provides the outer guide channel member 80 with necessary radial strength to constrain the stent over long periods of storage time. In contrast, where the a stent constraining inner surface 92 of an outer guide channel member 80 does not comprise the Flexor® sheath inner layer 44 or equivalent, the stent over time may tend to become imbedded in the inner surface 92 and, as a result, interfere with retraction of the outer guide channel member 80 at the time of deployment. In an outer guide channel member 80 that comprises a Flexor® sheath, in addition to the inner layer 44 and the reinforcing coil 43, the outer guide channel member 80 has a Flexor® sheath outer layer 42. The outer layer 42 comprises nylon and/or PEBA to provide the necessary stiffness for pushability, retraction, and control of the outer guide channel member 80 to facilitate proper deployment of the constrained self-expanding stent. Therefore, the Flexor® sheath is one non-limiting example of an embodiment of an outer sheath 50 and/or an outer guide channel member 80.

While Figure 6 shows an outer guide channel member 80 having the exit port 83 proximal to the entry port 82 in one embodiment of the outer guide channel member 80, the relative axial distances between the entry and exit ports 82, 83, respectively, vary when the outer guide channel member 80 is in a non-deployed state versus a deployed state, because the outer guide channel member 80 moves axially relative to the inner guide channel member 70. Otherwise stated, Figure 6 shows an embodiment where the exit port 83 is proximal to the entry port 82 in either a non-deployed stent position or in a deployed stent position. In a non-deployed stent position of another embodiment, however, the exit port 83 may be substantially co-planar to or aligned with the entry port 82. In the fully deployed stent position, the exit port 83 may likewise be proximal, co-planar, or aligned with the entry port 82. Optionally, the entry port 82 and exit port 83 are located at or near the transition region 60 to be discussed below.

Furthermore, the outer guide channel member 80 has a stepped 84, 85 profile, whereby the outer guide channel member 80 comprises a first outer diameter 84 intermediate the outer guide channel member first and second end portions 88, 87, respectively, and a second smaller outer diameter 85 located at or near the outer guide channel member second end portion 87 in the vicinity of the transition region 60 and the breech position opening 65. The stepped 84, 85 profile includes an embodiment where the outer guide channel member 80 transitions to the distal end portion 58 of the outer sheath 50 of the middle section delivery device 14. In describing embodiments of the invention, however, the stepped 84, 85 profile shall be discussed in reference to the outer guide channel member 80 in particular, but it should be understood as including a stepped 84, 85 profile in reference to the transition region 60 of the distal end 13 relative to the middle section delivery device 14 where the middle section delivery device 14 and distal end 13 are formed from separate units such as, by way of example only and not by way of limitation, separate "Flexor®" sheaths where one comprises a first outer diameter 84 and the other comprises a second smaller outer diameter 85.

As shown in Figure 6, the second smaller outer diameter 85 of the outer guide channel member 80 is located proximal to the larger first outer diameter 84 and, thereby, comprises a stepped 84, 85 profile. Having a second smaller outer diameter 85 reduces the profile of the outer guide channel member 80 and/or the outer guide channel member 80 transition to the middle section delivery device 14, which is advantageous in procedures involving narrow vessel passageways, endoscope working channels, or accessory channels for use with endoscopes. The difference in the.first diameter 84 and the second diameter 85 may vary. By way of illustration, the second diameter 85 may be approximately one-fourth to approximately nine-tenths that of the first diameter 84. In another embodiment, the second diameter 85 may be about one-half that of the first diameter 84. In another embodiment, the first diameter 84 is roughly 5 French while the second diameter 85 is roughly 4 French.

In one embodiment of the stepped 84, 85 profile of the outer guide channel member 80, the second smaller outer diameter 85 is located at or near the outer guide channel member second end portion 87. The second end portion 87 may decrease precipitously from the first outer diameter 84 to the second smaller diameter 85. In a precipitous step, the change from the diameters occurs over a short length along the longitudinal axis of the distal end 13. In a further example of a precipitous step, the plane formed by the exit port 83 may be substantially perpendicular to the longitudinal axis of the outer guide channel member 80. In an alternative embodiment, the second end portion 87 may decrease gradually from the first outer diameter 84 to the second smaller diameter 85. In a gradual step, the change from the two diameters occurs over a length of more than 1.0 millimeter ("mm") along the longitudinal axis of the distal end 13 at or near the transition region 60 and breech position opening 65, and in one instance this change occurs over a length from about 1.0 mm to about 10.0 mm. In a further example of a gradual step, the plane formed by the exit port 83 may be at an angle other than 90 degrees relative to the longitudinal axis of the distal end 13.

Figure 6 also shows a breech position opening 65 located at or near the second end portion 87 of the outer guide channel member 80 comprising the wire guide exit port 83. In other words, rather than the exit port 83 being an aperture in a lateral sidewall of the outer guide channel member 80 intermediate the first and second end portions 88, 87, respectively, in a breech position opening 65 embodiment the exit port 83 is at the rear, back, or proximal part of the distal end 13 at or near the outer member second end portion 87 and the stepped 84, 85 profile such that it opens in the direction of the outer surface of the outer sheath 50.

The breech position opening 65 may be used for front-loading and the more common procedure of back-loading a wire guide (or catheter, for instance). In a back-loading procedure for a delivery system having a breech position opening 65, the wire guide may pass proximally through the guide channel 71 of the inner guide channel member 70, proximally through the guide channel 81 of the outer guide channel member 80, and leave the exit port 83 of the second end portion 87 of the outer guide channel member 80 from a breech position opening 65 in a rear, back, or proximal part of the distal end 13. Conversely, in a front-loading procedure for a delivery system having a breech position opening 65, the physician may feed the wire guide distally into a breech position opening 65 at the rear, back, or proximal part of the distal end 13 by entering the exit port 83 of the second end portion 87 and the guide channel 81 of the outer guide channel member 80 and through the guide channel 71 of the inner guide channel member 70, where the wire guide may exit from the wire guide entry port 72 of the inner guide channel member 70 and/or wire guide entry port 172 of the atraumatic tip 170.

In a distal portion 13 having a breech position opening 65 that comprises an exit port 83 located at a breech position of the transition region 60 according to the invention, the wire guide does not need to make any sharp turns away from the longitudinal axis of the guide channel 71 of the inner guide channel member 70 and/or the distal portion 13 generally that may result in kinking of the wire guide. In one embodiment, the exit port 83 is spaced proximally of the inner guide channel member second end portion 77 and is substantially (e.g., less than 30 degrees and preferably less than 15 degrees) along the longitudinal axis of the inner guide channel member 70 such that the longitudinal axis of the inner guide channel member 70 intersects the exit port 83, runs through the exit port 83, or the exit port 83 otherwise lies along the plane of the longitudinal axis of the inner guide channel member 70. In another embodiment, the exit port 83 is within the plan form by the side walls of the outer guide channel member 80, as opposed to a sideport that is within the sidewall of the outer guide channel member 80, wherein the wire guide needs to may a sharp turn away from the longitudinal axis of the guide channel 71. In another embodiment, the breech position opening 65 comprises an exit port 83 according to embodiments of the invention, as those shown in Figures 6, 7, 8, and 9 (by way of example and not by way of limitation) is located proximal to the inner guide channel member second end portion 77 and may be transverse or angled relative to the longitudinal axis of the tubular inner guide channel member 70 and/or the distal portion 13. In one embodiment, the wire guide exit port 83 may be positioned at or near a breech position opening 65 of the distal portion 13, wherein the exit port 83 is located at or near the rear, back, or proximal part of the outer guide channel member 80 and/or second end portion 87; in other words, not positioned exclusively on the side (e.g., outer circumferential cylinder wall) or substantially a length of the side of the outer guide channel member 80.

In Figure 6, the breech position opening 65 comprises an exit port 83 that is illustrated as being oblique, although other configurations of the exit port may be utilized to aid the wire guide in exiting the rear of the outer member. In one example, the exit port 83 may form a plane substantially perpendicular to the longitudinal axis of the outer guide channel member second end portion 87. In another example, the plane formed by the exit port 83 may be at an angle other than 90 degrees relative to the longitudinal axis of the distal end 13. Optionally, the oblique exit port 83 of a breech position opening 65 has lateral walls 83a, 83b that act as guide rails to direct a wire guide proximally toward the middle section delivery device 14 and to run along the outside of the outer sheath 50.

The overall axial length of the exit port 83 of the breech position opening 65 may vary. In one embodiment, the length is approximately from about 1.0 mm to about 10.0 mm. Another embodiment has a length of approximately 5.0 mm. The overall width of the exit port 83 may also vary. In one example, the width of the exit port is approximately 1 French. In yet another instance, the width of the exit port 83 ranges from about 1 French to about 4 French. In another example, the width of the exit port 83 may be the approximate difference between the first outer diameter 84 and the second outer diameter 85 of the outer guide channel member 80. In yet another embodiment, the exit port 83 comprises a diameter defined as being substantially equal to the difference between the outer guide channel member first outer diameter 84 and the outer guide channel member second smaller outer diameter 85.

At the transition region 60, the exit port 73 of the inner guide channel member 70 is in communication with the outer guide channel member 80 wire guide entry port 82, while the second end portion 77 is operatively coupled to the distal mating end 48 of the inner guide channel member 70 as explained below. The length of the transition region 60 may vary. For instance, the transition region 60 may be approximately from about 0.5 cm to about 10.0 cm. In another embodiment, the transition region 60 has the approximate length of about 5.0 cm. Furthermore, the length of the transition region 60 is variable: from a shorter axial length when the outer guide channel member 80 is in a non-deployed axial position; to a greater axial length when the outer guide channel member 80 retracts proximally to deploy the stent. Likewise, the overall length of the transition region 60 varies in the embodiment where the exit port 83 is distal to the entry port 82 when the outer guide channel member 80 is in a non-deployed stent position, compared to the initial length of the transition region 60 in an embodiment where the exit port 83 is proximal to the entry port 82 when the outer guide channel member 80 is in a non-deployed-stent position.

In one use of the transition region 60 according to an embodiment of the invention, the outer guide channel member entry port 82 receives a wire guide from the inner guide channel member exit port 73 and the wire guide thereby is received in the outer member guide channel 81. At the transition region 60, the inner member guide channel 71 and outer member guide channel 81 are approximately aligned relatively coaxially in one embodiment. Approximate alignment of the guide channels 71, 81 facilitates a smooth transition of the wire guide. Smooth transition optimally reduces any bending of the wire guide as the wire guide moves proximally from the inner member guide channel 71 to the outer member guide channel 81.

Several factors individually or collectively may further ensure that the inner member guide channel 71 and outer member guide channel 81 are approximately aligned relatively coaxially so that the wire guide may find its way out the distal portion 13. The breech position opening 65 is located in a longitudinally optimal location at the rear, back rear, back, or proximal part of the distal portion 13. Also, the breech position opening 65 is large enough to provide an easy exit; if too large it compromises the structural integrity of the outer guide channel member 80 and/or the transition region 60, and a puller 54 as taught herein may be needed. Furthermore, the inside shape of the breech position opening 65 is conducive to receiving the wire guide and directing it out, as with the [lateral walls 83a, 83b that act as guide rails to direct a wire guide proximally toward the middle section delivery device 14 and to run along the outside of the outer sheath 50, and/or with the overall axial length of the exit port 83 of the breech position opening 65 from about 1.0 mm to about 10.0 mm and alternatively approximately 5.0 mm, and/or the overall width of the exit port 83 of approximately from about 1 French to about 4 French or the difference in the first outer diameter 84 and the second outer diameter 85. Furthermore, outer sheath 50 has a passageway that is only large enough for the inner compression member 41 to pass through but not large enough to provide a receptacle for the wire guide as it is being fed through the distal portion 13.

Moreover, as the delivery system is being assembled, a pseudo-wire guide may be threaded through the breech position opening "backward" from normal. The pseudo-wire guide is then threaded backward through the inner guide channel member 70. As the outer guide channel member 80 and inner guide channel member 70 are slid together longitudinally, the pseudo-wire guide ensures that the inner guide channel member exit port 73 and the outer guide channel member exit port 83 are rotationally aligned. Because the outer guide channel member 80 is operatively coupled to the outer sheath 50 and the inner guide channel member 70 is operatively to the inner compression member 41, by connected the outer sheath proximal end 57 and the inner compression member proximal end 40 at the system proximal portion 12 as described above, this locks the inner guide channel member exit port 73 and the outer guide channel member exit port 83 into a properly aligned relationship. Then, the pseudo-wire guide can be removed, or left to function as a shipping fixture that is removed by the end user.

As shown in Figure 6, the distal end 13 also comprises a self-expanding deployment device mounting region 90. This mounting region 90 may be used for implantable prosthesis such as expandable (self-expanding, balloon expandable, or otherwise expanding) and nonexpanding stents, prosthetic valve devices, and other implantable articles for placement inside a patient's body (the implantable prostheses being referred to individually and collectively as "stents"'without limiting the invention) and therefore may be referred to as a stent mounting region to include the foregoing implantable prostheses.

The stent mounting region 90 comprises a stent platform 91 on an outside surface of the inner guide channel member 70 located at or near the inner guide channel member second end portion 78. In describing embodiments of the invention, the platform 91 "at or near" the inner guide channel member second end portion 78 includes a region intermediate the inner guide channel member entry port 72 and the inner guide channel member exit port 73. The platform 91 may be any stent mounting surface, including but not limited to the outside surface of the inner guide channel member 70, a recess, or an indentation located at or near the first end portion 78 of the inner guide channel member 70. In a non-deployed state, a self-expanding stent for example (not shown) compresses against the stent platform 91 and disposes around the outside of the inner guide channel member 70.

The stent mounting region 90 controls the lateral movement (e.g., transverse expansion away from the inner guide channel member longitudinal axis) to avoid premature deployment of the stent. In order to control the lateral movement of the stent, the stent is sandwiched between the platform 91 on the inner surface of the stent and the stent constraining inner surface 92 of the outer guide channel member 80 to keep the stent in a compressed state. Because the stent is bound from above by the inner surface 92 of the outer guide channel member 80 and bound from below by the platform 91 of the inner guide channel member 70, the stent mounting region 90 maintains the stent in a substantially compressed state and controls premature deployment of the stent.

In addition to controlling a stent's lateral movement, the stent mounting region 90 restrains the axial movement of a stent to control the stent movement away from the target site. A proximal restraint 93 controls proximal axial movement of the stent. In one embodiment, the proximal restraint 93 is sized to be large enough to make sufficient contact with the loaded proximal end of the stent without making frictional contact with the inner surface 92 of the outer guide channel member 80. In addition to helping to stop the stent's proximal movement in the non-deployed state, this restraint 93 assists with "pushing" the stent out of the distal end 13 by helping to prevent the inner guide channel member 70 and/or the stent disposed on the stent mounting region 90 from migrating proximally when the outer guide channel member 80 retracts proximally relative to the stationary inner guide channel member 70 in order to expose and deploy the stent. Optionally, the restraint 93 may be radiopaque so as to aid in stent positioning within the vessel passageway at or near the target site within a patient. In one embodiment, an optional distal restraint 93' is large enough to make sufficient contact with the loaded distal end of the stent to control axially distal movement of the stent. Similarly, in another embodiment the proximal second end portion 177 of an optional atraumatic tip 170 controls the stent's distal axial movement. Indeed, because the medical device delivery system may be used for deploying an implantable prosthesis that comprises balloon expandable or non-expanding stents, prosthetic valve devices, and other implantable articles at a selected location inside a patient's body, the proximal restraint 93 and distal restraint 93' control the axial distal movement of the implantable prosthesis. Optionally, the distal restraint 93' and/or atraumatic tip 170 may comprise radiopaque materials so as to aid in stent positioning within the vessel passageway at or near the target site within a patient.

Figure 6 also illustrates that the inner compression member 41 and inner guide channel member second end portion 77 may be operatively coupled by any suitable means. In one embodiment, a melt bond 47 (described below) operatively couples an inner compression member distal mating end 48 ("mating end 48") and the second end portion 77 of the inner guide channel member 70. A melt bond 47 provides surface-to-surface contact between an outer engaging surface 48' of the mating end 48 and the inner guide channel second end portion 77, thereby forming a more solid connection between the inner compression member 41 and the inner guide channel member 70.

In one embodiment, the inner compression member outer engaging surface 48' may form a melt bond 47 to an inner surface 101 of the inner guide channel member second end portion 77. Alternatively, the inner compression member outer engaging surface 48' may form a melt bond 47 to the outer surface 102 of the inner guide channel second end portion 77. In yet another embodiment, the distal mating end 48 of a solid inner compression member 41 as shown in Figure 6 is implanted 49 between (and/or melt bonded 47 between) inner and outer surfaces 101, 102, respectively, of the inner guide channel member second end portion 77. In still another embodiment, an insert body operatively couples the inner compression member 41 and the second end inner guide channel member 70.

As used to describe an embodiment of the invention, melt bonding 47 (for shorthand purposes in describing embodiments according to the invention, melt bonding 47 includes implanting 49) comprises any suitable means for melting, liquefying, making semi-molten, making molten, softening, making tacky, fusing, or making malleable, pliant, supple, moldable, ductile, or otherwise penetrable by another component or fused to melt bonding material comprising the other element. For instance, melt bonding 47 involves bringing two components together at an interface, wherein one (or preferably both) of the component interfaces are in the melted state. Strictly speaking, true melt bonding 47 requires that both of the components be melted at the interface and that they may be sufficiently chemically and physically compatible such that they fuse together upon cooling.

The melt bonding materials comprising the two components may be the same or substantially same materials. In the alternative, the melt bonding materials may be different, so long as they have substantially similar melting points at standard atmospheric pressure such that the materials soften (or liquefy) under heat and thereby fuse together in a solid state melt bond 47 joining the first and second melt bonding materials of the components. If the materials had melting points that were too different, then one material may degrade or burn and the like before the second material begins to melt.

Melt bonding 47 may be single layer interface whereby one component interface/surface mates to a second component interface/surface, or may be multi-layer interface whereby one component is implanted 49 into a second component and then surrounded by the second component. The chemical compatibility can best be expressed in terms of having similar values for surface energy and/or solubility parameter. In simple terms, similar materials tend to have a mutual affinity and a greater propensity to adhere to one another than do dissimilar materials. Melt bonding includes bonding whereby one component is melted while the other component is at or above its melting point.

Melt bonding materials may have different "melt bonding" temperatures at which they soften and become almost tacky without substantial degradation. Melt bonding materials are available from vendors, including Zeus, Inc. in Orangeburg, South Carolina for instance; Cobalt Polymers in Cloverdale, California; and under the trade name of Pebax® PEBA from the Arkema Group. The melt bonding materials may include one or a combination of a class of suitable materials comprising nylon, nylon natural tubing, polyether block amide, polyetheretherketone, thermoplastic, acrylonitrile-butadiene-styrene copolymer, polypropylene, polyamide, ionomer, polycarbonate, polyphenylene oxide, polyphenylene sulphide, acrylic, liquid crystal polymer, polyolefin, polyethylene acrylate acid, polyvinylidene fluoride, polyvinyl, and polyvinyl chloride.

In one embodiment, PEEK material is used for the melt bonding material. PEEK melts at about 334°C (633°F), so the material may be heated from about 331°C (628°F) to about 337°C (638°F). For instance, a radiofrequency loop heater may be used for heating the melt bonding materials. Such a machine is available from Magnaforce, Incorporated and sold under the name and model Heatstation 1500. Another such machine is available from Cath-Tip, Inc. arid is sold under the model and name Cath-Tip II. There is a rise dwell and cool down time for the process. The total rise time is approximately 20 seconds and dwell time is approximately 10 seconds. During the dwell time the temperature is approximately 316°C (600°F). In one embodiment where nylon or PEBA are used, heating is at about 204°C (400°F), with dwell time of about 10 seconds.

Figures 6A and 6B present a schematic representation of a cross section 105 of components before and after melt bonding according to one embodiment of the invention. The cross section 105 of Figure 6A, for example, represents an inner component 106, a middle component 107, and an outer component 108. While all components are shown having interfaces in abutting physical contact, they need only be close enough to form a melt bond therebetween. Indeed, as previously explained in connection with the Flexor® sheath's outer layer 42 and inner layer 44, there may even be a middle layer comprising a coil 43 having spacings 43' through which the melt bonding material of the outer layer 42 may move to be into contact with the inner layer 44.

In the example represented in Figure 6A, the middle and outer components 107, 108, respectively, are intended to be melt bonded. The middle component 107 comprises a first melt bonding material 109 while the outer component 108 comprises a second melt bonding material 109', which may be the same material or may be separate materials that have similar melting points at atmospheric pressure.

Figure 6B shows some of the first melt bonding material 109 of the middle component 107 moving into some of the second melt bonding material 109' of the outer component 108. Likewise, some of the second melt bonding material 109' of the outer component 108 moves into some of the first melt bonding material 109 of the middle component 107. It should be noted that both of the first and second materials 109, 109' need not move into the other. Rather, the first and second materials 109, 109' need only bond at an interface, with or without mixing and the like. By way of example, the Flexor® sheath's outer layer 42 may melt to the middle layer coil 43 (which has not melted) and bond to the outer surface of the inner layer 44 with or without the outer surface of the inner layer 44 melting into the outer layer 42.

Figure 6B further shows that the first and second melt bonding materials 109, 109', respectively, of the middle component 107 and the outer component 108 or other components that have been melt bonded, upon cooling to solid state will form a melt bond 47 operatively coupling the components and/or the melt bonding materials that comprise the components. This results in additional strength and helps to form a more solid connection to the melt bonded components, because a solid-state bond results from using a suitable form of heat for melting and solidifying (e.g., fusing and/or cross-linking bonds formed at the melt bonded material interfaces).

Figure 7 illustrates a schematic view showing an alternative embodiment of a distal portion 13 of a delivery system for the rapid insertion of stents comprising an inner guide channel member 70, an outer guide channel member 80 axially slideable relative to the inner guide channel member 70, a deployment device mounting region 90 (e.g., a stent mounting region), and a transition region 60. Like elements from the previous drawings, embodiments, and description from above are labeled the same. In this embodiment, the inner compression member 41 optionally comprises a passageway 45 (e.g., hollow, having a lumen) that facilitates the conveyance, ventilation, flow, movement, blockage, evacuation, or regulation of medication and/or fluids or accommodates the insertion of a diagnostic, monitoring, scope, or other instrument.

The tubular inner compression member 41 may have a uniform inside diameter ranging from about 0.134 to about 0.335 cm (0.0527 to about 0.132 inches). The wall thickness of the tubular inner compression member 41 is approximately 0.004 cm (0.0015 inch). These dimensions are illustrative only, and the inner diameter and wall thickness may be constructed to be of any size necessary to accomplish the purposes for which the delivery system is to be employed (i.e., limited by the vessel passageway or working channel in which the device is to be used).

In addition, this inner compression member 41 has an optional distal one-way valve 61. Thus, the valve 61 may serve a dual function. First, a one-way valve is relatively resistant to contamination from bodily fluids entering the inner compression member passageway 45. Second, it allows the movement of medication and/or fluids to exit distally the inner compression member 41 passageway 45 at or near the transition region 60 and may direct medication and/or fluids into the inner member guide channel 71 and/or the outer member guide channel 81.

Indeed, the inner compression member passageway 45 may facilitate using the medical device delivery system for deploying an implantable prosthesis that comprise balloon expandable stents, prosthetic valve devices, and other implantable articles (individually and collectively, "stent") at a selected location inside a patient's body. The stent is disposed at the deployment device mounting region 90 intermediate the proximal restraint 93 and distal restraint 93' to control the axial distal movement of the implantable prosthesis.

In one embodiment for using the delivery system with a balloon expandable implantable prosthesis, the inner compression member distal mating end portion engaging surface 48' operatively couples to the inner guide channel member outer surface 102 (or is welded to an outer surface of a metal cannula that has the inner guide channel member second end portion 77 glued within the cannula lumen), and an inflation member (e.g., a balloon) extends distally from the inner compression member distal mating end portion and is disposed over the proximal restraint 93 and distally about the platform 91 of the stent mounting region 90 such that the balloon is located under the stent. The stent is positioned within the vessel passageway at or near the target site within a patient, wherein the outer sheath 50 and outer guide channel member 80 is axially slideable relative to the inner compression member 41 and inner guide channel member 70 upon corresponding axial slideable movement of the handle 30, thereby exposing and, ultimately, deploying the stent from the stent mounting region 90. The stylet 20 may be adapted to receive a syringe for allowing inflation fluid, such as saline, to travel from and through the proximal end 40 of the inner compression member 41 and out the valve 61 at the distal end portion 48 in order to fill the inflation chamber of the balloon. Therefore, balloon expands under the stent and, as a result, the stent expands radially to plastically deform the stent into a substantially permanent expanded condition. The physician then deflates the balloon and removes the inner guide channel member 70 and remainder of the delivery system from the patient's body. This description of using the delivery system for balloon expandable implantable prosthesis is given by way of example and not by way of limitation. Alternatively, a tubular inflation fluid carrying device is in the outer sheath passageway 59 and extends from the system proximal portion 12 to the system distal portion 13 and operatively couples to an inflation member disposed under the stent.

In one embodiment of the distal portion 13 of a delivery system illustrated in Figure 7, an internal joint 46 comprises a melt bond 47 that operatively couples the inner guide channel member distal mating end portion 48 and the second end portion 77 of the inner guide channel member 70. For example, the inner compression member outer engaging surface 48' may form a melt bond 47 to the inner surface 101 (or alternatively to the outer surface 102) of the inner guide channel member second end portion 77, as taught above.

The embodiment shown in Figure 7 also illustrates that the exit ports 83 and 73 may have various configurations. First, these exit ports curve, and second, compared to Figure 4 they slope over a longer overall axial length to aid the wire guide in exiting the inner member and outer member, respectively. Furthermore, the exit port 83 thereby has longer axial lateral walls 83a, 83b for acting as guide rails to direct a wire guide proximally toward the middle section 14 and to run along the outside of the outer sheath 50.

Moving to the atraumatic tip 170 as illustrated in Figure 7, this is a little less arrowhead-shaped compared to the atraumatic tip 170 shown in Figure 6. Instead, the atraumatic tip in Figure 7 has a second end portion 177 that comprises a right cylindrical tubular configuration. Furthermore, the sides of the atraumatic tip second end portion 177 are more uniformly parallel and do not form a proximal stop against the outer guide channel member distal opening 89 as in a beveled embodiment of the atraumatic tip second end portion 177 as illustrated in Figure 6. The atraumatic tip second end portion 177 optionally comprises a stent distal restraint 93' for controlling distal axial movement of the implantable prosthesis when the medical device delivery system is used for deploying balloon expandable or non-expanding stents, prosthetic valve devices, and other implantable articles at a selected location inside a patient's body.

Turning now to Figure 8, that figure shows a partially sectioned distal portion 13 in accordance with an embodiment of the device according to Figure 7 with a wire guide 16 inserted therein. In a back-loading procedure, the wire guide 16 enters the guide channel 171 of the atraumatic tip 170 and travels proximally toward the inner guide channel member 70. The wire guide 16 then enters the inner member guide channel 71 and travels proximally toward the outer guide channel member 80 via the entry port 82 and enters the outer member guide channel 81 and out the exit port 83. The less common front-loading procedure could be described as above but conversely stated.

In Figure 8, the inner and outer guide channels 71, 81, respectively, are substantially aligned coaxially along an approximate center longitudinal axis of the distal portion 13. Because the channels 71, 81 are substantially aligned, the wire guide 16 moves through the inner member guide channel 71 to the outer member guide channel 81 and out the outer guide channel member exit port 83 at or near the breech position opening 65 with relatively little kinking, bending, buckling, or bowing. It should be noted that for the ease of showing the wire guide 16, the wire guide 16 proximal to the exit port 83 is shown slightly offset from outer sheath 50, though the wire guide 16 may actually run along the outside of the outer sheath 50 or in a groove (not shown) in the outer sheath 50.

Figure 9 illustrates a longitudinally sectioned side view showing an alternative embodiment of a distal portion 13 of a delivery system for the rapid insertion of stents comprising an inner guide channel member 70, an outer guide channel member 80 axially slideable relative to the inner guide channel member 70, a deployment device mounting region 90 (e.g., a stent mounting region), and a transition region 60. Like elements from the previous drawings, embodiments, and description from above are labeled the same. This embodiment represents an alternative embodiment of a joint 46 for operatively coupling the inner compression member 41 and inner guide channel member 70 with a cannula 95.

In one embodiment, the cannula 95 is a hollow, rigid tube, cylinder, ring, cannula (with or without a trocar), or other coupling device comprising metal such as medical grade stainless steel or super-elastic alloys (e.g., nitinol) to name but a few non-limiting examples. In one embodiment, the cannula 95 comprises a generally right cylindrical configuration or is elliptical, hyperbolic, parabolic, curved, polygonal, rectangular, or irregular in shape or cross section. The cannula 95 is sized for receiving the inner guide channel second end portion 77 and/or the inner guide channel second end portion outer diameter 75. The outer surface 102 of the inner guide channel second end portion 77 is operatively coupled to an inner engaging surface of the securing body 95 by glue, adhesives, resins, chemical bonding materials or combinations thereof and the like (collectively and individually, "glue"). By way of example only, the glue may be Loctite 4061 instant adhesive, formulated to polymerise rapidly in thin films between two surfaces to form rigid thermoplastics. Loctite 4061 instant adhesive is a medical device adhesive particularly suitable for a wide variety of substrates such as rubber, plastics, and metals, ant it is available from the Loctite Corporation.

In addition to securing the outer surface 102 of the inner guide channel member second end portion 77 to an inner engaging surface of the cannula 95, the cannula 95 also operatively couples the inner guide channel member distal mating end portion 48. An outer engaging surface 48' of the mating end portion 48 is in an abutting relationship (e.g., touching, in contact directly or by intervening parts, or adjacent) to an outer engaging surface of the cannula 95, and the mating end portion 48 and cannula 95 are operatively coupled by any suitable means, including but not limiting to welding, soldering, brazing, or fusing. Soldering and brazing are used if a semi-permanent connection between the distal mating end portion 48 and the cannula 95 is desired, because solder or braze metals have a lower melting point than the metals that are joined. Thus, when sufficient heat is applied to melt the solder or braze metal, they form an alloy with the surfaces of the mating end portion 48 and the cannula 95 and, upon solidification, thus form a joint that can be unfastened during manufacturing (e.g., to redo in the event of a poor connection) by reheating without destroying the parts that have been joined. In contrast, welding involves melting the outer engaging surface 48' of the mating end portion 48 and an outer engaging surface of the cannula 95 at the interface, or involves combining temperature and pressure so as to cause localized coalescence. Consequently, in most instances higher temperatures are involved than for soldering, and the union is permanent.

Where the inner compression member distal mating end portion 48 and the cannula 95 are connected, an optional tube may be disposed about the joint 46. The tubing has the advantage of minimizing some of the sharp edges created by a welded, soldered, or fused joint. In one embodiment, the tube is a melt bonding tube disposed about and melt bonded to the joint 46. Whereas Figure 9 shows the distal most tip of the distal mating end portion 48 flush with (e.g., substantially co-planar) the distal end portion of the cannula 95, it may alternatively be set back approximately 0.5 mm proximally from the distal end portion of the cannula 95. The set back arrangement allows solder, weld, or fusion to form a smooth transition and fill the space between that distal end tip and the cannula 95. This would also minimize the profile compared to placing more of a circumferential solder, weld, or fusion about the joint.

According to Figures 9, 9A, 9B, and 9C, the distal mating end portion 48 comprises a contoured configuration 48" that is complementary to an outer engaging surface 95' of the cannula 95. Thus, in an embodiment comprising a cannula 95 that is curved or otherwise circular in cross-section, then Figure 9A shows that the contoured configuration 48" is fluted so that the outer engaging surface 48' is capable of being in an abutting relationship (e.g., touching, in contact directly or by intervening parts, or adjacent) relative to a curved or circular outer engaging surface 95' of the cannula 95. A fluted contoured configuration 48" comprises any curved, shoehorn shape, celery shape, semicircular shape, crescent shape, wishbone shape, saddle shape, C-shaped, V-shaped, U-shaped, or other arcuate configuration. In another embodiment, an outer engaging surface 95' of the cannula 95 could have a flat portion, and Figure 9B shows that the contoured configuration 48" is likewise flat so that the outer engaging surface 48' is capable of being in an abutting relationship (e.g., touching, in contact directly or by intervening parts, or adjacent) relative to the flat portion of the outer engaging surface of the cannula 95. Even when the outer engaging surface 95' of the cannula 95 is curved or circular in cross section, however, Figure 9C shows that the inner compression member contoured configuration 48" could be flat, because the soldering, brazing, or fusing may fill in the space between the outer engaging surface 48' and a tangent that the flat configuration 48" forms to the curved portion of the outer surface 95' of the cannula 95. Similarly, if welding 96 is used, then the flat configuration 48" will form to a curved or circular outer engaging surface 95' of the cannula 95.

In addition, the contoured configuration 48" maintains low profile, high-strength, and flexibility of the connection between the inner compression member distal mating end portion 48 and the cannula 95. The contoured configuration 48" is in contrast to a rounded inner compression member distal mating end portion 48, which would have a greater diameter at the connection between the inner compression member distal mating end portion 48 and the cannula 95.

In order to create the contoured configuration 48", the inner compression member distal mating end portion 48 may be formed, sheared, casted, or molded. By way of example only, forming can be done both hot and cold (except for stamping, which is always done cold) in order to modify the shape and/or physical properties of the material comprising the inner compression member distal mating end portion 48. Common forming processes include rolling the distal mating end portion 48 (between one or two rollers), stretching, forging, straight bending, and stamping.

Figures 10 and 11 illustrate an alternate embodiment of a distal portion 13 of a delivery system for the rapid insertion of self-expanding stents during deployment of a stent. In Figure 8, the distal portion 13 is in an un-deployed position, while Figure 9 depicts the distal portion 13 in a deployed position after proximal retraction of an outer guide channel member 80 relative to the inner guide channel member 70. In other words, the wire guide 16 both enters the distal portion 13 and exits the distal portion 13 of the delivery system at a substantially straight path so as to minimize kinking of the wire guide, and then runs proximally along the outside surface of the outer sheath 50. The outer guide channel member 80 has a stepped profile comprising a first outer diameter and a proximal second smaller outer diameter in order to maintain a low profile at the transition region, as has been previously describe in detail in relation to Figures 6, 7, 8, and 9. Moreover, a guide 51 helps to align the wire guide as it exits the distal portion 13. For example, the guide 51 may comprise a U-shaped recess or an eyelet for aligning and guiding the wire guide. Furthermore, an optional groove 52 on the outside surface of the outer sheath 50 helps support the wire guide as it runs alongside the outside of the outer sheath.

Figures 12, 13A through 13C, and 14A through 14F show embodiments (by way of illustration and not by way of limitation) of several partial schematic cross sectional views of Figures 10 and 11. Figure 12 is a cross sectional schematic view of the stent mounting region 90 taken along lines 12-12 of Figure 10. Figure 12 shows a guide wire 16 along a longitudinal axis (in the plane of the paper) of the tubular stent mounting region 90 and within the inner guide channel member 70. A stent 17 surrounds the inner guide channel member 70, and in turn an outer guide channel member 80 covers the stent 17.

Figures 13A through 13C are cross sectional schematic views taken along lines 13-13 of Figures 10 and 11. Figure 13A shows an inner compression member 41, which comprises an elongated pusher bar, stiffening member, or stiff polymer that helps to "push" the stent out at or near the distal portion 13 by preventing prolapse, recoil, kinking, or movement (collectively, "prolapse") of the inner guide channel member 70 relative to the outer guide channel member 80 during proximal retraction of the outer member to deploy self-expanding stent. While the inner compression member 41 is depicted at about 6 o'clock, that position is only illustrative, and can be located elsewhere within the boundary defined by the outer guide channel member 80. The inner compression member 41 may also have other configurations. By way of illustration, those configures include a winged-shaped inner compression member 41 as in Figure 13B, or a "D-shaped" inner compression member 41 as in Figure 11C for bonding and for an increased lumen area for higher flow with injecting fluids (the characteristics of which are described below in more detail).

Figures 14A through 14F are alternate illustrative embodiments of cross sectional schematic views taken along lines 14-14 of Figures 10 and 11. These figures show various embodiments of several features, including additional embodiments of the inner compression member 41, an optional guide 51, and various embodiments of the guide channels 71, 81 of the inner and outer members 70, 80. These figures and their corresponding features are discussed next, starting with the inner compression member 41 embodiments.

By way of illustration and not by way of limitation, Figures 14A through 14F depict an inner compression member 41, such as a pusher bar, of different configurations. In Figures 14A, 14B, and 14E, for instance, the inner compression member 41 is a pusher bar represented as a rod-like structure having a generally circular cross section. However, the inner compression member 41 may also assume other configurations, such as a semicircular shape (Fig. 14C), an arcuate shape (Fig. 14D), a crescent shape (Fig. 14F), or the winged-shape or "D-shaped" previously discussed in connection with Figures 13B and 13C, respectively, to name a few examples of alternate configurations. These configurations help to provide an inner compression member 41 with increased column strength guarding against prolapse and having a low profile.

More specifically, Figure 14A depicts an optional guide 51, which can take on numerous configurations so as to achieve the function of aligning the wire guide as it exits proximally the distal portion 13, such as a saddle or a shelf shape (Fig. 14A) or a butterfly or dog bone shape (Fig. 14B) to name a few. Additionally, it should be understood in Figure 14A that the saddle groove of the guide 51 could abut the inner compression member 41 (e.g., pusher bar) and allow limited passage of the wire guide 16.

Likewise, the guide channel 71 of the inner guide channel member 70 and the guide channel 81 of the outer guide channel member 80 may assume numerous configurations to ensure a low profile at the distal end, and to guide the wire guide 16. These configurations include but are not limited to spherical, elliptical, crescent, saddle, slit, or wishbone shapes, such as by way of illustration the co-axial channels 71, 81 of Figure 14C, a groove guide channel 71 leading to the interior lumen of channel 81 as shown in Figure 14D, or the channels 71, 81 may be concentric semi-circular channels as depicted in Figure 14E. Additionally, the diameters of channels 71, 81 need not be constant, and optionally in certain embodiments may be tapered (reduced circumference).

Figure 15 is an alternate illustrative embodiment of a cross sectional schematic view taken along lines 15-15 of Figures 10 and 11 (absent the wire guide 16). This figure shows an inner compression member 41 and an outer sheath 50. Furthermore, Figure 15 illustrates an optional groove 52 on the outside surface of the outer sheath 50 to help the wire guide run proximally alongside the outside of the outer sheath.

Figure 16 is an alternative embodiment of an outer guide channel member 80 used in a delivery system for the rapid insertion of self-expanding stents during deployment of a stent. The outer guide channel member 80 is tubular and includes a wire guide entry port 82 and a breech position opening 65 comprising a proximal wire guide exit port 83, the entry and exit ports defining a guide channel 81 of the outer guide channel member 80. The exit port 83 in this embodiment is slanted. The outer guide channel member 80 has a stepped 84, 85 profile comprising a first outer diameter 84 and a second smaller outer diameter 85 located proximal to the larger first outer diameter 84. The second smaller outer diameter 85 offsets the added diameter of the wire guide as it leaves the exit port 83 and runs proximally alongside the outside of the outer sheath, thereby maintaining a negligible dimension to the profile, which is a particular advantage in procedures involving narrow vessel passageways.

Figure 17 is an alternative embodiment of an inner guide channel member 70 used in a delivery system for the rapid insertion of self-expanding stents during deployment of a stent. The inner guide channel member 70 in this embodiment is tubular and has a distal arrowhead tip 70' that is rounded and chamfered to be better tolerated by the patient. Formed from a suitable material (discussed above), the distal tip 70' is soft, rounded, and flexible so as to provide further safety to the patient. The inner guide channel member 70 has a wire guide entry port 72 and a proximal wire guide exit port 73 that define a guide channel 71. The exit port 73 in this embodiment is transverse to an inner compression member 41 and attached thereto by any suitable means, such as welding, brazening, adhesives, wires, chemical cross-linking, heat source, light source, radiofrequency, lasering, or other energy source for attaching the inner guide channel member 70 to the inner compression member 41.

Figure 18 shows a schematic sectional side view of an alternative embodiment of a distal portion 13 of a delivery system for the rapid insertion of self-expanding stents. This embodiment further comprises a puller 54. Furthermore, this embodiment comprises an inner guide channel member 70, an outer guide channel member 80 axially slideable relative to the inner guide channel member 70, and a stent mounting region 90 sandwiched between the inner and outer guide channel members 70, 80, respectively. Moreover, the outer member guide channels 71, 81 are substantially aligned coaxially along an approximate center longitudinal axis. Therefore, the wire guide 16 moves through the guide channel 71 to the outer member guide channel 81 and out the outer member exit port 83, and thereby egresses the breech position opening 65 (e.g., rear, back, or proximal part of the distal portion 13) with relatively little kinking, bending, buckling, or bowing. The breech position opening 65 is oversized to facilitate the wire guide 16 exiting the distal portion 13 with relatively little kinking, bending, buckling, or bowing.

The puller 54 of the embodiment of the distal portion 13 of Figure 16 has a low profile relative to the first outer diameter 84 and a second smaller outer diameter 85 sufficient to provide a stiffening element, a tension element, and an occlusion element. Optionally, the puller 54 is sandwiched between an outer surface 47 of the inner compression member 41 and an inner surface 57 of the outer sheath 50. Also, the puller 54 may be unattached but wedged nearly immovably between the compression member outer surface 47 and the outer sheath inner surface 57, or it may be attached by mechanical structures, chemical bonding materials, and welding materials or combinations as described above in connection with the bridge 100. Optionally, the puller 54 is tubular and surrounds a portion of the inner compression member 41, in which case the puller 54 may have a plurality of thicknesses sufficient to lift the inner compression member 41 and occlude the wire guide 16 from entering a passageway 45 of the inner compression member 41 or a gap 55 (as described below). Furthermore, the puller 54 may be a cutout ring, with the cutout located on the portion of the puller closest to the exit port 83. In yet another embodiment, the puller 54 comprises a protuberance that lifts the inner compression member 41 and thereby occludes the wire guide 16 from the inner compression member 41 passageway 45 or a gap 55 between the outer sheath inner surface 57 and the compression member outer surface 47.

The puller 54 may vary in length and height. The length may vary from about 1 to 10 cm, from about 2 to 6 cm, and in one embodiment has a length of approximately 5 cm. Optionally, the puller could run approximately the length of the middle section delivery device 14, or about 125 cm, so long as the puller height increases at or near the proximal wire guide exit port 83 of the outer guide channel member 80 to lift the inner compression member 41 and occlude the wire guide 16. The overall height, as measured from a longitudinal axis of the distal portion 13 at or near the exit port 83, also may vary. In one embodiment, the height approximately equals or slightly exceeds the absolute difference between the outer diameter 84 and the inner diameter 85. In another embodiment, the height is from about 0.2 French to about 2.0 French, and in another the height approximates 1 French.

As a stiffening element, the puller 54 reduces the flexing, buckling, kinking, or bending of the distal end at or near the exit port 83. Moreover, the puller 54 may be used where structural integrity is compromised, as in a case where the exit port 83 is a large longitudinal gap and reduced material, low profile, and circumference at the breech position opening 65 as shown in Figure 20. Optionally, the construction of a bridge 100, as already explained, may be modified for a puller 54, which may comprise any suitable material used for a bridge.

As an optional occlusion element, the puller 54 may lift the inner compression member or the wire guide 16 in order to ensure that the wire guide 16 does not enter a passageway 45 (e.g., Fig. 7) of an inner compression member and/or the passageway 59 (e.g., Figs 6, 7, 8, 9) of the outer sheath 50. In other words, the wire guide 16 may unintentionally enter that inner compression member passageway 45 (e.g., Fig. 7) or outer sheath passageway 59 (e.g., Figs 6, 7, 8, 9) as the wire guide exits proximally from the proximal wire guide exit port 83 of the outer guide channel member 80. By lifting the inner compression member and occluding the wire guide, the wire guide 16 exits the distal portion 13 in a position exterior to the outer sheath 50.

Figures 18 and 19 illustrate deployment of an embodiment of a distal portion 13 having a puller 54. In Figure 19, the distal portion 13 is in an un-deployed position; while Figure 18 depicts the distal portion 13 in a deployed position after proximal retraction of an outer guide channel member 80 relative to the inner guide channel member 70. The wire guide 16 enters the distal portion 13 and then exits from a breech position opening 65 of the delivery system at a substantially straight path so as to minimize kinking of the wire guide, and then runs proximally along the outside surface of the outer sheath 50. At or near the exit port 83, the puller 54 produces a lift that causes the wire guide 16 to be guided toward the exit port 83 and leave the outer guide channel 80.

Figure 20 shows an embodiment of an outer guide channel member 80 used in a distal portion 13 having puller 54. The outer guide channel member 80 comprises a wire guide entry port 82 and a proximal wire guide exit port 83 defining a guide channel 81. The outer guide channel member 80 has a first outer diameter 84 and a proximal second smaller outer diameter 85 at or near the breech position opening 65. The exit port 83 in this embodiment is sloped in this embodiment, although any of the previously discussed configurations would be suitable. The puller 54 is positioned at or near the exit port 83 and extends proximal thereto a length as previously described.

Figure 21 shows an embodiment of an inner guide channel member 70 used in a distal portion 13 having a puller 54 according to Figures 18-20 and an inner compression member 41. The puller lifts the distal end of the inner compression member 41 at an optional ramp 61 of a slight angle or grade in a transition region 60. This lift helps to ensure that the guide channel 71 of the inner guide channel member 70 is approximately aligned and substantially coaxial with the outer member guide channel 81 and/or the exit port 83 (Figures 18-20). Alignment helps to facilitate transition of the wire guide and to direct the wire guide from the outer member wire guide entry port 82, through the guide channel 81, and toward the breech position opening 65 located at or near the rear, back, or the proximal part of the outer member second end portion 87 as illustrated in Figures 18, 19, and 20.

Figure 22 shows still another embodiment of a delivery system having a breech position opening 65 for the rapid insertion of self-expanding stents. This embodiment has several features that add to the utility and ease of aspirating the stent mounting region 90 where a stent is positioned about a stent platform 91 on the outside surface located at or near the distal end of the inner guide channel member 70 intermediate distal tip of the inner member and a proximal restraint 93, and is sandwiched between the platform 91 and the inner surface 92 of the outer guide channel member 80, which controls premature deployment of the stent.

In this embodiment, the distal portion 13 of the device comprises a venting structure. A flushing port 94 is positioned at the distal portion of the stent mounting region 90 to aspirate air out from inside the delivery system and from around the outer guide channel member 80, thereby reducing the chance of air embolisms. In addition, there may be a flushing pathway 74 in the outer diameter 75 of the inner member between the stent platform 91 and the proximal wire guide exit port 73, thereby improving flow of air out of the system in the area of the stent during air aspiration. Also, the flushing port 94 may be formed in the outer guide channel member 80 intermediate the stent mounting region 90 and the exit port 83. Furthermore, the outer guide channel member exit port 83 may comprise a slit 186 for the wire guide to exit the distal portion 13. In one embodiment, the distal end of the inner compression member 41 passageway 45 may comprise a valve 61 such as a simple flap or diaphragm type valve as previously explained for purging air from the interior of the delivery system in the area of the stent, and in a closed position may also act as an optional guide to direct the wire guide toward the slit 186. It should be understood that these venting structures are optional, and that the device may include one or more of these structures.

Figures 23A and 23B are cross sectional schematic views taken along lines A-A and B-B, respectively, of Figure 22. Figure 23A shows an outer guide channel member 80, an inner guide channel member 70 having a channel 71, and a longitudinal groove vent 74 in the outer diameter of the inner guide channel member 70. Figure 23B shows a cross sectional view of the inner compression member 41 defining a passageway 45, the outer guide channel member 80 having a guide channel 81, and a slit 186 formed in the outer guide channel member 80. The structures of Figures 23A and 23B are illustrative, and alternative structures consistent with the venting feature of Figure 22 are contemplated as being within this description.

Figure 24 is a schematic side view of an alternative embodiment of an elongate inner compression member 41 at or near a distal portion of a delivery system having a breech position opening for the rapid insertion of self-expanding stents. The distal end of the inner compression member 41 has an increasing degree of flattening at or near the exit port 83 so as to allow bending and room for the wire guide to pass.

The inner compression member 41 may have the dimensions (diameter, length) a previously described. Similarly, the inner compression member 41 may comprise the materials as previously described. In one embodiment, the inner compression member 41 comprises a proximal end 40, a middle section 40', and a distal mating end 48 having an outer engaging surface 48'. Like elements from the previous drawings, embodiments, and description from above are labeled the same.

The overall length of the distal mating end 48 may vary. in one embodiment, it is from about 1.0 mm to about 10.0 mm. In another embodiment, the distal mating end 48 is approximately 3.0 mm. Furthermore, the distal mating end 48 optionally comprises a decreasing height 148 (Figs. 24, 24B) discussed below. The decreasing height 148 may extend along the length of the distal mating end 48 to the distal mating end outer engaging surface 48' and/or to a contoured configuration 48" that is described below. In one embodiment, the decreasing height 148 occurs along a length of about 3.0 mm, while in another embodiment the decreasing height 148 occurs over a much shorter length, such as approximately 0.5 mm. The contoured configuration 48" and outer engaging surface 48' may occur along the entire length of the distal mating end 48 or the decreasing height 148. Alternatively, the distal mating end 48 has a decreasing height 148 down to the contoured configuration 48" and outer engaging surface 48', and in one embodiment the decreasing height 148 is about 0.5 mm in length while the contoured configuration 48" and outer engaging surface 48' may extend distally from the decreasing height 148 and measure approximately 2.5 mm in length, for a distal mating end 48 that is approximately 3.0 mm in overall length.

Figures 24A and 24B show cross sectional views of Figure 24 taken along the lines 24A-24A and 24B-24B, whereby the inner compression member 41 in Figure 24A has an approximate height (e.g., outer diameter or thickness) of approximately 0.024 inches and Figure 24B shows an embodiment of a decreasing height 148 from less than about 0.24 inches to greater than about 0.08 inches, and in one embodiment the decreasing height 148 is approximately 0.016 inches from top 148' to bottom 148". The term "decreasing height" and variations thereof describe embodiments of the invention, rather than any lexicographic definition. For instance, the inner compression member distal mating end 48 may gradually decrease from top 148' to bottom 148" over the length of the mating end 48 in a variety of ways. In other words, the distal mating end 48 may have a portion that becomes gradually lesser in height (Fig. 24B) - relative to the inner compression member 41 (Fig. 24A) - in the distal direction. A decreasing height 148 may result from or be measured in a height, thickness, cross-section, diameter, width, and/or other configuration, shape, form, profile, structure, external outline, and/or contour that is lesser in height from the top 148', from the bottom 148", or as shown in Figure 24B from both the top 148' and the bottom 148". It should be understood that the top 148' and bottom 148" may be relative, and the inventions includes without limitation an embodiment where top 148' and bottom 148" are viewed end-on, a side view, or any view along the x-axis and y-axis from 0 to 2π (e.g., including the top 148' designated from 0 radians and bottom might be π radians). In one embodiment, the top 148' is the portion facing the outer engaging surface 164 of the base securing section 154.

While the decreasing height 148 may have a gradually smaller cross sectional area when compared to the cross sectional area at the inner compression member 41 (Figs. 24, 24A) that is proximal to the distal mating end 48, the cross sectional area need not decrease. For example, the width might increase inversely proportional to the decrease in height as shown in Figure 24B, although if desired the increased width could be cut away, machined, trimmed, chipped, planed, and the like. Also, this is not to say that the distal mating end 48 may only decrease in height. Indeed, the decreasing height 148 might extend to a position at or near the contoured configuration 48", which might increase in height, such as with an upward curve or other configuration that reverses to form a fluted embodiment, a flare, or upward protrusion. Furthermore, the distal mating end 48 may comprise more than one portion having a decreasing height, whereby the distal mating end 48 decreases in height from top 148' to bottom 148", then increases in height from top 148' to bottom 148", only to decrease again along the length of the distal mating end 48.

According to the invention, the inner compression member distal mating end 48 and/or outer engaging surface 48' further comprise a contoured configuration 48" that is generally complementary to the cannula 95 (e.g., Figs. 9, 9A, 9B, 9C) and/or the second end of the inner guide channel member (e.g., Figs. 6, 7, 8, 9) as previously described. Figure 24C, by way of example and not by way of limitation, an embodiment of a contoured configuration 48" and an outer engaging surface 48' taken along the line 24C-24C. In Figure 24C, the contoured configuration 48" and outer engaging surface 48' are both flat. While the contoured configuration 48" has decreased in height relative to the inner compression member 41 shown in the background with a circular cross section, the contoured configuration 48" in Figure 24C has an increased width.

The decreasing height 148 and the contoured configuration 48" may be achieved by swaging, stamping, forging, or rolling between two rollers. For example, rolling the shaft between rollers of a mill will help to prevent any lessening of strength (i.e., fatigue strength) that could result if the decreasing height 148 and/or the contoured configuration 48" were machined. It should be understood that the shape of the inner compression member 41 can be chosen depending on the shape of the cannula 95, the outer guide channel member 80, and/or the inner guide channel member 70, and the decreasing height 148 and/or the contoured configuration 48" (or other configurations) may vary along the longitudinal axis of the inner compression member 41. Furthermore, the degree of flatness of an inner compression member 41 can determine the flexibility in a certain direction.

Figures 25A through 25C are schematic alternative embodiments of a distal portion 13 of a delivery system having a breech position opening 65 for the rapid insertion of self-expanding stents according to the invention. In these embodiments, the distal portion 13 has a stepped 84, 85 profile comprising a first outer diameter 84 and a second smaller outer diameter 85. The distal portion 13 further comprises an inner guide channel member 70, an outer guide channel member 80 axially slideable relative to the inner guide channel member 70, a stent mounting region 90, and a transition region 60.

Figures 25A and 25C show a two-piece embodiment of an outer sheath 50 and an outer guide channel member 80 joined by any suitable means as previously described. At or near the transition region 60, the inner guide channel member 70 has an inner sheath flange 176 that engages the outer sheath outer edge 152 on the outside surface of the outer sheath 50 at an overlapping engagement region 160. Because the flange 176 and outer sheath edge 152 overlap at the overlapping engagement region 160, this arrangement facilitates a wire guide 16 to run along the outside of the outer sheath instead of entering a lumen of the outer sheath or inner compression member lumen (not shown) of the outer sheath. During deployment, the length of the overlapping engagement region 160 will diminish as the outer guide channel member withdraws proximally. In the non-deployed state, the overlapping engagement region 160 of Figure 25A has a lesser length than that region 160 of Figure 259B. Therefore, the distal portion 13 of Figure 25A should have a reduced length relative to the distal portion 13 of Figure 25B.

Figure 25C shows an integral outer sheath 50 and outer guide channel member 80 formed from a continuous tube with a first outer diameter 84 and a second smaller outer diameter 85. Like Figures 25A and 25B, at or near the transition region 60, the inner guide channel member 70 has an inner sheath flange 176 that engages the outer sheath outer edge 152 on the outside surface of the outer sheath 50 at an overlapping engagement region 160.

### METHODS

Methods of manufacturing and of providing a medical device for delivering a self-expanding stent are also provided.

One method provides a delivery system 10 for the rapid insertion of self-expanding stents comprising a system proximal portion 12; a middle section delivery device 14; and a system distal portion 13 having an inner guide channel member 70 with a guide channel 71, an outer guide channel member 80 axially slideable relative to the inner guide channel member 70 and having a guide channel 81 arranged relative to the inner member guide channel 71 to reduce any bending of the wire guide as the wire guide moves proximally from the inner member guide channel 71 to the outer member guide channel 81, and a stepped 84, 85 profile; and wherein the system distal portion 13 further comprises a self-expanding deployment device mounting region 90 (e.g., a stent mounting region), a transition region 60, and a proximal breech opening 65 located at or near the rear, back, or proximal part 87 of the distal portion 13. A self-expanding device 17 is loaded into a radially compressed state at the stent mounting region 90. The distal portion 13 is slid over a guiding device such as a wire guide, catheter, and the like either by back-loading or front-loading. The outer guide channel member 80 is retracted proximally relative to the inner member so as to deploy the stent 17.

In a method of manufacturing the invention as an alternative to purchasing the inner and outer guide channel members 70, 80 and/or the outer sheath 50 from Cook Incorporated, a first rectangular sheet 401 and a second rectangular sheet 401' of inner and outer member 70, 80 materials are provided-each sheet 401, 401' having opposing A and B sides and opposing C and D sides. Opposing sides A and B of the first sheet 401 are joined to form an inner guide channel member 70 having an entry port 72 at a first end portion 78 and an exit port 73 at a second end portion 77, the ports 72, 73 defining a guide channel 71, and a self-expanding deployment device mounting region 90 (e.g., a stent mounting region) intermediate the first and second end portions 78, 77, respectively. Adjacent corners of side C of the second sheet 401' are cut and the opposing sides A and B joined to form an outer guide channel member 80 having an entry port 82 at a first end portion 88 (side D) and exit port 83 at a second end portion 87 (side C), the ports 82, 83 defining a guide channel 81, with the outer member having a stepped 84, 85 profile, whereby the outer member comprises a greater first outer diameter 84 at the first end portion 88 and a second smaller outer diameter 85 located at or near the outer member second end portion 87. Alternatively, when joining opposing sides of a rectangular sheet, features such as entry ports 72, 82 and exit ports 73, 83 and guide channels 71, 81 can be shaped from the open ends. Also, the configuration of the exit port, such as an oblique shape for the outer member exit port 83, may be stamped or cut away from the sheet after joining the edges (e.g., sides) of the sheet. The inner member is slidably joined within the second member guide channel 81. In all of these manufacturing methods, the inner and outer members 70, 80, respectively, may vary in length given the different sizes of the vessels and vessel passageways intended for the use of the device.

In a method of manufacturing a sheath for use with the middle section delivery device 14 and/or distal portion 13, as another alternative to purchasing the outer sheath 50 or the inner and outer guide channel members 70, 80 from Cook Incorporated, when forming the middle section delivery device 14 or distal portion 13 from a sheet of material (such as a material comprising Teflon) consistent with the outer sheath 50 or one of the members 70, 80, a sheet of material for the inner layer 44 may be formed into a tubular body on a mandrel and then heated in an oven while inside a shrink tube. After cooling, the shrink tube is peeled off and, if the tubular body inner layer 44 is to be used as an inner compression member 41 or an inner guide channel member 70, the mandril is removed. For assembling the outer sheath or the outer guide channel member 80, the tubular body inner layer 44 is left on the mandril while a coil 43 is wrapped around the tubular body inner layer 44. A sheet of material for the outer layer 42 is then formed around the coil 43 layer of the tubular body inner layer 44, and the mandril pulled out. In order to assemble an outer guide channel member 80 with an eccentric stepped 84, 85 profile (i.e., a reduction in outer diameter) in the area of the proximal wire guide exit hole (e.g., the exit port 83), the outer guide channel member 80 is formed from material of inner layer 44 on a mandril having a larger diameter 85 for a first end portion 88 and a second mandril having a smaller diameter 84 (or one mandril of these diameters 85, 84) according to the coil 43 step. Then, an outer layer 42 of materials, such as a Nylon and/or PEBA overcoat, is extended across the mandrel outer diameter and across a step down diameter (i.e., the second smaller outer diameter 85 relative to the first outer diameter 84) at the proximal end of the mandrel, the assembly is heated and shrunk together and, once cooled, the mandril pulled out, and the second end portion 87 cut, machined, or milled in order to provide the outer member wire guide exit port 83 and the optional lateral walls 83a, 83b.
This results in a one-piece, multiple diameter delivery system outer guide channel member 80 that has the advantages of a reinforced type of restraining sheath in the area of the stent. Thus, outer guide channel member permits the wire guide to exit without adding to the diameter of the outer member and, in fact, presenting a lower profile at or near the exit port 83 of the outer member.

In a method of manufacturing a delivery apparatus for the rapid insertion of self-expanding devices, a middle section delivery device 14 having an inner compression member 41 and outer sheath 50 and a passageway 59 and having an insert 130 comprising an outer sheath mating portion 132 secured at or near an outer sheath distal end 50' and having an interfacing portion 134 and guide channel 133 (as described above and shown in the figures) is provided.

A distal portion 13 has an outer guide channel member 80 with a second end portion 87 and an insert 120 comprising a distal insert mating portion 122 secured at or near an outer member second end portion 87 and having an interfacing portion 124 and guide channel 81' (as described above is provided), the distal portion 13 further having an inner guide channel member 70 with an guide channel 71; the outer guide channel member 80 axially slideable relative to the inner guide channel member 70 and having a guide channel 81 and a stepped 84, 85 profile; a self-expanding deployment device mounting region 90 (e.g., a stent mounting region); a transition region 60; and a proximal breech opening 65 located at or near the rear, back, or second end portion 87 of the distal portion 13.

Materials and methods of manufacturing a suitable middle section delivery device 14 and distal portion 13 are described in Published United States Patent Application 2004/0116833 (Koto et al.) and in United States Patent No. 6,589,227 (Sonderskov Klint, et al.; Assigned to Cook Incorporated of Bloomington, Indiana and William Cook Europe of Bjaeverskov, Denmark). Examples further include a multifilar embodiment and a "Flexor® sheath" as described above and shown in the figures, or other component. Furthermore, the "Flexor® sheath," manufactured and sold by Cook Incorporated of Bloomington, Indiana, may be adapted for use with the distal portion 13 and/or the middle section delivery device 14. The Flexor® sheath, as previously described and shown in the figures above, may be provided for the distal portion 13 and/or the middle section delivery device 14. The insert interfacing ends 124, 134 are brought together. The interfacing ends 124, 134 are secured by a connector 110.

A method of manufacturing and of providing a medical device for delivering a self-expanding stent need not be performed sequentially. For instance, a self-expanding device 17 may be loaded before the delivery system 10 is provided. The adjacent corners of side C of the second sheet 401' may be cut and the opposing sides A and B joined to form an outer guide channel member 80 before the inner member is formed by joining opposing sides A and B of the first sheet 401. The distal portion 13 may be provided before providing the middle section delivery device 14.

It is intended that the foregoing detailed description of the delivery system and devices for the rapid insertion of self-expanding stents be regarded as illustrative rather than limiting, and that it be understood that it is the following claims, including all equivalents, that are intended to define the spirit and scope of this invention. Terms are to be given their reasonable plain and ordinary meaning. Also, the embodiment of any figure and features thereof may be combined with the embodiments depicted in other figures. Other features known in the art and not inconsistent with the structure and function of the present invention may be added to the embodiments.

While particular elements, embodiments and applications of the present invention have been shown and described, it will be understood, of course, that the invention is not limited thereto since modifications may be made by those skilled in the art, particularly in light of the foregoing teachings. Therefore, it is therefore contemplated by the appended claims to cover such modifications as incorporate those features which come within the scope of the invention.

## Claims

1. A delivery apparatus (10) for the rapid insertion of medical devices, comprising:
a system proximal portion (12);
a middle section delivery device (14) comprising an elongate outer sheath (50) and an elongate inner compression member (41), the outer sheath comprising a proximal end portion (57) having an opening (53) and a distal end portion (58) having an opening (52) and defining a passageway (59) therebetween, the outer sheath proximal end portion being operatively coupled to the handle, the inner compression member having a middle section (40') extending through the outer sheath passageway, and a distal mating end portion (48) ; and
a system distal portion (13) comprising an inner guide channel member and an outer guide channel member (80), the inner guide channel member (70) including a distal first end portion (78) having an entry port (72) and a proximal second end portion (77) having an exit port (73) and defining a guide channel therebetween, the outer guide channel member axially slidably receiving the inner guide channel member second end portion and further comprising a distal first end portion (88) and a proximal second end portion (87), an entry port (82) and an exit port (83) defining a guide channel (81) therebetween, and a stepped profile (84,85) having a first outer diameter (84) intermediate the outer guide channel member first and second end portions and a second smaller outer diameter (85) located at or near the outer guide channel member second end portion;
**characterised in that** the system distal portion and middle section delivery device are operatively coupled.

2. The device of claim 1 further comprising a self-expanding deployment device mounting region disposed within the outer guide channel member and comprising a proximal stent restraint, an inner guide channel member medical device platform, and an outer guide channel member stent constraining inner surface.

3. The device of claim 2 further comprising a transition region proximal to the self-expanding deployment device mounting region, wherein the inner guide channel member exit port is in communication with the outer guide channel member exit port and comprising a breech position opening located proximal to the inner member exit port.

4. The device of claim 1 wherein the guide channel of the inner guide channel member and the guide channel of the outer guide channel member are substantially coaxially aligned.

5. The device of claim 1 wherein the inner guide channel member is tubular and comprises a longitudinal axis, and the outer guide channel member exit port is substantially aligned with the inner guide channel member longitudinal axis.

6. The device of claim 5 such that the inner guide channel member longitudinal axis intersects the outer guide channel member exit port without the inner guide channel member second end portion bending more than about 15 degrees.

7. The device of claim 1 wherein the system distal portion and middle section delivery device are operatively coupled by operatively coupling the outer guide channel member second end portion and the outer sheath distal end portion.

8. The device of claim 1 wherein the system distal portion and middle section delivery device are operatively coupled by operatively coupling the inner guide channel member second end portion and the inner compression member distal mating end portion.

9. The device of claim 1 wherein the system distal portion and middle section delivery device are operatively coupled by a bridging device.

10. The device of claim 1 wherein the system distal portion and middle section delivery device are operatively coupled by an insert.

11. The device of claim 1 wherein the system distal portion and middle section delivery device are operatively coupled by an integrally formed outer sheath and outer guide channel member.

## Patentansprüche

1. Zuführvorrichtung (10) zum schnellen Einführen von medizinischen Vorrichtungen, umfassend:
einen proximalen Systemabschnitt (12),
eine Zuführvorrichtung (14) der mittleren Sektion, die eine längliche äußere Hülle (50) und ein längliches inneres Kompressionsglied (41) umfasst, wobei die äußere Hülle einen proximalen Endabschnitt (57) mit einer Öffnung (53) und einen distalen Endabschnitt (58) mit einer Öffnung (52) umfasst, der einen Durchgang (59) dazwischen definiert, wobei der proximale Endabschnitt der äußeren Hülle mit dem Griff wirkgekoppelt ist, wobei das innere Kompressionsglied eine mittlere Sektion (40') hat, die sich durch den Durchgang der äußeren Hülle erstreckt, sowie einen zusammenpassenden distalen Endabschnitt (48) und
einen distalen Systemabschnitt (13), der ein inneres Führungskanalglied und ein äußeres Führungskanalglied (80) umfasst, wobei das innere Führungskanalglied (70) einen distalen ersten Endabschnitt (78) mit einem Eingangsport (72) und einen proximalen zweiten Endabschnitt (77) aufweist, der einen Ausgangsport (73) hat und einen Führungskanal dazwischen definiert, wobei das äußere Führungskanalglied den zweiten Endabschnitt des inneren Führungskanalglieds axial gleitend aufnimmt, und ferner einen distalen ersten Endabschnitt (88) und einen proximalen zweiten Endabschnitt (87), einen Eingangsport (82) und einen Ausgangsport (83) umfasst, der einen Führungskanal (81) dazwischen definiert, sowie ein abgestuftes Profil (84, 85) mit einem ersten äußeren Durchmesser (84) zwischen dem ersten und dem zweiten Endabschnitt des äußeren Führungskanalglieds und einem zweiten kleineren äußeren Durchmesser (85), der sich an dem zweiten Endabschnitt des äußeren Führungskanalglieds oder in dessen Nähe befindet,
**dadurch gekennzeichnet, dass** der distale Systemabschnitt und die Zuführvorrichtung der mittleren Sektion wirkgekoppelt sind.

2. Vorrichtung nach Anspruch 1, ferner umfassend einen Montagebereich für eine selbstexpandierende Absetzvorrichtung, der im äußeren Führungskanalglied angeordnet ist und eine proximale Stenthalterung, eine Plattform des inneren Führungskanalglieds für eine medizinische Vorrichtung und eine einen Stent festhaltende innere Fläche des äußeren Führungskanalglieds umfasst.

3. Vorrichtung nach Anspruch 2, ferner umfassend einen Übergangsbereich proximal zu dem Montagebereich für eine selbstexpandierende Absetzvorrichtung, wobei der Ausgangsport des inneren Führungskanalglieds mit dem Ausgangsport des äußeren Führungskanalglieds kommuniziert und eine Verschlusspositionsöffnung umfasst, die proximal zum Ausgangsport des inneren Glieds angeordnet ist.

4. Vorrichtung nach Anspruch 1, wobei der Führungskanal des inneren Führungskanalglieds und der Führungskanal des äußeren Führungskanalglieds im Wesentlichen koaxial aufeinander ausgerichtet sind.

5. Vorrichtung nach Anspruch 1, wobei das innere Führungskanalglied röhrenförmig ist und eine Längsachse umfasst und der Ausgangsport des äußeren Führungskanalglieds im Wesentlichen auf die Längsachse des inneren Führungskanalglieds ausgerichtet ist.

6. Vorrichtung nach Anspruch 5, wobei die Längsachse des inneren Führungskanalglieds den Ausgangsport des äußeren Führungskanalglieds schneidet, ohne dass sich der zweite Endabschnitt des inneren Führungskanalglieds um mehr als ungefähr 15 Grad biegt.

7. Vorrichtung nach Anspruch 1, wobei der distale Systemabschnitt und die Zuführvorrichtung der mittleren Sektion durch Wirkkoppelung des zweiten Endabschnitts des äußeren Führungskanalglieds mit dem distalen Endabschnitt der äußeren Hülle wirkgekoppelt sind.

8. Vorrichtung nach Anspruch 1, wobei der distale Systemabschnitt und die Zuführvorrichtung der mittleren Sektion durch Wirkkoppelung des zweiten Endabschnitts des inneren Führungskanalglieds mit dem distalen zusammenpassenden Endabschnitt des inneren Kompressionsglieds wirkgekoppelt sind.

9. Vorrichtung nach Anspruch 1, wobei der distale Systemabschnitt und die Zuführvorrichtung der mittleren Sektion durch eine Überbrückungsvorrichtung wirkgekoppelt sind.

10. Vorrichtung nach Anspruch 1, wobei der distale Systemabschnitt und die Zuführvorrichtung der mittleren Sektion durch einen Einsatz wirkgekoppelt sind.

11. Vorrichtung nach Anspruch 1, wobei der distale Systemabschnitt und die Zuführvorrichtung der mittleren Sektion durch eine äußere Hülle und ein äußeres Führungskanalglied, die integral ausgebildet sind, wirkgekoppelt sind.

## Revendications

1. Appareil (10) d'administration destiné à l'insertion rapide de dispositifs médicaux, comportant :
une partie (12) proximale de système ;
un dispositif (14) d'administration à section moyenne comportant une gaine (50) extérieure allongée et un élément (41) de compression intérieur allongé, la gaine extérieure comportant une partie (57) extrémité proximale dotée d'une ouverture (53) et une partie (58) extrémité distale dotée d'une ouverture (52) et délimitant une voie de passage (59) entre les deux, la partie extrémité proximale de la gaine extérieure étant accouplée de manière fonctionnelle à la poignée, l'élément de compression intérieur doté d'une section (40') moyenne qui s'étend dans la voie de passage de la gaine extérieure, et une partie (48) extrémité d'appariement distale ; et
une partie (13) distale de système comportant un élément de canal de guidage intérieur et un élément (80) de canal de guidage extérieur, l'élément (70) de canal de guidage intérieur comprenant une première partie (78) extrémité distale dotée d'un orifice (72) d'entrée et une seconde partie (77) extrémité proximale dotée d'un orifice (73) de sortie et délimitant un canal de guidage entre les deux, l'élément de canal de guidage extérieur recevant de manière coulissante axialement la seconde partie extrémité de l'élément de canal de guidage intérieur et comportant en outre une première partie (88) extrémité distale et une seconde partie (87) extrémité proximale, un orifice (82) d'entrée et un orifice (83) de sortie délimitant un canal (81) de guidage entre les deux, et un profil (84, 85) en gradins doté d'un premier diamètre (84) extérieur entre lesdites première et seconde parties extrémités de l'élément de canal de guidage extérieur et un second diamètre (85) extérieur plus petit situé au niveau ou à proximité de la seconde partie extrémité de l'élément de canal de guidage extérieur ;
**caractérisé en ce que** la partie distale du système et le dispositif d'administration à section moyenne sont accouplés de manière fonctionnelle.

2. Dispositif selon la revendication 1 comportant en outre une zone de montage du dispositif de déploiement auto-extensible disposée à l'intérieur de l'élément de canal de guidage extérieur et comportant une retenue proximale d'extenseur, une plate-forme de dispositif médical de l'élément de canal de guidage intérieur, et une surface intérieure de contrainte de l'extenseur de l'élément de canal de guidage extérieur.

3. Dispositif selon la revendication 2 comportant en outre une zone de transition proximale à la zone de montage du dispositif de déploiement auto-extensible, dans lequel l'orifice de sortie de l'élément de canal de guidage intérieur est en communication avec l'orifice de sortie de l'élément de canal de guidage extérieur et comprend une ouverture en position postérieure située proximalement à l'orifice de sortie de l'élément intérieur.

4. Dispositif selon la revendication 1 dans lequel le canal de guidage de l'élément de canal de guidage intérieur et le canal de guidage de l'élément de canal de guidage extérieur sont sensiblement alignés coaxialement.

5. Dispositif selon la revendication 1 dans lequel l'élément de canal de guidage intérieur est tubulaire et comporte un axe longitudinal, et l'orifice de sortie de l'élément de canal de guidage extérieur est sensiblement aligné avec l'axe longitudinal de l'élément de canal de guidage intérieur.

6. Dispositif selon la revendication 5 tel que l'axe longitudinal du canal de guidage intérieur croise l'orifice de sortie de l'élément de canal de guidage extérieur sans que la seconde partie extrémité de l'élément de canal de guidage intérieur se courbe de plus d'environ 15 degrés.

7. Dispositif selon la revendication 1 dans lequel la partie distale du système et le dispositif d'administration à section moyenne sont accouplés de manière fonctionnelle en accouplant de manière fonctionnelle la seconde partie extrémité de l'élément de canal de guidage extérieur et la partie extrémité distale de la gaine extérieure.

8. Dispositif selon la revendication 1 dans lequel la partie distale du système et le dispositif d'administration à section moyenne sont accouplés de manière fonctionnelle en accouplant de manière fonctionnelle la seconde partie extrémité de l'élément de canal de guidage intérieur et la partie extrémité d'appariement distale de l'élément de compression.

9. Dispositif selon la revendication 1 dans lequel la partie distale du système et le dispositif d'administration à section moyenne sont accouplés de manière fonctionnelle par un dispositif faisant pont.

10. Dispositif selon la revendication 1 dans lequel la partie distale du système et le dispositif d'administration à section moyenne sont accouplés de manière fonctionnelle par un insert.

11. Dispositif selon la revendication 1 dans lequel la partie distale du système et le dispositif d'administration à section moyenne sont accouplés de manière fonctionnelle par une gaine extérieure faisant partie intégrante de l'élément de canal de guidage extérieur.
